(19) Europäisches Patentamt
European Patent Office
Office européen des brevets

(11) Publication number: 0 383 469
A1

(12) EUROPEAN PATENT APPLICATION

(21) Application number: 90301210.2

(22) Date of filing: 06.02.90

(51) Int. Cl.5: C07D 493/08, C07D 493/18, A01N 43/90, //(C07D493/08, 311:00,311:00),(C07D493/18, 317:00,311:00,311:00), (C07D493/18,311:00,311:00, 303:00)

(30) Priority: 10.02.89 US 309771

(43) Date of publication of application: 22.08.90 Bulletin 90/34

(84) Designated Contracting States: GR

(71) Applicant: E.I. DU PONT DE NEMOURS AND COMPANY
1007 Market Street
Wilmington Delaware 19898(US)

(72) Inventor: Schlecht, Matthew Fred
18 West Ridge Court
Newark, Delaware 19711(US)
Inventor: Semple, John Edward
436 Coldspring Run
Newark, Delaware 19711(US)

(74) Representative: Hildyard, Edward Martin et al
Frank B. Dehn & Co. European Patent Attorneys Imperial House 15-19 Kingsway
London WC2B 6UZ(GB)

(54) **Herbicidal oxabicyclononane ethers.**

(57) Novel oxabicyclononane ether derivatives have the general formula:-

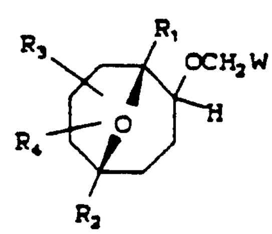

I

where each of $R_1$ - $R_4$ independently is hydrogen or a substituent and W is optionally substituted phenyl or a heterocyclic ring.

The compounds show selective herbicidal activity and may be used to control the growth of undesired vegetation in e.g. soybeans, cotton, sugar beet, rape, corn, wheat, barley and particularly in rice.

EP 0 383 469 A1

# HERBICIDAL OXABICYCLONONANE ETHERS

## CROSS-REFERENCE TO RELATED APPLICATION

This application is a continuation-in-part of copending application U.S.S.N. 07/309,771 filed on February 10, 1989.

## BACKGROUND OF THE INVENTION

### Field of the Invention

The present invention relates to novel oxabicyclononane ether derivative compounds, to compositions containing these ether derivative compounds, and to methods of using these compounds or compositions to control the growth of undesired vegetation in soybeans, cotton, sugar beets, rape, corn, wheat, barley and particularly in rice.

### Description of the Prior Art

U.S. Patents 4,567,283 and 4,670,041 disclose a variety of herbicidal bicyclic ethers of the Formula

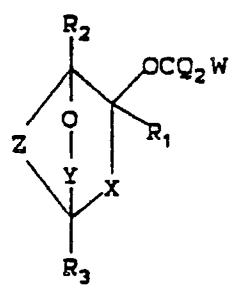

.

DE 2937645 generically discloses ethers as herbicides including oxabicyclo[3.3.1]nonane ethers.
U.S. 4,388,104 discloses ethers as herbicides including dioxabicyclo[3.2.1]nonane ethers.
EP-A 302,599 discloses [3.3.1]dioxabicyclononane ethers.

## SUMMARY OF THE INVENTION

This invention pertains to compounds of Formula I:

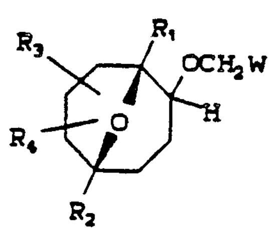

I

wherein:

$R_1$ is H or a $C_1$-$C_4$ straight chain alkyl group;

$R_2$ is H, $C_1$-$C_6$ alkyl, CN, $C_2$-$C_4$ alkenyl, $C_2$-$C_4$ alkynyl, phenyl, $CH_2Ph$ or $C_1$-$C_3$ alkyl substituted with OH, CN, $C_1$-$C_3$ alkoxy, OPH, $C_1$-$C_3$ alkyl- sulfonyl, $SO_2Ph$, $SO_2CH_2Ph$, $N_3$, $C_1$-$C_6$ alkoxy- carbonyl or $CO_2H$.

$R_3$ is H, F, Cl, Br, I, OH, PhSe, $C_1$-$C_3$ alkoxy, $OSO_2Ph$ optionally substituted with 1-3 $CH_3$, $PhSO_n$ optionally substituted with 1-3 $CH_3$, $CH_3Se$, phenyl, $C_2$-$C_4$ alkenyl or $C_1$-$C_4$ alkyl optionally substituted with 1-3 halogens or 1-3 $C_1$-$C_3$ alkoxy, or amino substituted with 0-2 alkyl groups of 1-3 carbons provided that when $R_3$ is $C_1$-$C_4$ alkyl substituted by $C_1$-$C_3$ alkoxy, $R_3$ is not on the carbon adjacent to the carbon bearing $R_1$;

$R_4$ is H, OH, $C_1$-$C_4$ alkyl, phenyl or CN;

$R_3$ and $R_4$ may be taken together to form a 5 membered ring acetonide optionally substituted with 1-2 $CH_3$ or $R_3$ and $R_4$ when on the same carbon may be taken together to form a ketone or $R_3$ may be taken together with an adjacent carbon to form a double bond with the proviso said double bond is not at a bridgehead carbon or the $OCH_2W$ carbon.

$R_3$ and $R_4$ on adjacent carbons may be taken together to form an epoxide; if $R_4$ = OH and $R_3$ and $R_4$ are on the same carbon, then $R_3$ cannot be OH, F, Cl, Br, I, PhSe, $CH_3Se$, alkoxy or amino;

W is phenyl optionally substituted with 1-3 substituents selected from F, Cl, Br, $C_1$-$C_3$ alkyl, $C_1$-$C_3$ alkoxy, OH, CN, $C_1$-$C_3$ haloalkyl, $C_1$-$C_3$ haloalkoxy, $C_1$-$C_3$ alkylthio, $C_2$-$C_4$ alkenyl or $C_2$-$C_4$ alkynyl, or W is a 4, 5, 6 or 7-membered heterocyclic ring containing at least one from among 0-3 nitrogen, 0-2 oxygen or 0-2 sulfur, each ring optionally substituted with 1-2 substituents selected from F, Br, CN, Cl, $CH_3$ or $OCH_3$. A representative exemplification of such heterocycles includes but is not limited to oxetane, furan, dioxane, oxepane, dioxolane, oxadiazole, thiadiazole, triazole, thiophene, tetrahydropyran, tetrahydrofuran, isoxazole, oxazole, pyrazole, imidazole, thiazole, pyridine and pyrazines; provided that the total number of heteroatoms is 3 or less; and

n is 0, 1 or 2.

Preferred embodiments are:

1. Compounds of Formula I wherein Formula I is;

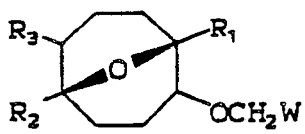 ,

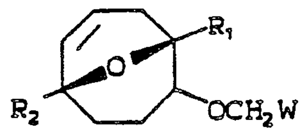 ,

Ia Ib

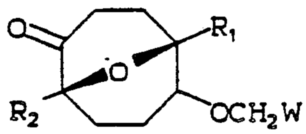 ,

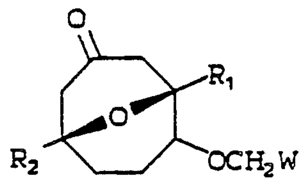 ,

Ic Id

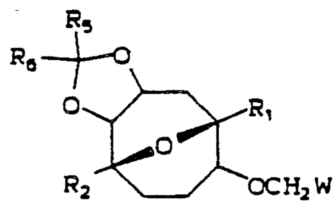 ,

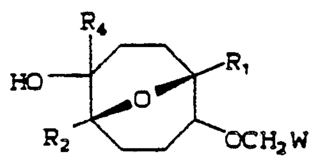

Ie If

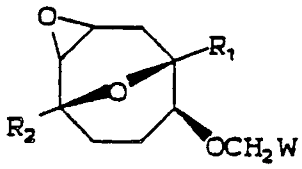

or

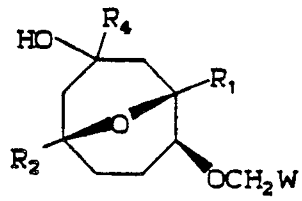

Ig Ih

wherein:
$R_5$ is H or $CH_3$; and;
$R_6$ is H, or $CH_3$.

2. Compound of Preferred 1 wherein;
$R_1$ is $C_1$-$C_3$ alkyl;

$R_2$ is $C_1$-$C_3$ alkyl, $C_2$-$C_3$ alkenyl, $C_2$-$C_3$ alkynyl, phenyl, $CH_2Ph$ or $C_1$-$C_2$ alkyl substituted by $OCH_3$, CN, $SO_2CH_3$ or $C_1$-$C_3$ alkoxycarbonyl.

3. Compounds of Preferred 2 wherein;

W is phenyl optionally substituted by 1-2 substituents selected from F, Cl, R, $CH_3$ or $OCH_3$ or W is a 5 or 6-membered heterocyclic ring containing 0-2 nitrogen, 0-2 oxygen or 0-2 sulfur;

$R_3$ is H, F, Br or Cl; and;

$R_4$ is H.

4. Compounds of Preferred 3 wherein

W is phenyl optionally substituted by 1-2 substituents selected from F, Cl, Br, $CH_3$ or $OCH_3$ or W is tetrahydropyran, tetrahydrofuran, thiophene, thiazole, isoxazole, pyridine or pyrazine, each ring optionally substituted with 1-2 substituents selected from F, Cl, Br, $CH_3$ or $OCH_3$.

5. Compounds of Preferred 4 wherein Formula I is Ia.

Specifically preferred for their biological activity and/or their ease of synthesis are:

1. 2-[(2-fluorophenyl)methoxy]-1,5-dimethyl-9-oxabicyclo[3.3.1]nonane; and
2. 2-[(2,6-difluorophenyl)methoxy]-1,5-dimethyl-9-oxabicyclo[3.3.1]nonane.

The symbol Ph when used in a compound as hereinabove means phenyl.

## DETAILED DESCRIPTION OF THE INVENTION

The compounds of Formula I exhibit geometrical and optical isomerism and may be prepared in geometrically or optically pure or mixed forms. The various individual optical and geometrical forms and various combinations thereof of the materials of the invention usually have some difference in herbicidal properties. Generally preferred for herbicidal activity are those geometrical isomers of Formula I wherein the $-OCH_2W$ group bears a syn relationship to the -O- bridge, i.e., the $-OCH_2W$ group resides in the exo orientation on the same face of the carbocyclic ring as the -O- bridge. The present invention contemplates all of the herbicidally active forms resulting from synthesis and from deliberately created mixtures.

Compounds of Formula II (i.e., Formula Ia wherein $R_1 = R_2 = CH_3$ and $R_3 = H$) are synthesized through the sequence shown in Scheme 1. Starting from the commercially available 1,5-dimethyl-1,5-cyclooctadiene, Formula III, epoxidation (see D. Swern in Organic Reactions, Vol. 7, A. H. Blatt, A. C. Cope, F. C. McGrew, C. Niemann and H. R. Snyder eds., J. Wiley & Sons, N.Y. (1953), pp. 378-433) with peracetic acid buffered with sodium carbonate provides a monoepoxide which is reductively cleaved with lithium aluminum hydride following a literature procedure (J. G. Trayhnam & P. M. Greene, J. Am. Chem. Soc., 86, 2658 (1964)) to yield the dimethylcyclooctenol of Formula IV. This intermediate is epoxidized with peracetic acid to give a mixture of cis and trans epoxides. The mixture is treated with para-toluenesulfonic acid in methylene chloride to bring about rearrangement to a mixture of exo and endo 9-oxabicyclo[3.3.1]nonan-2-ols. This type of acid-catalyzed rearrangement is taught in the art (U.S. 4,670,041). The mixture of exo and endo alcohols is separated chromatographically to give the pure exo isomer of Formula V. The 9-oxabicyclo[3.3.1]nonan-2-ol of Formula V is transformed into the 9-oxabicyclo[3.3.1]nonan-2-yl ether of Formula II by the well-known Williamson Ether Synthesis (see N. Baggett in Comprehensive Organic Chemistry, D. Barton and W. D. Ollis eds., Vol. 1, pp. 819-832 Pergamon Press, N.Y. (1979)). Thus, the target herbicides result when the bicyclic alcohol of Formula V is treated with a base such as sodium hydride in N,N-dimethylacetamide and the appropriate alkylating agent represented as $WCH_2X$ where W represents those organic radicals defined previously and X represents leaving group moieties such as chloride, bromide, iodide and sulfonate esters. The alkylating agents $WCH_2X$ are prepared from the alcohols $WCH_2OH$ in the conventional manners known to those skilled in the art.

SCHEME 1

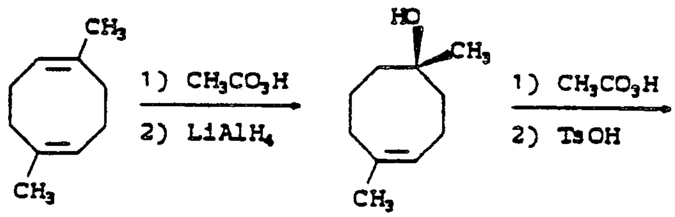


Formula III

Formula IV

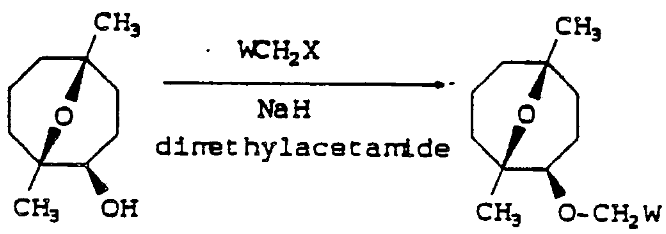


Formula V

Formula II

A general preparation of compounds of Formula VI (Formula Ia with $R_1 = R_2 = CH_3$) is shown in Scheme 2. The first step in this sequence involves the cis-hydroxylation of 1,5-dimethyl-1,5-cyclooctadiene (Formula III) using a catalytic amount of osmium tetroxide with trimethylamine oxide or N-methylmorpholine-N-oxide as stoichiometric oxidant, which is adapted from a literature procedure (R. Ray & D. S. Matteson, Tet. Lett., 21, 449 (1980)). The product glycol of Formula VII is subjected to the Williamson ether coupling reaction with the appropriate alkylating agent $WCH_2X$ in a fashion analogous to the conversion of the alcohol of Formula V to the ether of Formula II in Scheme 1. It is well known in the art that when an unsymmetrically substituted glycol such as Formula VII is subjected to this type of coupling reaction, the new bond is formed selectively with the less substituted hydroxyl group to give a glycol monoether of the type shown as Formula VIII. This glycol monoether can be cyclized with various electrophilic reagents such as sulfuric acid, camphorsulfonic or toluenesulfonic acid, N-chlorosuccinimide, N-bromosuccinimide, phenylselenenyl chloride, phenylsulfenyl chloride, N-phenylselenophthalimide, or meta-chloroperoxybenzoic acid/naphthalenesulfonic acid, to produce 9-oxabicyclo[3.3.1]nonan-2-yl ethers of Formula VI with X= H, Br, Cl, PhS, PhSe or OH.

SCHEME 2

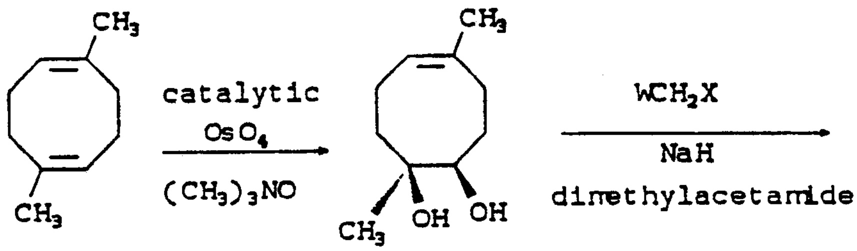


Formula III Formula VII

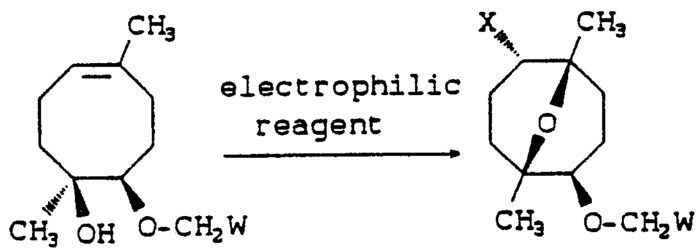


Formula VIII Formula VI

| Electrophilic Reagent = | X = |
|---|---|
| camphorsulfonic or toluene sulfonic acid | H |
| sulfuric acid | H |
| N-chlorosuccinimide | Cl |
| N-bromosuccinimide | Br |
| phenylselenenyl chloride | PhSe |
| 1)m-chloroperoxybenzoic acid, and 2)naphthalenesulfonic acid | OH |
| phenylsulfenyl chloride | PhS |

A general synthesis of the compounds of Formula I in which the groups $R_1$ and $R_2$ may be varied independently is shown in Scheme 3. This pathway begins with the commercially available cyclooctadiene (Formula IX). Application of the hydroboration/oxidation sequence as described in the literature (R. K. Sharma, B. A. Shoulders & P. D. Gardner, Chem. Ind. (London), 2087 (1962); E. F. Knights & H. C. Brown, J. Am. Chem. Soc., 90, 5280 (1968)) leads to cis-1,5-cyclooctandiol, Formula X. Chromic acid oxidation of this diol is known (H. C. Brown & C. P. Garg, J. Am. Chem. Soc., 83, 2951 (1961) to convert selectively only one hydroxyl to the ketone due to the intermediacy of a hemiacetal. Standard tosylation of this intermediate hemiacetal is described (J. K. Crandall, R. D. Huntington & G. L. Brunner, J. Org. Chem., 37, 2911 (1961)) to give the tosyloxy ketone of Formula XI. The transformation of this ketone to the tosyloxycyclooctene bearing the $R_1$ group in the proper position (Formula XII) is effected by addition of the corresponding

Grignard reagent $R_1MgBr$ to the carbonyl group (see Vogel's Textbook of Practical Organic Chemistry, B. S. Furniss, A. J. Hannaford, V. Rogers, P. W. G. Smith & A. R. Tatchell eds., Longman, Inc., N.Y. (1978), pp. 363-375) and subsequent mild dehydration of the resulting tertiary alcohol (see I. Eichorn in Preparative Organic Chemistry, G. Hilgetag & A. Martini eds., Wiley Interscience N.Y. (1972), pp. 813-819)). The tosylate protecting group in Formula XII is cleaved with sodium in liquid ammonia by employing the general procedure reported in the literature (D. B. Denney & B. Goldstein, J. Org. Chem., 21, 479 (1956)), and the resulting alcohol is oxidized with chromic acid to yield the 5-alkylcyclooct-4-en-1-one of Formula XIII. At this stage the $R_2$ group is appended via addition of the corresponding Grignard reagent, $R_2MgBr$, to the carbonyl group in Formula XIII generating a cycloct-4-en-1-ol, which is epoxidized and subjected to acid-catalyzed rearrangement to give the 9-oxabicyclo[3.3.1]nonan-2-ol of Formula XIV, in a fashion analogous to that used in Scheme 1. The target herbicidal 9-oxabicyclo[3.3.1]nonan-2-yl ethers of Formula XV are prepared in the final step via the Williamson Ether coupling reaction with the appropriate alkylating agent $WCH_2X$ as described above for the final step in Scheme 1.

## SCHEME 3

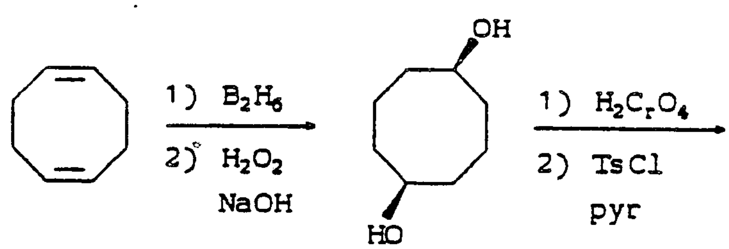


Formula IX Formula X

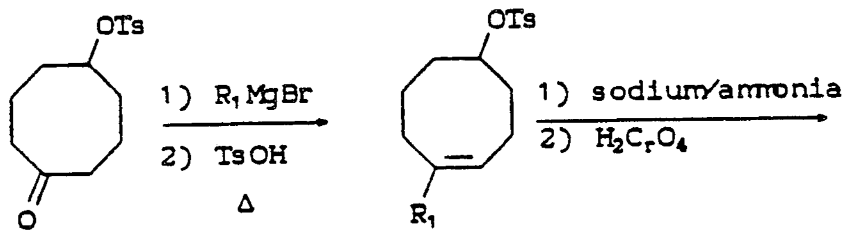


Formula XI

Formula XII

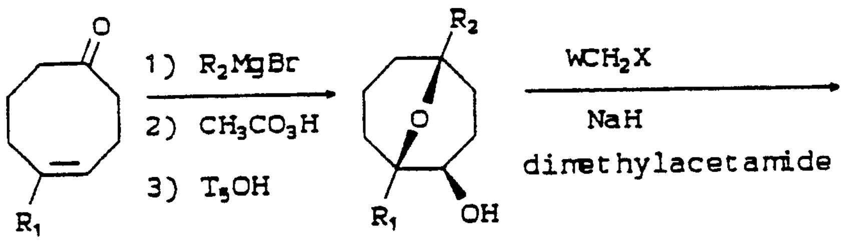


Formula XIII

Formula XIV

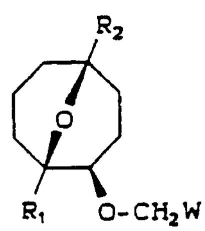

Formula XV

An additional method of preparation for the 9-oxabicyclo[3.3.1]nonan-2-ol of Formula V is shown in Scheme 4. The cyclooctene glycol of Formula VII is selectively acetylated with acetyl chloride in Pyridine to furnish the glycol monoacetate of Formula XVI. This glycol monoacetate is treated with N-bromosuccinimide to bring about an electrophilic ring closure, which provides the bromacetate of Formula XVII. This cyclization reaction can also be carried out with bromine. Reduction of the bromoacetate of Formula XVII with lithium aluminum hydride in tetrahydrofuran at reflux effects both the reductive removal of the bromine atom and the reductive hydrolysis of the acetate ester, and yields the 9-oxabicyclo[3.3.1]nonan-2-ol of Formula V.

The alkenes of Formula Ib may be prepared by the oxidative elimination of the selenides of Formula Ia ($R_3$ = PhSe) using hydrogen peroxide in methylene chloride (by the method reported by H. J. Reich, J. M. Renga and I. L. Reich in J. Am. Chem. Soc., 97, 5434 (1975)).

The ketones of Formula Ic may be prepared by oxidation of the corresponding alcohols of Formula If

($R_4$ = H) with a standard oxidizing agent such as aqueous chromic acid.

The ketones of Formula Id may be prepared by oxidation of the corresponding alcohols of Formula Ih ($R_4$ = H) with a standard oxidizing agent such as aqueous chromic acid.

## SCHEME 4

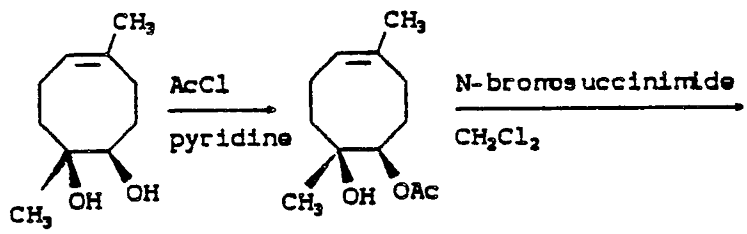


Formula VII Formula XVI

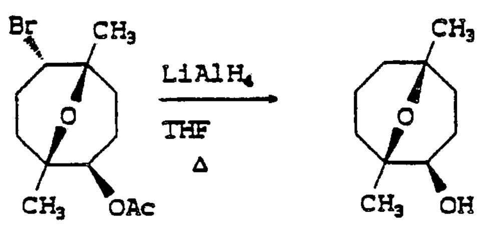


Formula XVII Formula V

An acetonide of Formula Ie would be prepared from the alkene of Formula Ib by the two-step procedure detailed in Scheme 5. The alkene of Formula Ib can be hydroxylated with the catalytic osmylation described earlier in Scheme 2, to give the diol of Formula XVIII. This diol of Formula XVIII can be reacted with 2,2-dimethoxypropane in the presence of acid to provide the acetonide of Formula Ie.

The secondary alcohols of Formula If ($R_3$ = H) can be prepared by the epoxidation of a glycol monoether such as VIII, followed by acid-catalyzed opening, as described in Scheme 2. Tertiary alcohols of Formula If could be prepared by the addition of a suitable Grignard reagent, or hydrogen cyanide, to the ketone of Formula Ic.

The epoxides of Formula Ig can be prepared by epoxidation of the alkenes Ib with a reagent such as meta-chloroperoxybenzoic acid in methylene chloride.

## SCHEME 5

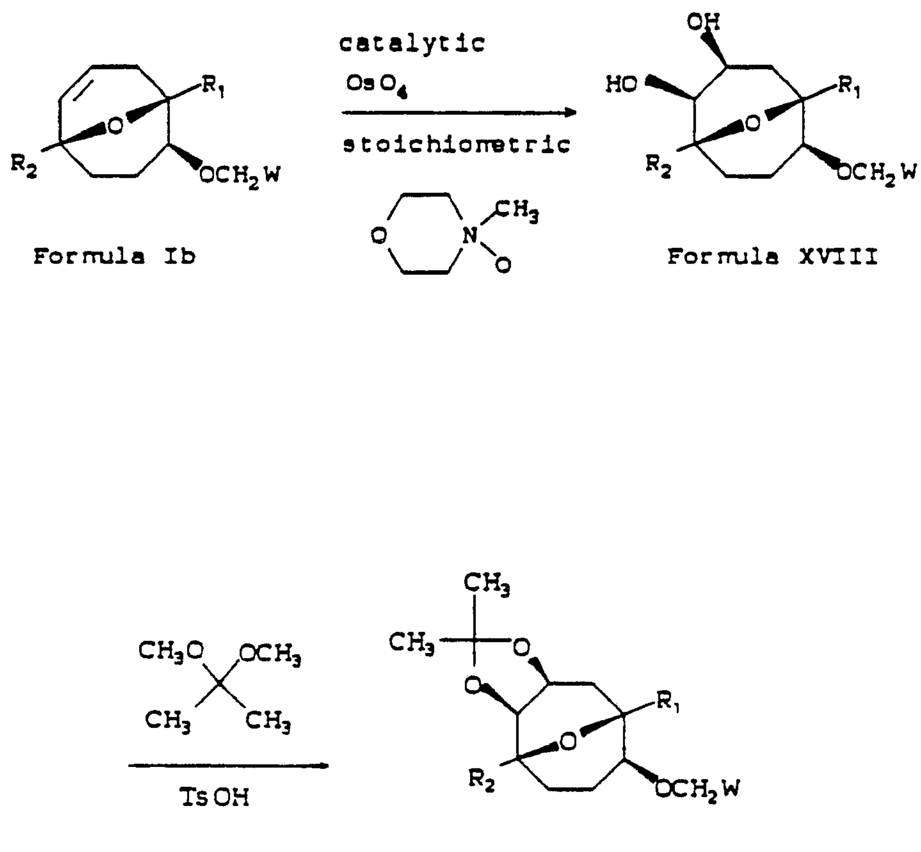


Formula Ib

Formula XVIII

Formula Ie

The alcohols of Formula Ih can be prepared as shown in Scheme 6. The phenylselenides of Formula XIX are prepared as in Scheme 2 from the glycol monoether of Formula VIII with phenylselenenyl chloride. The selenides may be subjected to oxidative elimination in concentrated solution in the absence of base (see H. J. Reich et al. in J Org. Chem., 43, 1697 (1978)), to give a mixture of the alkenes Ib and the vicinal-hydroxy phenylselenyl ethers of Formula XX. The phenylselenyl ethers of Formula XX can undergo reductive removal of the phenylselenyl group with Raney nickel to produce the alcohols of Formula Ih. Tertiary alcohols of Formula Ih will result from the addition of the proper Grignard reagent $R_3MgBr$, or the addition of hydrogen cyanide to the ketones of Formula Id.

## SCHEME 6

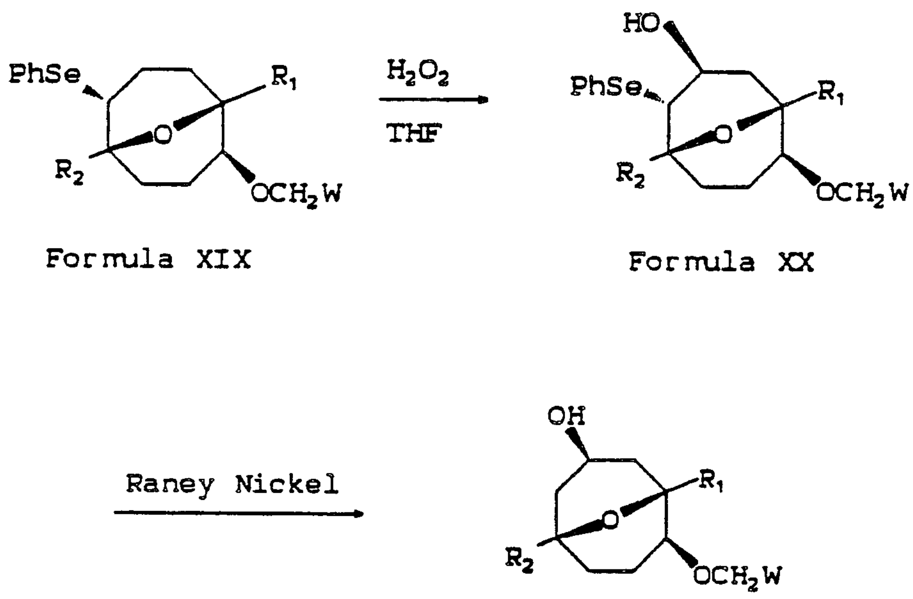


Formula XIX

Formula XX

Formula Ih
($R_4$ = H)

## EXAMPLE 1

(1 R,S; 2 R,S)1,5-dimethyl-9-oxabicyclo[3.3.1]nonan-2-ol and (1 R,S; 2 S,R)1,5-dimethyl9-oxabicyclo[3.3.1]-nonan-2-ol, route 1

A mechanically stirred suspension of sodium carbonate (63.59 g, 0.60 mole) and 1,5-dimethylcycloocta-1,5-diene (40.87 g, 0.30 mole, Formula III) in 400 mL of dry methylene chloride at 0 °C under nitrogen was charged dropwise with a 32% solution of peracetic acid in acetic acid (78.44 g, 0.33 mole) over a period of 1.5 h. The heavy slurry was stirred for a further 2.5 h at 0 °C and then for 1 h at room temperature. The solid was removed by filtration and this was washed with methylene chloride. The combined organic portions were extracted sequentially with 100 mL of water, 100 mL of 5% aqueous sodium carbonate solution, 100 mL of aqueous sodium sulfite solution, 100 mL of water and 50 mL of brine, and was dried over anhydrous magnesium sulfate. Concentration in vacuo gave 46.59 g of crude epoxide which was distilled in vacuo through a 15 cm Vigreux column to yield 5.42 g of recovered 1,5-dimethylcycloocta 1,5-diene, b.p. 75-99 °C/20 mm (13% recovery), and 27.84 g of 1,5-dimethyl-9-oxabicyclo[6.1.0]non-4-ene, b.p. 101-104 °C/20 mm (61% of theory).

$^1$H NMR ($CDCl_3$, 60 MHz). δ 1.27 (s, 3H), 1.70 (br s, 3H), 1.40-2.70 (m, 8H), 2.77 (t, 1H), 5.32 (t, 1H). IR ($CH_2Cl_2$): 2975 (vs, sh), 2890 (vs, sh), 1675 (vw), 1490 (m), 1450 (s), 1380 (s) $cm^{-1}$.

MS (Chem. Ion.) 152.

Analysis Calculated for $C_{10}H_{16}O$:

C, 78.6 H, 10.6%.

Found:

C, 78.0 H, 10.2%.

A magnetically stirred solution of 1,5-dimethyl-9-oxabicyclo[6.1.0]non-4-ene (8.24 g, 54.0 mmol) in 100 mL of dry dimethoxyethane was charged portionwise with lithium aluminum hydride (2.05 g, 54.0 mmol). The resulting solution was stirred and heated to reflux under nitrogen for 19 h. The reaction mixture was cooled and then diluted with 250 mL of ether, and then water was added dropwise to hydrolyze the aluminum complex. The resulting solution was dried over anhydrous magnesium sulfate, and concentration in vacuo gave 11.19 g of crude product which was fractionally distilled through a 15 cm Vigreux column to yield 6.54 g of 1,5-dimethylcyclooct-4-en-1-ol, b.p. 108-110° C/20 mm (79% of theory, Formula IV).

$^1$H NMR ($CDCl_3$, 60 MHz): δ 1.20 (s, 3H), 1.4-2.4 (m 14H), 5.47 (t, 1H).

IR ($CH_2Cl_2$). 3610 (m), 3550 (m) 3475 (m, sh), 2940 (vs), 2880 (s, sh), 1475 (m), 1450 (s), 1380 (s) $cm^{-1}$.

MS (Chem. Ion.) 154.

Analysis Calculated for $C_{10}H_{18}O$:

C, 77.9 H, 11.8%.

Found:

C, 77.8 H, 11.1%.

A magnetically stirred solution of 1,5-dimethylcyclooct-4-en-1-ol (18.46 g, 0.120 mmol) in 390 mL of methylene chloride at 0° C under nitrogen was treated dropwise with 32% peracetic acid in acetic acid (31.4 g, 0.132 mole) over a period of 1 h. The ice bath was removed and the solution was stirred for 2.5 h. The reaction mixture was extracted sequentially with 2 x 100 mL of a 5% aqueous sodium carbonate, 100 mL saturated sodium sulfite solution, 2 x 200 mL water, 100 mL of brine, and the solution was dried over magnesium sulfate. This solution was filtered and cooled to 0° C under nitrogen, and was charged with a solution of p-toluenesulfonic acid (0.46 g, 0.0024 mole) in 10 mL of DME. The ice bath was removed and the mixture was stirred at room temperature for 23 h. The reaction mixture was extracted sequentially with 2 x 100 mL of a 5% aqueous solution of sodium carbonate, 2 x 100 mL of water, 100 mL of brine, and the solution was dried over magnesium sulfate. Removal of solvent in vacuo gave 22.8 g of crude product which was prepurified by column chromatography on silica gel using hexane/ether, 1:1 eluent. The isomeric alcohols were then separated by HPLC with recycling to yield 8.87 g of the endo product (1 R,S; 2 R,S)1,5-dimethyl-9-oxabicyclo[3.3.1]nonan-2-ol (44% of theory) as a colorless oil, and 2.87 g of the exo product (1 R,S; 2 S,R)1,5-dimethyl-9-oxabicyclo[3.3.1]nonan-2-ol (14% of theory, Formula V) as a colorless oil.

For the exo alcohol:

$^1$H NMR ($CDCl_3$, 60 MHz): δ 1.15 (s, 3H), 1.20 (s 3H), 0.8-2.5 (m, 11H), 3.32 (br s, 1H).

IR ($CH_2Cl_2$): 3570 (m), (hydroxyl absorption remains constant upon one-fold dilution), 3465 (m, br), 3050 (w, sh), 2975 (s, sh), 2925 (vs), 1445 (m), 1370 (m) $cm^{-1}$.

MS (Chem. Ion.) 170.

Analysis Calculated for $C_{10}H_{18}O_2$:

C, 70.6 H, 10.7%.

Found:

C, 70.0 H, 11.2%.

For the endo alcohol:

$^1$H NMR ($CDCl_3$, 60 MHz): δ 1.14 (s, 3H), 1.19 (s 3H), 0.8-2.4 (m, 11H), 3.53 (t, 1H).

IR ($CH_2Cl_2$): 3600 (m), 3450 (m, br), (hydroxyl absorption shifts to 3620 $cm^{-1}$ upon one-fold dilution), 3050 (w, sh), 2970 (s, sh), 2930 (vs), 2875 (s, sh), 1450 (m), 1370 (m) $cm^{-1}$.

MS (Chem. Ion.) 170.

Analysis Calculated for $C_{10}H_{18}O_2$:

C, 70.6 H, 10.7%.

Found:

C, 70.0 H, 10.7%.

## EXAMPLE 2

(1 R,S; 2 S,R)1,5-dimethyl-9-oxabicyclo[3.3.1]nonan-2-ol, route 2

A 5 L four neck round bottom flask equipped with a reflux condensor ($N_2$ inlet), mechanical stirrer, thermometer and stopper was charged with 157 mL (0.999 mol) of 1,5-dimethylcycloocta-1,5-diene, 2 L of t-butanol, 230 mL (1.33 mol) of a 60% aqueous solution of N-methylmorpholine N-oxide, 100 mL of water, 85 mL of pyridine and 700 mg (2.75 mmol, 0.28 mole) of osmium tetroxide. The resulting mixture was heated

to reflux for 22 h. The still hot reaction mixture was charged with 800 mL of 20% $NaHSO_3$ (aq), and a distillation head was attached and the reaction vessel was heated. When 2250 mL of distillate had been obtained, the temperature began to rise and the heating was stopped and the mixture was allowed to stand overnight to cool.

The reaction mixture was extracted successively with three 100 mL portions of ether and with 100 mL of methylene chloride. The combined organics were extracted with a mixture of 100 mL of ice and 350 mL of 10% $H_2SO_4$ (aq), and then with 250 mL of brine. The organic portion was diluted with 200 mL of acetone (to prevent early crystallization), and was dried ($MgSO_4$) and concentrated to give 53.125 g of a dull orange viscous liquid which partially crystallized on standing. The crude product was triturated with 200 mL of petroleum ether to give 19.571 g of a white powdery solid identified as cis 1,5-dimethylcyclooct-5-en-1,2-diol. The petroleum ether fraction was concentrated to give 27.937 g of orange liquids which was purified by chromatography on 350 g of silica, using as solvent 20% acetone in petroleum ether, followed by 30% and 50% acetone, to give three fractions. Fraction 1: 4.966 g pale yellow liquid identified by NMR as starting 1,5-dimethylcycloocta-1,5-diene. Fraction 2: 3.244 g of an unidentified pale yellow oil. Fraction 3. 16.549 g of a light yellow viscous liquid which crystallized on standing, identified as cis 1,5-dimethylcyclooct-5-en-1,2-diol. The combined yield for cis 1,5-dimethylcyclooct-5-en-1,2-diol was 36.12 g (21%)% m.p. 72-77° C. $^1H$ NMR ($CDCl_3$, 90 MHz): $\delta$ 1.26 (s, 3H), 1.6-2.6 (m 8H), 2.16 (3, 3H), 3.68 (m, 1H), 5.40 (t, 1H, J = 7 Hz).

A solution of 6.232 g (36.6 mmol) of cis 1,5-dimethylcyclooct-5-en-1,2-diol in 75 mL of pyridine was chilled in an ice/water bath and charged slowly with 3.7 mL of acetyl chloride. Some pinkish solids formed, and this mixture was allowed to come to room temperature and stir for 14 h. The reaction mixture was poured into a mixture of 150 mL of ice and 250 mL of 10% $H_2SO_4$ (aq), and this was extracted with three 40 mL portions of ether. The combined layers were extracted with 100 mL of brine, and were dried ($MgSO_4$) and concentrated to give 7.634 g of crude cis 2-acetoxy-1,5-dimethylcyclooct-5-en-1-ol.

This crude glycol monoacetate was taken up in 100 mL of methylene chloride, and the resulting solution was cooled in a cold water bath and was charged portionwise with 7.168 g (40.3 mmol) of N-bromosuccinimide. After 15 min, the mixture was concentrated to give a white semisolid. The crude product was purified by triturating the semisolid with 5% acetone in petroleum ether and passing this solution through a column of 200 g of silica gel, eluting with the same solvent. The major fraction was isolated as 9.379 g of (1 R,S; 2 S,R; 6 R,S)6-bromo-2-acetoxy-1,5-dimethyl-9-oxabicyclo[3.3.1]nonane as a pale yellow liquid.

A solution of this acetoxybromide in 50 mL of THF was added slowly to a suspension of 7.002 g (185 mmol) of lithium aluminum hydride in 150 mL of THF, and the resulting mixture was heated to reflux for 26 h, then cooled and stood at room temperature for two days, then heated again to reflux for another 18 h. The reaction mixture was cooled and charged slowly with 35 mL of saturated $Na_2SO_4$ (aq), and approximately 3 g of $MgSO_4$. The resulting suspension was vacuum filtered and concentrated to give 6.851 g of a pale yellow liquid, which was purified by chromatography on 150 g of silica with 5% acetone in petroleum ether, followed by 40% and 50% acetone, to give three fractions. Fraction 1: 68 mg of unidentified yellow oil. Fraction 2: 5.604 g (90% yield from the diol) of 9-oxabicyclo′(3.3.1]nonan-2-ol as a pale yellow oil which crystallized on standing. Fraction 3: 888 mg of an unidentified fluffy white solid. Fraction 2 was identical in all respects with the alcohol prepared by other routes.

## EXAMPLE 3

### (1 R,S; 2 S,R)-exo-1,5-Dimethyl-2[(2′-fluorophenyl)methoxy]-9-oxabicyclo[3.3.1]nonane

A magnetically stirred suspension of sodium hydride (0.12 g, 5.02 mmol) in 5 mL of dry dimethylacetamide was charged with a solution of (1 R,S; 2 S,R)1,5-dimethyl-9-oxabicyclo[3.3.1]nonan-2-ol (0.57 g, 3.35 mmol) in 10 mL of dimethylacetamide under nitrogen. The reaction mixture was stirred at room temperature for 30 min and then was heated to 90° C for 1 h. After cooling to 25° C, o-fluorobenzyl chloride (0.53 g, 3.7 mmol) was added and the reaction temperature rose to 33° C. After 14 h at room temperature, the reaction mixture was heated to 90° C for 1.5 h. The solution was cooled, diluted with 50 mL of water and was extracted with 4 x 50 mL of ether. The combined ether layers were extracted with 3 x 50 mL of water, 50 mL of brine and were dried over magnesium sulfate. Concentration *in vacuo* gave 0.95 g of crude product which was purified by HPLC with hexane/ether, 6:1 as eluent to yield 0.50 g of (1 R,S; 2 S,R)1,5-dimethyl-2[(2′-fluorophenyl)methoxyl-9-oxabicyclo[3.3.1]nonane (54% of theory).

$^1H$ NMR ($CDCl_3$, 60 MHz): δ 1.18 (s, 3H), 1.22 (s 3H), 0.8-2.2 (m, 10H), 3.08 (t, 1H), 4.58 (m, 2H), 6.75-7.61 (m, 4H).
IR ($CH_2Cl_2$): 3000 (w, sh), 2940 (s, sh), 2915 (vs), 2855 (s, sh), 1600 (m), 1570 (m) 1475 (s), 1435 (s), 1050 (vs) $cm^{-1}$.
MS (Chem. Ion.) 278.
Analysis Calculated for $C_{17}H_{23}FO_2$:
C, 73.4 H, 8.3%.
Found:
C, 73.4 H, 8.7%.
Elution of the column with ether produced 0.11 g of the starting exo 1,5-dimethyl-9-oxabicyclo[3.3.1]nonan-2-ol.

## EXAMPLE 4

(1 R,S; 2 S,R)-1,5-Dimethyl-2[(2′,6′-difluorophenyl)methoxy]-9-oxabicyclo[3.3.1]nonane

A suspension of 620 mg (15.5 mmol) of 60% sodium hydride oil dispersion in 12 mL of THF was charged with a solution of 600 mg (3.52 mmol) of exo 1,5-dimethyl-9-oxabicyclo[3.3.1]nonan-2-ol in a total of 4 mL of THF, and this was diluted with 8 mL of DMAC. This mixture was charged with 804 mg (3.88 mmol) of 2,6-difluorobenzyl bromide. The resulting mixture was heated to reflux for 9 h, and then was stood at room temperature for another 10 h. The reaction mixture was poured into a mixture of 75 mL of ice and 100 mL of 10% $H_2SO_4$ (aq), and this mixture was extracted three times with 30 mL of ether. The combined organic portions were extracted with 100 mL of brine, and were dried ($MgSO_4$) and concentrated t give 1.377 g of orange oil. This crude product was chromatographed on 40 g of silica with 3% acetone in petroleum ether, increasing to 6% acetone, to yield two fractions. The first fraction was 583 mg (56% yield) of 1 R,S; 2 S,R)-exo-1,5-dimethyl-2[(2′,6′-difluorophenyl)methoxy]-9-oxabicyclo[3.3.1]nonane.
$^1H$ NMR ($CDCl_3$, 200 MHz): δ (1.14 (s, 3), 1.16) (s 3H), 1.35-1.37 (m, 6H), 1.7-2.2 (m, 4H), 3.04 (t, 1H, J=2.8 Hz), 4.49 (ddt, 1H, J=6.2, 11, 1.2 Hz), 4.70 (dt, 1H, J=11, 1.5 Hz), 6.8-7.0 (m, 2H), 7.15-7.35 (m, 1H).
$^{13}C$-NMR ($CDCl_3$, 50 MHz) δ 19.5, 23.8, 26.8, 30.6, 31.7, 33.0, 34.8, 58.0 (t, J=3.1 Hz), 69.8, 72.8, 76.7, 110.09 (d, J=25.9 Hz), 110.97 (dd, J=3.1, 6.8 Hz), 129.8 (t, J=10.4 Hz), 162.0 (dd, J=8.1, 248 Hz).
MS (Chem. Ion.) 296.

By the general procedures described or by obvious modifications thereof, the compounds of Tables 1 through 14 may be prepared.

TABLE 1

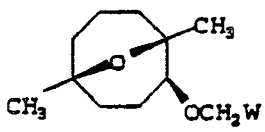

where W =

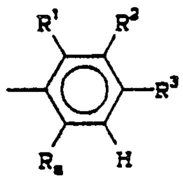

$R_a$ is H

| $R^1$ | $R^2$ | $R^3$ | $R^1$ | $R^2$ | $R^3$ |
|---|---|---|---|---|---|
| H | H | H | $(CH_2)_2CH_3$ | H | H |
| F | H | H | H | $(CH_2)_2CH_3$ | H |
| H | F | H | H | H | $(CH_2)_2CH_3$ |
| H | H | F | $CH(CH_3)_2$ | H | H |
| Cl | H | H | H | $CH(CH_3)_2$ | H |
| H | Cl | H | H | H | $CH(CH_3)_2$ |
| H | H | Cl | $OCH_3$ | H | H |
| Br | H | H | H | $OCH_3$ | H |
| H | Br | H | H | H | $OCH_3$ |
| H | H | Br | $OCH_2CH_3$ | H | H |
| OH | H | H | H | $OCH_2CH_3$ | H |
| H | OH | H | H | H | $OCH_2CH_3$ |
| H | H | OH | $O(CH_2)_2CH_3$ | H | H |
| CN | H | H | H | $O(CH_2)_2CH_3$ | H |
| H | CN | H | H | H | $O(CH_2)_2CH_3$ |
| H | H | CN | $OCH(CH_3)_2$ | H | H |
| $CH_3$ | H | H | H | $OCH(CH_3)_2$ | H |
| H | $CH_3$ | H | H | H | $OCH(CH_3)_2$ |
| H | H | $CH_3$ | $SCH_3$ | H | H |
| $CH_2CH_3$ | H | H | H | $SCH_3$ | H |
| H | $CH_2CH_3$ | H | H | H | $SCH_3$ |
| H | H | $CH_2CH_3$ | $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $R^1$ | $R^2$ | $R^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| E$CH=CHCH_3$ | H | H |
| H | E$CH=CHCH_3$ | H |
| H | H | E$CH=CHCH_3$ |
| Z$CH=CHCH_3$ | H | H |
| H | Z$CH=CHCH_3$ | H |
| H | H | Z$CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| E$CH=CHCH_2CH_3$ | H | H |
| H | E$CH=CHCH_2CH_3$ | H |
| H | H | E$CH=CHCH_2CH_3$ |
| Z$CH=CHCH_2CH_3$ | H | H |
| H | Z$CH=CHCH_2CH_3$ | H |
| H | H | Z$CH=CHCH_2CH_3$ |
| E$CH_2CH=CHCH_3$ | H | H |
| H | E$CH_2CH=CHCH_3$ | H |
| H | H | E$CH_2CH=CHCH_3$ |
| Z$CH_2CH=CHCH_3$ | H | H |
| H | Z$CH_2CH=CHCH_3$ | H |
| H | H | Z$CH_2CH=CHCH_3$ |

| $\underline{R}^1$ | $\underline{R}^2$ | $\underline{R}^3$ |
|---|---|---|
| $(CH_2)_2CH=CH_2$ | H | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH_2$ |
| $\underline{E}C(CH_3)=CHCH_3$ | H | H |
| H | $\underline{E}C(CH_3)=CHCH_3$ | H |
| H | H | $\underline{E}C(CH_3)=CHCH_3$ |
| $\underline{Z}C(CH_3)=CHCH_3$ | H | H |
| H | $\underline{Z}C(CH_3)=CHCH_3$ | H |
| H | H | $\underline{Z}C(CH_3)=CHCH_3$ |
| $CH_2C(CH_3)=CH_2$ | H | H |
| H | $CH_2C(CH_3)=CH_2$ | H |
| H | H | $CH_2C(CH_3)=CH_2$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is F

| $R^1$ | $R^2$ | $R^3$ | $R^1$ | $R^2$ | $R^3$ |
|---|---|---|---|---|---|
| | | | $(CH_2)_2CH_3$ | H | H |
| F | H | H | H | $(CH_2)_2CH_3$ | H |
| H | F | H | H | H | $(CH_2)_2CH_3$ |
| H | H | F | $CH(CH_3)_2$ | H | H |
| Cl | H | H | H | $CH(CH_3)_2$ | H |
| H | Cl | H | H | H | $CH(CH_3)_2$ |
| H | H | Cl | $OCH_3$ | H | H |
| Br | H | H | H | $OCH_3$ | H |
| H | Br | H | H | H | $OCH_3$ |
| H | H | Br | $OCH_2CH_3$ | H | H |
| OH | H | H | H | $OCH_2CH_3$ | H |
| H | OH | H | H | H | $OCH_2CH_3$ |
| H | H | OH | $O(CH_2)_2CH_3$ | H | H |
| CN | H | H | H | $O(CH_2)_2CH_3$ | H |
| H | CN | H | H | H | $O(CH_2)_2CH_3$ |
| H | H | CN | $OCH(CH_3)_2$ | H | H |
| $CH_3$ | H | H | H | $OCH(CH_3)_2$ | H |
| H | $CH_3$ | H | H | H | $OCH(CH_3)_2$ |
| H | H | $CH_3$ | $SCH_3$ | H | H |
| $CH_2CH_3$ | H | H | H | $SCH_3$ | H |
| H | $CH_2CH_3$ | H | H | H | $SCH_3$ |
| H | H | $CH_2CH_3$ | $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |
| $\underline{Z}CH_2CH=CHCH_3$ | H | H |
| H | $\underline{Z}CH_2CH=CHCH_3$ | H |
| H | H | $\underline{Z}CH_2CH=CHCH_3$ |

| $R^1$ | $R^2$ | $R^3$ |
|---|---|---|
| $(CH_2)_2CH=CH_2$ | H | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH_2$ |
| $\underline{E}C(CH_3)=CHCH_3$ | H | H |
| H | $\underline{E}C(CH_3)=CHCH_3$ | H |
| H | H | $\underline{E}C(CH_3)=CHCH_3$ |
| $\underline{Z}C(CH_3)=CHCH_3$ | H | H |
| H | $\underline{Z}C(CH_3)=CHCH_3$ | H |
| H | H | $\underline{Z}C(CH_3)=CHCH_3$ |
| $CH_2C(CH_3)=CH_2$ | H | H |
| H | $CH_2C(CH_3)=CH_2$ | H |
| H | H | $CH_2C(CH_3)=CH_2$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is Cl

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |

| $R^1$ | $R^2$ | $R^3$ |
|---|---|---|
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |
| $\underline{Z}CH_2CH=CHCH_3$ | H | H |
| H | $\underline{Z}CH_2CH=CHCH_3$ | H |
| H | H | $\underline{Z}CH_2CH=CHCH_3$ |

| $R^1$ | $R^2$ | $R^3$ |
|---|---|---|
| $(CH_2)_2CH=CH_2$ | H | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH_2$ |
| EC$(CH_3)=CHCH_3$ | H | H |
| H | EC$(CH_3)=CHCH_3$ | H |
| H | H | EC$(CH_3)=CHCH_3$ |
| ZC$(CH_3)=CHCH_3$ | H | H |
| H | ZC$(CH_3)=CHCH_3$ | H |
| H | H | ZC$(CH_3)=CHCH_3$ |
| $CH_2C(CH_3)=CH_2$ | H | H |
| H | $CH_2C(CH_3)=CH_2$ | H |
| H | H | $CH_2C(CH_3)=CH_2$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is Br

| $R^1$ | $R^2$ | $R^3$ | $R^1$ | $R^2$ | $R^3$ |
|---|---|---|---|---|---|
| F | H | H | $OCH_2CH_3$ | H | H |
| H | F | H | H | $OCH_2CH_3$ | H |
| H | H | F | H | H | $OCH_2CH_3$ |
| Cl | H | H | $O(CH_2)_2CH_3$ | H | H |
| H | Cl | H | H | $O(CH_2)_2CH_3$ | H |
| H | H | Cl | H | H | $O(CH_2)_2CH_3$ |
| Br | H | H | $OCH(CH_3)_2$ | H | H |
| H | Br | H | H | $OCH(CH_3)_2$ | H |
| H | H | Br | H | H | $OCH(CH_3)_2$ |
| OH | H | H | $SCH_3$ | H | H |
| H | OH | H | H | $SCH_3$ | H |
| H | H | OH | H | H | $SCH_3$ |
| CN | H | H | $SCH_2CH_3$ | H | H |
| H | CN | H | H | $SCH_2CH_3$ | H |
| H | H | CN | H | H | $SCH_2CH_3$ |
| $CH_3$ | H | H | $S(CH_2)_2CH_3$ | H | H |
| H | $CH_3$ | H | H | $S(CH_2)_2CH_3$ | H |
| H | H | $CH_3$ | H | H | $S(CH_2)_2CH_3$ |
| $CH_2CH_3$ | H | H | $SCH(CH_3)_2$ | H | H |
| H | $CH_2CH_3$ | H | H | $SCH(CH_3)_2$ | H |
| H | H | $CH_2CH_3$ | H | H | $SCH(CH_3)_2$ |
| $(CH_2)_2CH_3$ | H | H | | | |
| H | $(CH_2)_2CH_3$ | H | | | |
| H | H | $(CH_2)_2CH_3$ | | | |
| $CH(CH_3)_2$ | H | H | | | |
| H | $CH(CH_3)_2$ | H | | | |
| H | H | $CH(CH_3)_2$ | | | |
| $OCH_3$ | H | H | | | |
| H | $OCH_3$ | H | | | |
| H | H | $OCH_3$ | | | |

$R_a$ is OH

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3H$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

$R_a$ is CN

| $R^1$ | $R^2$ | $R^3$ | $R^1$ | $R^2$ | $R^3$ |
|---|---|---|---|---|---|
| | | | $(CH_2)_2CH_3$ | H | H |
| F | H | H | H | $(CH_2)_2CH_3$ | H |
| H | F | H | H | H | $(CH_2)_2CH_3$ |
| H | H | F | $CH(CH_3)_2$ | H | H |
| Cl | H | H | H | $CH(CH_3)_2$ | H |
| H | Cl | H | H | H | $CH(CH_3)_2$ |
| H | H | Cl | $OCH_3$ | H | H |
| Br | H | H | H | $OCH_3$ | H |
| H | Br | H | H | H | $OCH_3$ |
| H | H | Br | $OCH_2CH_3$ | H | H |
| OH | H | H | H | $OCH_2CH_3$ | H |
| H | OH | H | H | H | $OCH_2CH_3$ |
| H | H | OH | $O(CH_2)_2CH_3$ | H | H |
| CN | H | H | H | $O(CH_2)_2CH_3$ | H |
| H | CN | H | H | H | $O(CH_2)_2CH_3$ |
| H | H | CN | $OCH(CH_3)_2$ | H | H |
| $CH_3$ | H | H | H | $OCH(CH_3)_2$ | H |
| H | $CH_3$ | H | H | H | $OCH(CH_3)_2$ |
| H | H | $CH_3$ | $SCH_3$ | H | H |
| $CH_2CH_3$ | H | H | H | $SCH_3$ | H |
| H | $CH_2CH_3$ | H | H | H | $SCH_3$ |
| H | H | $CH_2CH_3$ | $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |

$R_a$ is $CH_3$

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |

| $R^1$ | $R^2$ | $R^3$ |
|---|---|---|
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |
| $\underline{Z}CH_2CH=CHCH_3$ | H | H |
| H | $\underline{Z}CH_2CH=CHCH_3$ | H |
| H | H | $\underline{Z}CH_2CH=CHCH_3$ |

| $R^1$ | $R^2$ | $R^3$ |
|---|---|---|
| $(CH_2)_2CH=CH_2$ | H. | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH_2$ |
| $\underline{E}C(CH_3)=CHCH_3$ | H | H |
| H | $\underline{E}C(CH_3)=CHCH_3$ | H |
| H | H | $\underline{E}C(CH_3)=CHCH_3$ |
| $\underline{Z}C(CH_3)=CHCH_3$ | H | H |
| H | $\underline{Z}C(CH_3)=CHCH_3$ | H |
| H | H | $\underline{Z}C(CH_3)=CHCH_3$ |
| $CH_2C(CH_3)=CH_2$ | H | H |
| H | $CH_2C(CH_3)=CH_2$ | H |
| H | H | $CH_2C(CH_3)=CH_2$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is $OCH_3$

| $R^1$ | $R^2$ | $R^3$ | $R^1$ | $R^2$ | $R^3$ |
|---|---|---|---|---|---|
| | | | $(CH_2)_2CH_3$ | H | H |
| F | H | H | H | $(CH_2)_2CH_3$ | H |
| H | F | H | H | H | $(CH_2)_2CH_3$ |
| H | H | F | $CH(CH_3)_2$ | H | H |
| Cl | H | H | H | $CH(CH_3)_2$ | H |
| H | Cl | H | H | H | $CH(CH_3)_2$ |
| H | H | Cl | $OCH_3$ | H | H |
| Br | H | H | H | $OCH_3$ | H |
| H | Br | H | H | H | $OCH_3$ |
| H | H | Br | $OCH_2CH_3$ | H | H |
| OH | H | H | H | $OCH_2CH_3$ | H |
| H | OH | H | H | H | $OCH_2CH_3$ |
| H | H | OH | $O(CH_2)_2CH_3$ | H | H |
| CN | H | H | H | $O(CH_2)_2CH_3$ | H |
| H | CN | H | H | H | $O(CH_2)_2CH_3$ |
| H | H | CN | $OCH(CH_3)_2$ | H | H |
| $CH_3$ | H | H | H | $OCH(CH_3)_2$ | H |
| H | $CH_3$ | H | H | H | $OCH(CH_3)_2$ |
| H | H | $CH_3$ | $SCH_3$ | H | H |
| $CH_2CH_3$ | H | H | H | $SCH_3$ | H |
| H | $CH_2CH_3$ | H | H | H | $SCH_3$ |
| H | H | $CH_2CH_3$ | $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |
| $\underline{Z}CH_2CH=CHCH_3$ | H | H |
| H | $\underline{Z}CH_2CH=CHCH_3$ | H |
| H | H | $\underline{Z}CH_2CH=CHCH_3$ |

| $\underline{R}^1$ | $\underline{R}^2$ | $\underline{R}^3$ |
|---|---|---|
| $(CH_2)_2CH=CH_2$ | H | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH_2$ |
| $\underline{E}C(CH_3)=CHCH_3$ | H | H |
| H | $\underline{E}C(CH_3)=CHCH_3$ | H |
| H | H | $\underline{E}C(CH_3)=CHCH_3$ |
| $\underline{Z}C(CH_3)=CHCH_3$ | H | H |
| H | $\underline{Z}C(CH_3)=CHCH_3$ | H |
| H | H | $\underline{Z}C(CH_3)=CHCH_3$ |
| $CH_2C(CH_3)=CH_2$ | H | H |
| H | $CH_2C(CH_3)=CH_2$ | H |
| H | H | $CH_2C(CH_3)=CH_2$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is $SCH_3$

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

$R_a$ is $CFH_2$

| $R^1$ | $R^2$ | $R^3$ | $R^1$ | $R^2$ | $R^3$ |
|---|---|---|---|---|---|
| | | | $(CH_2)_2CH_3$ | H | H |
| F | H | H | H | $(CH_2)_2CH_3$ | H |
| H | F | H | H | H | $(CH_2)_2CH_3$ |
| H | H | F | $CH(CH_3)_2$ | H | H |
| Cl | H | H | H | $CH(CH_3)_2$ | H |
| H | Cl | H | H | H | $CH(CH_3)_2$ |
| H | H | Cl | $OCH_3$ | H | H |
| Br | H | H | H | $OCH_3$ | H |
| H | Br | H | H | H | $OCH_3$ |
| H | H | Br | $OCH_2CH_3$ | H | H |
| OH | H | H | H | $OCH_2CH_3$ | H |
| H | OH | H | H | H | $OCH_2CH_3$ |
| H | H | OH | $O(CH_2)_2CH_3$ | H | H |
| CN | H | H | H | $O(CH_2)_2CH_3$ | H |
| H | CN | H | H | H | $O(CH_2)_2CH_3$ |
| H | H | CN | $OCH(CH_3)_2$ | H | H |
| $CH_3$ | H | H | H | $OCH(CH_3)_2$ | H |
| H | $CH_3$ | H | H | H | $OCH(CH_3)_2$ |
| H | H | $CH_3$ | $SCH_3$ | H | H |
| $CH_2CH_3$ | H | H | H | $SCH_3$ | H |
| H | $CH_2CH_3$ | H | H | H | $SCH_3$ |
| H | H | $CH_2CH_3$ | $SCH_2CH_3$ | H | H |

| $R^1$ | $R^2$ | $R^3$ | $R^1$ | $R^2$ | $R^3$ |
|---|---|---|---|---|---|
| H | $SCH_2CH_3$ | H | $F_2CH$ | H | H |
| H | H | $SCH_2CH_3$ | H | $F_2CH$ | H |
| $S(CH_2)_2CH_3$ | H | H | H | H | $F_2CH$ |
| H | $S(CH_2)_2CH_3$ | H | $F_2CHCH_2$ | H | H |
| H | H | $S(CH_2)_2CH_3$ | H | $F_2CHCH_2$ | H |
| $SCH(CH_3)_2$ | H | H | H | H | $F_2CHCH_2$ |
| H | $SCH(CH_3)_2$ | H | $CF_3(CH_3)CH$ | H | H |
| H | H | $SCH(CH_3)_2$ | H | $CF_3(CH_3)CH$ | H |
| $FCH_2$ | H | H | H | H | $CF_3(CH_3)CH$ |
| H | $FCH_2$ | H | $CF_3$ | H | H |
| H | H | $FCH_2$ | H | $CF_3$ | H |
| $ClCH_2$ | H | H | H | H | $CF_3$ |
| H | $ClCH_2$ | H | $CCl_3$ | H | H |
| H | H | $ClCH_2$ | H | $CCl_3$ | H |
| $BrCH_2$ | H | H | H | H | $CCl_3$ |
| H | $BrCH_2$ | H | $OCF_2H$ | H | H |
| H | H | $BrCH_2$ | H | $OCF_2H$ | H |
| $F(CH_2)_2$ | H | H | H | H | $OCF_2H$ |
| H | $F(CH_2)_2$ | H | $OCF_3$ | H | H |
| H | H | $F(CH_2)_2$ | H | $OCF_3$ | H |
| $CH_3CFH$ | H | H | H | H | $OCF_3$ |
| H | $CH_3CFH$ | H | $O(CH_2)_2Cl$ | H | H |
| H | H | $CH_3CFH$ | H | $O(CH_2)_2Cl$ | H |
| $F(CH_2)_3$ | H | H | H | H | $O(CH_2)_2Cl$ |
| H | $F(CH_2)_3$ | H | $OCH_2CHF_2$ | H | H |
| H | H | $F(CH_2)_3$ | H | $OCH_2CHF_2$ | H |
| $CH_3CHClCH_2$ | H | H | H | H | $OCH_2CHF_2$ |
| H | $CH_3CHClCH_2$ | H | $OCH_2CF_3$ | H | H |
| H | H | $CH_3CHClCH_2$ | H | $OCH_2CF_3$ | H |
| $(CH_3)_2CBr$ | H | H | H | H | $OCH_2CF_3$ |
| H | $(CH_3)_2CBr$ | H | $OCH_2CCl_3$ | H | H |
| H | H | $(CH_3)_2CBr$ | H | $OCH_2CCl_3$ | H |
| $CH(CH_3)CH_2F$ | H | H | H | H | $OCH_2CCl_3$ |
| H | $CH(CH_3)CH_2F$ | H | $O(CH_2)_3F$ | H | H |
| H | H | $CH(CH_3)CH_2F$ | H | $O(CH_2)_3F$ | H |

| $\underline{R}^1$ | $\underline{R}^2$ | $\underline{R}^3$ |
|---|---|---|
| H | H | $O(CH_2)_3F$ |
| $OCH_2CHClCH_3$ | H | H |
| H | $OCH_2CHClCH_3$ | H |
| H | H | $OCH_2CHClCH_3$ |
| $OCH(CH_3)CH_2F$ | H | H |
| H | $OCH(CH_3)CH_2F$ | H |
| H | H | $OCH(CH_3)CH_2F$ |
| $CH=CH_2$ | H | H |
| H | $CH=CH_2$ | H |
| H | H | $CH=CH_2$ |
| $\underline{E}CH=CHCH_3$ | H | H |
| H | $\underline{E}CH=CHCH_3$ | H |
| H | H | $\underline{E}CH=CHCH_3$ |
| $\underline{Z}CH=CHCH_3$ | H | H |
| H | $\underline{Z}CH=CHCH_3$ | H |
| H | H | $\underline{Z}CH=CHCH_3$ |
| $CH_2CH=CH_2$ | H | H |
| H | $CH_2CH=CH_2$ | H |
| H | H | $CH_2CH=CH_2$ |
| $C(CH_3)=CH_2$ | H | H |
| H | $C(CH_3)=CH_2$ | H |
| H | H | $C(CH_3)=CH_2$ |
| $\underline{E}CH=CHCH_2CH_3$ | H | H |
| H | $\underline{E}CH=CHCH_2CH_3$ | H |
| H | H | $\underline{E}CH=CHCH_2CH_3$ |
| $\underline{Z}CH=CHCH_2CH_3$ | H | H |
| H | $\underline{Z}CH=CHCH_2CH_3$ | H |
| H | H | $\underline{Z}CH=CHCH_2CH_3$ |
| $\underline{E}CH_2CH=CHCH_3$ | H | H |
| H | $\underline{E}CH_2CH=CHCH_3$ | H |
| H | H | $\underline{E}CH_2CH=CHCH_3$ |
| $\underline{Z}CH_2CH=CHCH_3$ | H | H |
| H | $\underline{Z}CH_2CH=CHCH_3$ | H |
| H | H | $\underline{Z}CH_2CH=CHCH_3$ |

| $R^1$ | $R^2$ | $R^3$ |
|---|---|---|
| $(CH_2)_2CH=CH_2$ | H | H |
| H | $(CH_2)_2CH=CH_2$ | H |
| H | H | $(CH_2)_2CH=CH_2$ |
| $CH(CH_3)CH=CH_2$ | H | H |
| H | $CH(CH_3)CH=CH_2$ | H |
| H | H | $CH(CH_3)CH=CH$ |
| $EC(CH_3)=CHCH_3$ | H | H |
| H | $EC(CH_3)=CHCH_3$ | H |
| H | H | $EC(CH_3)=CHCH$ |
| $ZC(CH_3)=CHCH_3$ | H | H |
| H | $ZC(CH_3)=CHCH_3$ | H |
| H | H | $ZC(CH_3)=CHCH$ |
| $CH_2C(CH_3)=CH_2$ | H | H |
| H | $CH_2C(CH_3)=CH_2$ | H |
| H | H | $CH_2C(CH_3)=CH$ |
| $CH=C(CH_3)_2$ | H | H |
| H | $CH=C(CH_3)_2$ | H |
| H | H | $CH=C(CH_3)_2$ |
| $C\equiv CH$ | H | H |
| H | $C\equiv CH$ | H |
| H | H | $C\equiv CH$ |
| $C\equiv CCH_3$ | H | H |
| H | $C\equiv CCH_3$ | H |
| H | H | $C\equiv CCH_3$ |
| $CH_2C\equiv CH$ | H | H |
| H | $CH_2C\equiv CH$ | H |
| H | H | $CH_2C\equiv CH$ |
| $C\equiv CCH_2CH_3$ | H | H |
| H | $C\equiv CCH_2CH_3$ | H |
| H | H | $C\equiv CCH_2CH_3$ |
| $CH_2C\equiv CCH_3$ | H | H |
| H | $CH_2C\equiv CCH_3$ | H |
| H | H | $CH_2C\equiv CCH_3$ |
| $(CH_2)_2C\equiv CH$ | H | H |
| H | $(CH_2)_2C\equiv CH$ | H |
| H | H | $(CH_2)_2C\equiv CH$ |

$R_a$ is $CF_3$

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

$R_a$ is $OCF_2H$

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

$R_a$ is $CH_2$=CH

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

$R_a$ is C≡CH

| $R^1$ | $R^2$ | $R^3$ |
|---|---|---|
| F | H | H |
| H | F | H |
| H | H | F |
| Cl | H | H |
| H | Cl | H |
| H | H | Cl |
| Br | H | H |
| H | Br | H |
| H | H | Br |
| OH | H | H |
| H | OH | H |
| H | H | OH |
| CN | H | H |
| H | CN | H |
| H | H | CN |
| $CH_3$ | H | H |
| H | $CH_3$ | H |
| H | H | $CH_3$ |
| $CH_2CH_3$ | H | H |
| H | $CH_2CH_3$ | H |
| H | H | $CH_2CH_3$ |
| $(CH_2)_2CH_3$ | H | H |
| H | $(CH_2)_2CH_3$ | H |
| H | H | $(CH_2)_2CH_3$ |
| $CH(CH_3)_2$ | H | H |
| H | $CH(CH_3)_2$ | H |
| H | H | $CH(CH_3)_2$ |
| $OCH_3$ | H | H |
| H | $OCH_3$ | H |
| H | H | $OCH_3$ |

| $R^1$ | $R^2$ | $R^3$ |
|---|---|---|
| $OCH_2CH_3$ | H | H |
| H | $OCH_2CH_3$ | H |
| H | H | $OCH_2CH_3$ |
| $O(CH_2)_2CH_3$ | H | H |
| H | $O(CH_2)_2CH_3$ | H |
| H | H | $O(CH_2)_2CH_3$ |
| $OCH(CH_3)_2$ | H | H |
| H | $OCH(CH_3)_2$ | H |
| H | H | $OCH(CH_3)_2$ |
| $SCH_3$ | H | H |
| H | $SCH_3$ | H |
| H | H | $SCH_3$ |
| $SCH_2CH_3$ | H | H |
| H | $SCH_2CH_3$ | H |
| H | H | $SCH_2CH_3$ |
| $S(CH_2)_2CH_3$ | H | H |
| H | $S(CH_2)_2CH_3$ | H |
| H | H | $S(CH_2)_2CH_3$ |
| $SCH(CH_3)_2$ | H | H |
| H | $SCH(CH_3)_2$ | H |
| H | H | $SCH(CH_3)_2$ |

TABLE 2

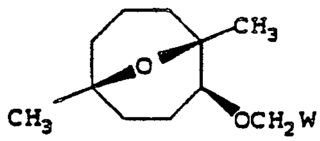

where W =

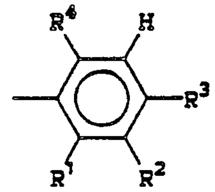

| $R^1$ | $R^2$ | $R^3$ | $R^4$ |
|---|---|---|---|
| F | F | H | F |
| F | F | F | H |
| F | H | F | F |
| F | $CH_3$ | H | F |
| $CH_3$ | F | H | F |
| Cl | F | H | F |
| Br | F | H | F |
| Cl | F | F | H |
| CN | H | F | F |
| CN | F | H | F |
| F | Cl | H | F |
| F | Cl | F | H |
| F | Br | H | F |
| F | Br | F | H |
| Cl | F | H | Cl |
| Cl | $CH_3$ | H | Cl |
| Br | $CH_3$ | H | F |
| $OCH_3$ | F | H | F |
| $OCH_3$ | F | F | H |
| $OCH_3$ | F | H | Cl |
| $OCH_3$ | Cl | H | F |
| CN | Br | H | F |
| CN | F | H | Br |
| CN | F | H | $CH_3$ |
| CN | Cl | H | Br |
| CN | Cl | H | $CH_3$ |
| CN | F | H | Cl |
| CN | F | H | $OCH_3$ |
| Br | F | H | $OCH_3$ |
| $CH_3$ | F | H | Br |
| Br | F | H | F |
| Br | F | F | H |
| $SCH_3$ | F | H | F |
| F | H | F | $CH=CH_2$ |
| $CF_3$ | F | H | F |
| $CF_3$ | F | H | Cl |
| $CH_3$ | F | H | $CF_3$ |

TABLE KEY - TABLES 3-7

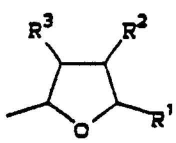

W-1

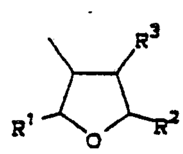

W-2

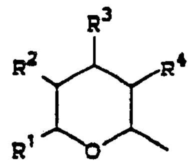

W-3

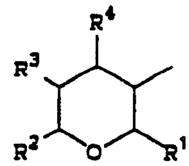

W-4

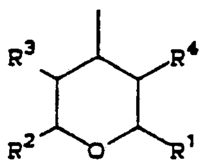

W-5

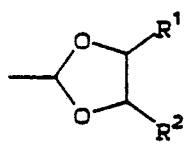

W-6

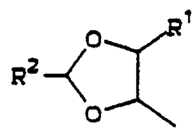

W-7

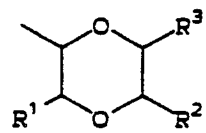

W-8

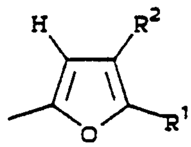

W-9

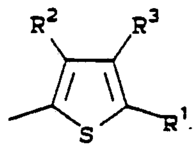

W-10

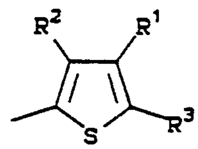

W-11

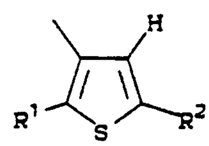

W-12

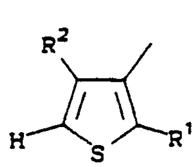

W-13

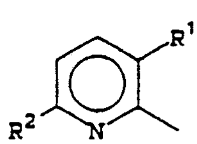

W-14

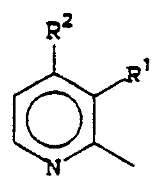

W-15

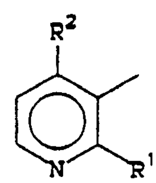

W-16

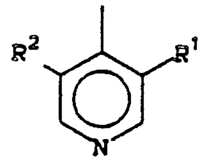
W-17

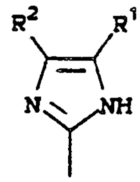
W-18

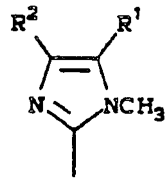
W-19

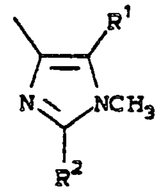
W-20

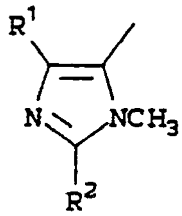
W-21

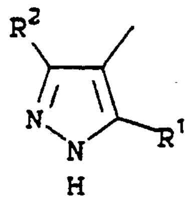
W-22

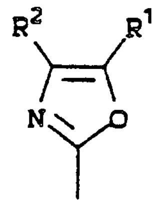
W-23

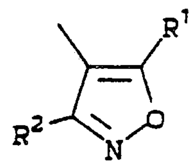
W-24

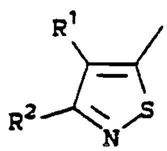
W-25

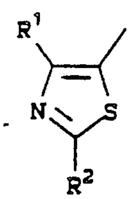
W-26

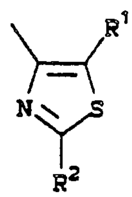
W-27

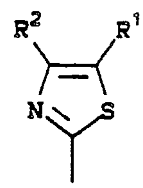
W-28

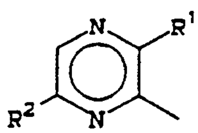
W-29

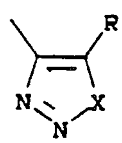
W-30

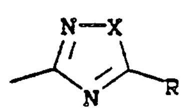
W-31

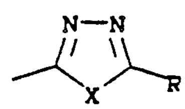
W-32

TABLE 3

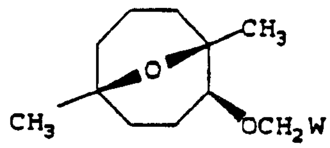

W = W-1

| $R^1$ | $R^2$ | $R^3$ | $R^1$ | $R^2$ | $R^3$ |
|---|---|---|---|---|---|
| H | H | $CH_3$ | H | Br | $OCH_3$ |
| H | H | $OCH_3$ | H | Br | Cl |
| H | H | Cl | H | Br | Br |
| H | H | Br | $CH_3$ | H | H |
| H | $CH_3$ | H | $CH_3$ | H | $CH_3$ |
| H | $CH_3$ | $CH_3$ | $CH_3$ | H | $OCH_3$ |
| H | $CH_3$ | $OCH_3$ | $CH_3$ | H | Cl |
| H | $CH_3$ | Cl | $CH_3$ | H | Br |
| H | $CH_3$ | Br | $CH_3$ | $CH_3$ | H |
| H | $OCH_3$ | H | $CH_3$ | $OCH_3$ | H |
| H | $OCH_3$ | $CH_3$ | $CH_3$ | Cl | H |
| H | $OCH_3$ | $OCH_3$ | $CH_3$ | Br | H |
| H | $OCH_3$ | Cl | $OCH_3$ | H | H |
| H | $OCH_3$ | Br | $OCH_3$ | H | $CH_3$ |
| H | Cl | H | $OCH_3$ | H | $OCH_3$ |
| H | Cl | $CH_3$ | $OCH_3$ | H | Cl |
| H | Cl | $OCH_3$ | $OCH_3$ | H | Br |
| H | Cl | Cl | $OCH_3$ | $CH_3$ | H |
| H | Cl | Br | $OCH_3$ | $OCH_3$ | H |
| H | Br | H | $OCH_3$ | Cl | H |
| H | Br | $CH_3$ | $OCH_3$ | Br | H |

W = W-2

| $R^1$ | $R^2$ | $R^3$ | $R^1$ | $R^2$ | $R^3$ |
|---|---|---|---|---|---|
| H | $CH_3$ | H | $CH_3O$ | H | $CH_3O$ |
| H | $CH_3O$ | H | $CH_3O$ | H | Cl |
| $CH_3$ | H | H | $CH_3O$ | H | Br |
| $CH_3$ | $CH_3$ | H | | | |
| $CH_3$ | $CH_3O$ | H | | | |
| $CH_3O$ | H | H | | | |
| $CH_3O$ | $CH_3$ | H | | | |
| $CH_3O$ | $CH_3$ | H | | | |
| H | $CH_3$ | $CH_3$ | | | |
| H | $CH_3$ | $CH_3O$ | | | |
| H | $CH_3$ | Cl | | | |
| H | $CH_3$ | Br | | | |
| H | $CH_3O$ | $CH_3$ | | | |
| H | $CH_3O$ | $CH_3O$ | | | |
| H | $CH_3O$ | Cl | | | |
| H | $CH_3O$ | Br | | | |
| $CH_3$ | H | $CH_3$ | | | |
| $CH_3$ | H | $CH_3O$ | | | |
| $CH_3$ | H | Cl | | | |
| $CH_3$ | H | Br | | | |
| $CH_3O$ | H | $CH_3$ | | | |

W = W-3

| $R^1$ | $R^2$ | $R^3$ | $R^4$ | $R^1$ | $R^2$ | $R^3$ | $R^4$ |
|---|---|---|---|---|---|---|---|
| $CH_3$ | H | H | H | H | H | Br | H |
| $OCH_3$ | H | H | H | $CH_3$ | H | Br | H |
| H | $CH_3$ | H | H | $OCH_3$ | H | Br | H |
| $CH_3$ | $CH_3$ | H | H | H | H | H | $CH_3$ |
| $OCH_3$ | $CH_3$ | H | H | $CH_3$ | H | H | $CH_3$ |
| H | $OCH_3$ | H | H | $OCH_3$ | H | H | $CH_3$ |
| $CH_3$ | $OCH_3$ | H | H | H | H | H | $OCH_3$ |
| $OCH_3$ | $OCH_3$ | H | H | $CH_3$ | H | H | $OCH_3$ |
| H | Cl | H | H | $OCH_3$ | H | H | $OCH_3$ |
| $CH_3$ | Cl | H | H | H | H | H | Cl |
| $OCH_3$ | Cl | H | H | $CH_3$ | H | H | Cl |
| H | Br | H | H | $OCH_3$ | H | H | Cl |
| $CH_3$ | Br | H | H | H | H | H | Br |
| $OCH_3$ | Br | H | H | $CH_3$ | H | H | Br |
| H | H | $CH_3$ | H | $OCH_3$ | H | H | Br |
| $CH_3$ | H | $CH_3$ | H | H | $CH_3$ | $CH_3$ | H |
| $OCH_3$ | H | $CH_3$ | H | H | $CH_3$ | $OCH_3$ | H |
| H | H | $OCH_3$ | H | H | $CH_3$ | Cl | H |
| $CH_3$ | H | $OCH_3$ | H | H | $CH_3$ | Br | H |
| $OCH_3$ | H | $OCH_3$ | H | H | $OCH_3$ | $CH_3$ | H |
| H | H | Cl | H | H | $OCH_3$ | $OCH_3$ | H |
| $CH_3$ | H | Cl | H | H | $OCH_3$ | Cl | H |
| $OCH_3$ | H | Cl | H | H | $OCH_3$ | Br | H |

| $R^1$ | $R^2$ | $R^3$ | $R^4$ |
|---|---|---|---|
| H | Cl | $CH_3$ | H |
| H | Cl | $OCH_3$ | H |
| H | Br | $CH_3$ | H |
| H | Br | $OCH_3$ | H |
| H | $CH_3$ | H | $CH_3$ |
| H | $CH_3$ | H | $OCH_3$ |
| H | $CH_3$ | H | Cl |
| H | $CH_3$ | H | Br |
| H | $OCH_3$ | H | $CH_3$ |
| H | $OCH_3$ | H | $OCH_3$ |
| H | $OCH_3$ | H | Cl |
| H | $OCH_3$ | H | Br |
| H | Cl | H | $CH_3$ |
| H | Cl | H | $OCH_3$ |
| H | Cl | H | Cl |
| H | Cl | H | Br |
| H | Br | H | $CH_3$ |
| H | Br | H | $OCH_3$ |
| H | Br | H | Cl |
| H | Br | H | Br |
| H | H | $CH_3$ | $CH_3$ |
| H | H | $CH_3$ | $OCH_3$ |
| H | H | $CH_3$ | Cl |
| H | H | $CH_3$ | Br |
| H | H | $OCH_3$ | $CH_3$ |
| H | H | $OCH_3$ | $OCH_3$ |
| H | H | $OCH_3$ | Cl |
| H | H | $OCH_3$ | Br |
| H | H | Cl | $CH_3$ |
| H | H | Cl | $OCH_3$ |
| H | H | Br | Cl |
| H | H | Br | Br |

W = W-4

| $R^1$ | $R^2$ | $R^3$ | $R^4$ |
|---|---|---|---|
| $CH_3$ | H | H | H |
| $OCH_3$ | H | H | H |
| H | $CH_3$ | H | H |
| $CH_3$ | $CH_3$ | H | H |
| $OCH_3$ | $CH_3$ | H | H |
| H | $OCH_3$ | H | H |
| $CH_3$ | $OCH_3$ | H | H |
| $OCH_3$ | $OCH_3$ | H | H |
| H | H | $CH_3$ | H |
| $CH_3$ | H | $CH_3$ | H |
| $OCH_3$ | H | $CH_3$ | H |
| H | H | $OCH_3$ | H |
| $CH_3$ | H | $OCH_3$ | H |
| $OCH_3$ | H | $OCH_3$ | H |
| H | H | Cl | H |
| $CH_3$ | H | Cl | H |
| $OCH_3$ | H | Cl | H |
| H | H | Br | H |
| $CH_3$ | H | Br | H |
| $OCH_3$ | H | Br | H |
| H | H | H | $CH_3$ |
| $CH_3$ | H | H | $CH_3$ |
| $OCH_3$ | H | H | $CH_3$ |
| H | H | H | $OCH_3$ |
| $CH_3$ | H | H | $OCH_3$ |
| $OCH_3$ | H | H | $OCH_3$ |
| H | H | H | Cl |
| $CH_3$ | H | H | Cl |
| $OCH_3$ | H | H | Cl |
| H | H | H | Br |
| $CH_3$ | H | H | Br |
| $OCH_3$ | H | H | Br |
| H | $CH_3$ | $CH_3$ | H |
| H | $CH_3$ | $OCH_3$ | H |
| H | $CH_3$ | Cl | H |
| H | $CH_3$ | Br | H |
| H | $OCH_3$ | $CH_3$ | H |
| H | $OCH_3$ | $OCH_3$ | H |
| H | $OCH_3$ | Cl | H |
| H | $OCH_3$ | Br | H |

W = W-5

| $R^1$ | $R^2$ | $R^3$ | $R^4$ |
|---|---|---|---|
| $CH_3$ | H | H | H |
| $OCH_3$ | H | H | H |
| H | $CH_3$ | H | H |
| $CH_3$ | $CH_3$ | H | H |
| $OCH_3$ | $CH_3$ | H | H |
| H | $OCH_3$ | H | H |
| $CH_3$ | $OCH_3$ | H | H |
| $OCH_3$ | $OCH_3$ | H | H |
| H | H | $CH_3$ | H |
| $CH_3$ | H | $CH_3$ | H |
| $OCH_3$ | H | $CH_3$ | H |
| H | H | $OCH_3$ | H |
| $CH_3$ | H | $OCH_3$ | H |
| $OCH_3$ | H | $OCH_3$ | H |
| H | H | Cl | H |
| $CH_3$ | H | Cl | H |
| $OCH_3$ | H | Cl | H |
| H | H | Br | H |
| $CH_3$ | H | Br | H |
| $OCH_3$ | H | Br | H |
| H | H | H | $CH_3$ |
| $CH_3$ | H | H | $CH_3$ |
| $OCH_3$ | H | H | $CH_3$ |
| H | H | H | $OCH_3$ |
| $CH_3$ | H | H | $OCH_3$ |
| $OCH_3$ | H | H | $OCH_3$ |
| H | H | H | Cl |
| $CH_3$ | H | H | Cl |
| $OCH_3$ | H | H | Cl |
| H | H | H | Br |
| $CH_3$ | H | H | Br |
| $OCH_3$ | H | H | Br |

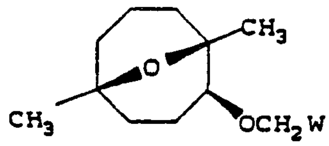

| W = W-6 | | W = W-7 | | W = W-8 | | |
|---|---|---|---|---|---|---|
| $R^1$ | $R^2$ | $R^1$ | $R^2$ | $R^1$ | $R^2$ | $R^3$ |
| H | H | H | H | H | H | H |
| H | $CH_3$ | H | $CH_3$ | H | $CH_3$ | H |
| H | $CH_3O$ | H | $CH_3O$ | H | $CH_3O$ | H |
| $CH_3$ | H | $CH_3$ | H | $CH_3$ | H | H |
| $CH_3$ | $CH_3$ | $CH_3$ | $CH_3$ | $CH_3$ | $CH_3$ | H |
| $CH_3$ | $CH_3O$ | $CH_3$ | $CH_3O$ | $CH_3$ | $CH_3O$ | H |
| $CH_3O$ | H | $CH_3O$ | H | $CH_3O$ | H | H |
| $CH_3O$ | $CH_3$ | $CH_3O$ | $CH_3$ | $CH_3O$ | $CH_3$ | H |
| $CH_3O$ | $CH_3$ | $CH_3O$ | $CH_3O$ | $CH_3O$ | $CH_3$ | H |
| | | | | $CH_3$ | H | $CH_3$ |
| | | | | $CH_3$ | H | $OCH_3$ |
| | | | | $OCH_3$ | H | $CH_3$ |
| | | | | $OCH_3$ | H | $OCH_3$ |
| | | | | H | H | $OCH_3$ |
| | | | | H | $CH_3$ | $CH_3$ |
| | | | | H | $CH_3$ | $CH_3O$ |
| | | | | H | $CH_3O$ | $CH_3$ |
| | | | | H | $CH_3O$ | $CH_3O$ |

| W = W-9 | | | | W = W-10 ($R^3$=H) | | | |
|---|---|---|---|---|---|---|---|
| $R^1$ | $R^2$ | $R^1$ | $R^2$ | $R^1$ | $R^2$ | $R^1$ | $R^2$ |
| H | H | Br | $CH_3O$ | H | H | $CH_3$ | Cl |
| F | H | Br | CN | F | H | $CH_3$ | Br |
| Cl | H | $CH_3$ | F | F | F | $CH_3$ | $CH_3$ |
| Br | H | $CH_3$ | Cl | F | Cl | $CH_3$ | $CH_3O$ |
| $CH_3$ | H | $CH_3$ | Br | F | Br | $CH_3$ | CN |
| $CH_3O$ | H | $CH_3$ | $CH_3$ | F | $CH_3$ | $CH_3O$ | H |
| CN | H | $CH_3$ | $CH_3O$ | F | $CH_3O$ | $CH_3O$ | F |
| F | F | $CH_3$ | CN | F | CN | $CH_3O$ | Cl |
| F | Cl | $CH_3O$ | F | Cl | H | $CH_3O$ | Br |
| F | Br | $CH_3O$ | Cl | Cl | F | $CH_3O$ | $CH_3$ |
| F | $CH_3$ | $CH_3O$ | Br | Cl | Cl | $CH_3O$ | $CH_3O$ |
| F | $CH_3O$ | $CH_3O$ | $CH_3$ | Cl | Br | $CH_3O$ | CN |
| F | CN | $CH_3O$ | $CH_3O$ | Cl | $CH_3$ | CN | H |
| Cl | F | $CH_3O$ | CN | Cl | $CH_3O$ | CN | F |
| Cl | Cl | CN | F | Cl | CN | CN | Cl |
| Cl | Br | CN | Cl | Br | H | CN | Br |
| Cl | $CH_3$ | CN | Br | Br | F | CN | $CH_3$ |
| Cl | $CH_3O$ | CN | $CH_3$ | Br | Cl | CN | $CH_3O$ |
| Cl | CN | CN | $CH_3O$ | Br | Br | CN | CN |
| Br | F | CN | CN | Br | $CH_3$ | H | F |
| Br | Cl | H | F | Br | $CH_3O$ | H | Cl |
| Br | Br | H | Cl | Br | CN | H | Br |
| Br | $CH_3$ | H | Br | $CH_3$ | H | H | $CH_3$ |
| | | H | $CH_3$ | $CH_3$ | F | H | $CH_3O$ |
| | | H | $CH_3O$ | | | H | CN |
| | | H | CN | | | | |

W = W-11 ($R^3$=H)

| $R^1$ | $R^2$ | $R^1$ | $R^2$ |
|---|---|---|---|
| F | H | Br | CN |
| F | F | $CH_3$ | H |
| F | Cl | $CH_3$ | F |
| F | Br | $CH_3$ | Cl |
| F | $CH_3$ | $CH_3$ | Br |
| F | $CH_3O$ | $CH_3$ | $CH_3$ |
| F | CN | $CH_3$ | $CH_3O$ |
| Cl | H | $CH_3$ | CN |
| Cl | F | $CH_3O$ | H |
| Cl | Cl | $CH_3O$ | F |
| Cl | Br | $CH_3O$ | Cl |
| Cl | $CH_3$ | $CH_3O$ | Br |
| Cl | $CH_3O$ | $CH_3O$ | $CH_3$ |
| Cl | CN | $CH_3O$ | $CH_3O$ |
| Br | H | $CH_3O$ | CN |
| Br | F | CN | H |
| Br | Cl | CN | F |
| Br | Br | CN | Cl |
| Br | $CH_3$ | CN | Br |
| Br | $CH_3O$ | CN | $CH_3$ |
| | | CN | $CH_3O$ |
| | | CN | CN |

W = W-12

| $R^1$ | $R^2$ | $R^1$ | $R^2$ |
|---|---|---|---|
| H | H | Br | F |
| F | H | Br | Cl |
| Cl | H | Br | Br |
| Br | H | Br | $CH_3$ |
| $CH_3$ | H | Br | $CH_3O$ |
| $CH_3O$ | H | Br | CN |
| CN | H | $CH_3$ | F |
| F | F | $CH_3$ | Cl |
| F | Cl | $CH_3$ | Br |
| F | Br | $CH_3$ | $CH_3$ |
| F | $CH_3$ | $CH_3$ | $CH_3O$ |
| F | $CH_3O$ | $CH_3$ | CN |
| F | CN | $CH_3$ | F |
| F | $CH_3O$ | Cl | |
| Cl | Cl | $CH_3O$ | Br |
| Cl | Br | $CH_3O$ | $CH_3$ |
| Cl | $CH_3$ | $CH_3O$ | $CH_3O$ |
| Cl | $CH_3O$ | $CH_3O$ | CN |
| Cl | CN | CN | F |
| | | CN | Cl |
| | | CN | Br |
| | | CN | $CH_3$ |
| | | CN | $CH_3O$ |
| | | CN | CN |
| | | H | F |
| | | H | Cl |
| | | H | Br |
| | | H | $CH_3$ |
| | | H | $CH_3O$ |
| | | H | CN |

W = W-13

| $R^1$ | $R^2$ | $R^1$ | $R^2$ |
|---|---|---|---|
| F | F | CN | F |
| F | Cl | CN | Cl |
| F | Br | CN | Br |
| F | $CH_3$ | CN | $CH_3$ |
| F | $CH_3O$ | CN | $CH_3O$ |
| F | CN | CN | CN |
| Cl | F | H | F |
| Cl | Cl | H | Cl |
| Cl | Br | H | Br |
| Cl | $CH_3$ | H | $CH_3$ |
| Cl | $CH_3O$ | H | $CH_3O$ |
| Cl | CN | H | CN |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |
| $CH_3O$ | F | | |
| $CH_3O$ | Cl | | |
| $CH_3O$ | Br | | |
| $CH_3O$ | $CH_3$ | | |
| $CH_3O$ | $CH_3O$ | | |
| $CH_3O$ | CN | | |

W = W-14

| $R^1$ | $R^2$ | $R^1$ | $R^2$ |
|---|---|---|---|
| F | F | CN | F |
| F | Cl | CN | Cl |
| F | Br | CN | Br |
| F | $CH_3$ | CN | $CH_3$ |
| F | $CH_3O$ | CN | $CH_3O$ |
| F | CN | CN | CN |
| Cl | F | H | F |
| Cl | Cl | H | Cl |
| Cl | Br | H | Br |
| Cl | $CH_3$ | H | $CH_3$ |
| Cl | $CH_3O$ | H | $CH_3O$ |
| Cl | CN | H | CN |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | CH | | |
| $CH_3$ | $CH_3O$ | | |
| $CH_3$ | CN | | |
| $CH_3O$ | F | | |
| $CH_3O$ | Cl | | |
| $CH_3O$ | Br | | |
| $CH_3O$ | $CH_3$ | | |
| $CH_3O$ | $CH_3O$ | | |
| $CH_3O$ | CN | | |

W = W-15

| $R^1$ | $R^2$ | $R^1$ | $R^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

W = W-16

| $R^1$ | $R^2$ | $R^1$ | $R^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

W = W-17

| $R^1$ | $R^2$ | $R^1$ | $R^2$ |
|---|---|---|---|
| H | H | $CH_3$ | CN |
| F | H | $CH_3O$ | F |
| Cl | H | $CH_3O$ | Cl |
| Br | H | $CH_3O$ | Br |
| $CH_3$ | H | $CH_3O$ | $CH_3$ |
| $CH_3O$ | H | $CH_3O$ | $CH_3O$ |
| CN | H | $CH_3O$ | CN |
| F | F | CN | F |
| F | Cl | CN | Cl |
| F | Br | CN | Br |
| F | $CH_3$ | CN | $CH_3$ |
| F | $CH_3O$ | CN | $CH_3O$ |
| F | CN | CN | CN |
| Cl | F | H | F |
| Cl | Cl | H | Cl |
| Cl | Br | H | Br |
| Cl | $CH_3$ | H | $CH_3$ |
| $Cl_3$ | $CH_3O$ | H | $CH_3O$ |
| Cl | CN | H | CN |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |

W = W-18

| $R^1$ | $R^2$ | $R^1$ | $R^2$ |
|---|---|---|---|
| H | H | $CH_3$ | CN |
| F | H | $CH_3O$ | F |
| Cl | H | $CH_3O$ | Cl |
| Br | H | $CH_3O$ | Br |
| $CH_3$ | H | $CH_3O$ | $CH_3$ |
| $CH_3O$ | H | $CH_3O$ | $CH_3O$ |
| CN | H | $CH_3O$ | CN |
| F | F | CN | F |
| F | Cl | CN | Cl |
| F | Br | CN | Br |
| F | $CH_3$ | CN | $CH_3$ |
| F | $CH_3O$ | CN | $CH_3O$ |
| F | CN | CN | CN |
| Cl | F | H | F |
| Cl | Cl | H | Cl |
| Cl | Br | H | Br |
| Cl | $CH_3$ | H | $CH_3$ |
| Cl | $CH_3O$ | H | $CH_3O$ |
| Cl | CN | H | CN |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |

| W = W-19 | | W = W-20 | | W = W-21 | |
|---|---|---|---|---|---|
| $R^1$ | $R^2$ | $R^1$ | $R^2$ | $R^1$ | $R^2$ |
| H | H | H | H | H | H |
| H | F | H | F | H | F |
| H | Cl | H | Cl | H | Cl |
| H | Br | H | Br | H | Br |
| H | $CH_3$ | H | $CH_3$ | H | $CH_3$ |
| H | $CH_3O$ | H | $CH_3O$ | H | $CH_3O$ |
| H | CN | H | CN | H | CN |
| F | H | F | H | F | H |
| Cl | H | Cl | H | Cl | H |
| Br | H | Br | H | Br | H |
| $CH_3$ | H | $CH_3$ | H | $CH_3$ | H |
| $CH_3O$ | H | $CH_3O$ | H | $CH_3O$ | H |
| CN | H | CN | H | CN | H |

| W = W-22 | | | | W = W-23 | | | |
|---|---|---|---|---|---|---|---|
| $R^1$ | $R^2$ | $R^1$ | $R^2$ | $R^1$ | $R^2$ | $R^1$ | $R^2$ |
| H | H | Br | $CH_3$ | H | H | $CH_3$ | Br |
| F | H | Br | $CH_3O$ | F | H | $CH_3$ | $CH_3$ |
| Cl | H | Br | CN | Cl | H | $CH_3$ | $CH_3O$ |
| Br | H | $CH_3$ | F | Br | H | $CH_3$ | CN |
| $CH_3$ | H | $CH_3$ | Cl | $CH_3$ | H | $CH_3O$ | F |
| $CH_3O$ | H | $CH_3$ | Br | $CH_3O$ | H | $CH_3O$ | Cl |
| CN | H | $CH_3$ | $CH_3$ | CN | H | $CH_3O$ | Br |
| F | F | $CH_3$ | $CH_3O$ | F | F | $CH_3O$ | $CH_3$ |
| F | Cl | $CH_3$ | CN | F | Cl | $CH_3O$ | $CH_3O$ |
| F | Br | $CH_3O$ | F | F | Br | $CH_3O$ | CN |
| F | $CH_3$ | $CH_3O$ | Cl | F | $CH_3$ | CN | F |
| F | $CH_3O$ | $CH_3O$ | Br | F | $CH_3O$ | CN | Cl |
| F | CN | $CH_3O$ | $CH_3$ | F | CN | CN | Br |
| Cl | F | $CH_3O$ | $CH_3O$ | Cl | F | CN | $CH_3$ |
| Cl | Cl | $CH_3O$ | CN | Cl | Cl | CN | $CH_3O$ |
| Cl | Br | CN | F | Cl | Br | CN | CN |
| Cl | $CH_3$ | CN | Cl | Cl | $CH_3$ | H | F |
| Cl | $CH_3O$ | CN | Br | Cl | $CH_3O$ | H | Cl |
| Cl | CN | CN | $CH_3$ | Cl | CN | H | Br |
| Br | F | CN | $CH_3O$ | Br | F | H | $CH_3$ |
| Br | Cl | CN | CN | Br | Cl | H | $CH_3O$ |
| Br | Br | H | F | Br | Br | H | CN |
| | | H | Cl | Br | $CH_3$ | | |
| | | H | Br | Br | $CH_3O$ | | |
| | | H | $CH_3$ | Br | CN | | |
| | | H | $CH_3O$ | $CH_3$ | F | | |
| | | H | CN | $CH_3$ | Cl | | |

| W = W-24 | | | | W = W-25 | | | |
|---|---|---|---|---|---|---|---|
| $R^1$ | $R^2$ | $R^1$ | $R^2$ | $R^1$ | $R^2$ | $R^1$ | $R^2$ |
| H | H | $CH_3O$ | F | H | H | $CH_3O$ | F |
| F | H | $CH_3O$ | Cl | F | H | $CH_3O$ | Cl |
| Cl | H | $CH_3O$ | Br | Cl | H | $CH_3O$ | Br |
| Br | H | $CH_3O$ | $CH_3$ | Br | H | $CH_3O$ | $CH_3$ |
| $CH_3$ | H | $CH_3O$ | $CH_3O$ | $CH_3$ | H | $CH_3O$ | $CH_3O$ |
| $CH_3O$ | H | $CH_3O$ | CN | $CH_3O$ | H | $CH_3O$ | CN |
| CN | H | CN | F | CN | H | CN | F |
| F | F | CN | Cl | F | F | CN | Cl |
| F | Cl | CN | Br | F | Cl | CN | Br |
| F | Br | CN | $CH_3$ | F | Br | CN | $CH_3$ |
| F | $CH_3$ | CN | $CH_3O$ | F | $CH_3$ | CN | $CH_3O$ |
| F | $CH_3O$ | CN | CN | F | $CH_3O$ | CN | CN |
| F | CN | H | F | F | CN | H | F |
| Cl | F | H | Cl | Cl | F | H | Cl |
| Cl | Cl | H | Br | Cl | Cl | H | Br |
| Cl | Br | H | $CH_3$ | Cl | Br | H | $CH_3$ |
| Cl | $CH_3$ | H | $CH_3O$ | Cl | CH | H | $CH_3O$ |
| $Cl_3$ | $CH_3O$ | H | CN | Cl | $CH_3O$ | H | CN |
| Cl | CN | | | Cl | CN | | |
| Br | F | | | Br | F | | |
| Br | Cl | | | Br | Cl | | |
| Br | Br | | | Br | Br | | |
| Br | $CH_3$ | | | Br | $CH_3$ | | |
| Br | $CH_3O$ | | | Br | $CH_3O$ | | |
| Br | CN | | | Br | CN | | |
| $CH_3$ | F | | | $CH_3$ | F | | |
| $CH_3$ | Cl | | | $CH_3$ | Cl | | |
| $CH_3$ | Br | | | $CH_3$ | Br | | |
| $CH_3$ | $CH_3$ | | | $CH_3$ | $CH_3$ | | |
| $CH_3$ | $CH_3O$ | | | $CH_3$ | $CH_3O$ | | |
| $CH_3$ | CN | | | $CH_3$ | CN | | |

W = W-26

| $R^1$ | $R^2$ | $R^1$ | $R^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

W = W-27

| $R^1$ | $R^2$ | $R^1$ | $R^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

W = W-28

| $\underline{R}^1$ | $\underline{R}^2$ | $\underline{R}^1$ | $\underline{R}^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

W = W-29

| $\underline{R}^1$ | $\underline{R}^2$ | $\underline{R}^1$ | $\underline{R}^2$ |
|---|---|---|---|
| H | H | $CH_3O$ | F |
| F | H | $CH_3O$ | Cl |
| Cl | H | $CH_3O$ | Br |
| Br | H | $CH_3O$ | $CH_3$ |
| $CH_3$ | H | $CH_3O$ | $CH_3O$ |
| $CH_3O$ | H | $CH_3O$ | CN |
| CN | H | CN | F |
| F | F | CN | Cl |
| F | Cl | CN | Br |
| F | Br | CN | $CH_3$ |
| F | $CH_3$ | CN | $CH_3O$ |
| F | $CH_3O$ | CN | CN |
| F | CN | H | F |
| Cl | F | H | Cl |
| Cl | Cl | H | Br |
| Cl | Br | H | $CH_3$ |
| Cl | $CH_3$ | H | $CH_3O$ |
| Cl | $CH_3O$ | H | CN |
| Cl | CN | | |
| Br | F | | |
| Br | Cl | | |
| Br | Br | | |
| Br | $CH_3$ | | |
| Br | $CH_3O$ | | |
| Br | CN | | |
| $CH_3$ | F | | |
| $CH_3$ | Cl | | |
| $CH_3$ | Br | | |
| $CH_3$ | $CH_3$ | | |
| $CH_3$ | $CH_3O$ | | |
| $CH_3$ | CN | | |

| W = W-30 | | | | W = 31 | | | |
|---|---|---|---|---|---|---|---|
| X | R | X | R | X | R | X | R |
| NH | H | S | Br | NH | H | S | Cl |
| NH | F | S | $CH_3$ | NH | F | S | Br |
| NH | Cl | S | $CH_3O$ | NH | Cl | S | $CH_3$ |
| NH | Br | S | CN | NH | Br | S | $CH_3O$ |
| NH | $CH_3$ | | | NH | $CH_3$ | S | CN |
| NH | $CH_3O$ | | | NH | $CH_3O$ | | |
| NH | CN | | | NH | CN | | |
| $NCH_3$ | H | | | $NCH_3$ | H | | |
| $NCH_3$ | F | | | $NCH_3$ | F | | |
| $NCH_3$ | Cl | | | $NCH_3$ | Cl | | |
| $NCH_3$ | Br | | | $NCH_3$ | Br | | |
| $NCH_3$ | $CH_3$ | | | $NCH_3$ | $CH_3$ | | |
| $NCH_3$ | $CH_3O$ | | | $NCH_3$ | $CH_3O$ | | |
| $NCH_3$ | CN | | | $NCH_3$ | CN | | |
| NCN | H | | | NCN | H | | |
| NCN | F | | | NCN | F | | |
| NCN | Cl | | | NCN | Cl | | |
| NCN | Br | | | NCN | Br | | |
| NCN | $CH_3$ | | | NCN | $CH_3$ | | |
| NCN | $CH_3O$ | | | NCN | $CH_3O$ | | |
| O | H | | | O | H | | |
| O | F | | | O | F | | |
| O | Cl | | | O | Cl | | |
| O | Br | | | O | Br | | |
| O | $CH_3$ | | | O | $CH_3$ | | |
| O | $CH_3O$ | | | O | $CH_3O$ | | |
| O | CN | | | O | CN | | |
| S | H | | | S | H | | |
| S | F | | | S | F | | |
| S | Cl | | | | | | |

W = W-32

| X | R | X | R |
|---|---|---|---|
| NH | H | S | Br |
| NH | F | S | $CH_3$ |
| NH | Cl | S | $CH_3O$ |
| NH | Br | S | CN |
| NH | $CH_3$ | | |
| NH | $CH_3O$ | | |
| NH | CN | | |
| $NCH_3$ | H | | |
| $NCH_3$ | F | | |
| $NCH_3$ | Cl | | |
| $NCH_3$ | Br | | |
| $NCH_3$ | $CH_3$ | | |
| $NCH_3$ | $CH_3O$ | | |
| $NCH_3$ | CN | | |
| NCN | H | | |
| NCN | F | | |
| NCN | Cl | | |
| NCN | Br | | |
| NCN | $CH_3$ | | |
| NCN | $CH_3O$ | | |
| O | H | | |
| O | F | | |
| O | Cl | | |
| O | Br | | |
| O | $CH_3$ | | |
| O | $CH_3O$ | | |
| O | CN | | |
| S | H | | |
| S | F | | |
| S | Cl | | |

TABLE 4

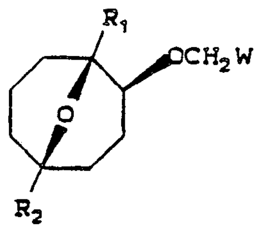

where W =

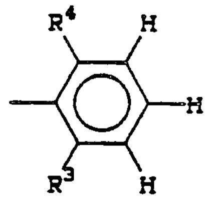

| $R_1$ | $R_2$ | $R^3$ | $R^4$ | $R_1$ | $R_2$ | $R^3$ | $R^4$ |
|---|---|---|---|---|---|---|---|
| H | H | H | H | H | $CH_3$ | F | Cl |
| H | H | F | H | H | $CH_3$ | F | F |
| H | H | Me | H | H | $CH_3$ | Cl | Cl |
| H | H | F | F | H | $CH_3$ | Cl | H |
| H | H | Cl | H | H | $CH_3$ | Me | H |
| H | H | F | Cl | H | CH3 | Br | H |
| H | H | Cl | Cl | $C_2H_5$ | $C_2H_5$ | H | H |
| H | H | Br | H | $C_2H_5$ | $C_2H_5$ | F | H |
| H | H | $CH_3O$ | H | $C_2H_5$ | $C_2H_5$ | F | Cl |
| H | H | $CH_3S$ | H | $C_2H_5$ | $C_2H_5$ | F | F |
| H | H | CN | H | $C_2H_5$ | $C_2H_5$ | Cl | Cl |
| $CH_3$ | H | H | H | $C_2H_5$ | $C_2H_5$ | Cl | H |
| $CH_3$ | H | F | H | $C_2H_5$ | $C_2H_5$ | Me | H |
| $CH_3$ | H | F | Cl | $C_2H_5$ | $C_2H_5$ | Br | H |
| $CH_3$ | H | F | F | $C_2H_5$ | H | H | H |
| $CH_3$ | H | Cl | Cl | $C_2H_5$ | H | F | H |
| $CH_3$ | H | Cl | H | $C_2H_5$ | H | F | Cl |
| $CH_3$ | H | Me | H | $C_2H_5$ | H | F | F |
| $CH_3$ | H | Br | H | $C_2H_5$ | H | Cl | Cl |
| H | $CH_3$ | H | H | $C_2H_5$ | H | Cl | H |
| H | $CH_3$ | F | H | $C_2H_5$ | H | Me | H |

| $R_1$ | $R_2$ | $R^3$ | $R^4$ | $R_1$ | $R_2$ | $R^3$ | $R^4$ |
|---|---|---|---|---|---|---|---|
| $C_2H_5$ | H | Br | H | $CH_3$ | $i-C_3H_7$ | H | H |
| H | $CH_3CH_2$ | H | H | $CH_3$ | $i-C_3H_7$ | H | H |
| H | $CH_3CH_2$ | F | H | $CH_3$ | $i-C_3H_7$ | F | H |
| H | $CH_3CH_2$ | F | Cl | $CH_3$ | $i-C_3H_7$ | F | H |
| H | $CH_3CH_2$ | F | F | $CH_3$ | $i-C_3H_7$ | Br | H |
| H | $CH_3CH_2$ | Cl | Cl | $CH_3$ | $i-C_3H_7$ | $CH_3O$ | H |
| H | $CH_3CH_2$ | Cl | H | $CH_3$ | $i-C_3H_7$ | $CH_3S$ | H |
| H | $CH_3CH_2$ | Me | H | $CH_3$ | $i-C_3H_7$ | CN | H |
| H | $CH_3CH_2$ | Br | H | $n-C_4H_9$ | $CH_3CH_2$ | H | H |
| $C_2H_5$ | $CH_3$ | H | H | $n-C_4H_9$ | $CH_3CH_2$ | F | H |
| $C_2H_5$ | $CH_3$ | F | H | $n-C_4H_9$ | $CH_3CH_2$ | F | Cl |
| $C_2H_5$ | $CH_3$ | F | Cl | $n-C_4H_9$ | $CH_3CH_2$ | F | F |
| $C_2H_5$ | $CH_3$ | F | F | $n-C_4H_9$ | $CH_3CH_2$ | Cl | Cl |
| $C_2H_5$ | $CH_3$ | Cl | Cl | $n-C_4H_9$ | $CH_3CH_2$ | Cl | H |
| $C_2H_5$ | $CH_3$ | Cl | H | $n-C_4H_9$ | $CH_3CH_2$ | Me | H |
| $C_2H_5$ | $CH_3$ | Me | H | $n-C_4H_9$ | $CH_3CH_2$ | Br | H |
| $C_2H_5$ | $CH_3$ | Br | H | H | $n-C_3H_7$ | H | H |
| $CH_3$ | $CH_3CH_2$ | H | H | H | $i-C_3H_7$ | F | H |
| $CH_3$ | $CH_3CH_2$ | F | H | H | $n-C_4H_9$ | Me | H |
| $CH_3$ | $CH_3CH_2$ | F | Cl | H | $(CH_3)_2CHCH_2$ | F | F |
| $CH_3$ | $CH_3CH_2$ | F | F | H | CN | H | H |
| $CH_3$ | $CH_3CH_2$ | Cl | Cl | H | $CH=CH_2$ | F | H |
| $CH_3$ | $CH_3CH_2$ | Cl | H | $CH_3$ | $CH_2CO_2CH_3$ | F | F |
| $CH_3$ | $CH_3CH_2$ | Me | H | $CH_2CH_3$ | $CH_3SO_2CH_2$ | H | H |
| $CH_3$ | $CH_3CH_2$ | Br | H | $CH_3$ | Ph | F | H |
| $CH_3$ | $i-C_3H_7$ | H | H | H | $CH_2Ph$ | F | F |
| $CH_3$ | $i-C_3H_7$ | F | H | | | | |
| $CH_3$ | $i-C_3H_7$ | Me | H | | | | |
| $CH_3$ | $i-C_3H_7$ | F | F | | | | |
| $CH_3$ | $i-C_3H_7$ | Cl | H | | | | |
| $CH_3$ | $i-C_3H_7$ | F | Cl | | | | |
| $CH_3$ | $i-C_3H_7$ | Cl | Cl | | | | |

TABLE 5

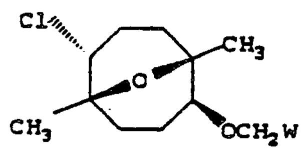

where W =

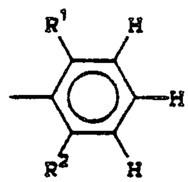

| $R^1$ | $R^2$ | $R^1$ | $R^2$ | $R^1$ | $R^2$ |
|---|---|---|---|---|---|
| H | H | Br | $CH_3$ | H | Cl |
| F | H | Br | $CH_3O$ | H | Br |
| Cl | H | Br | CN | H | $CH_3$ |
| Br | H | $CH_3$ | F | H | $CH_3O$ |
| $CH_3$ | H | $CH_3$ | Cl | H | CN |
| $CH_3O$ | H | $CH_3$ | Br | | |
| CN | H | $CH_3$ | $CH_3$ | | |
| F | F | $CH_3$ | $CH_3O$ | | |
| F | Cl | $CH_3$ | CN | | |
| F | Br | $CH_3O$ | F | | |
| F | $CH_3$ | $CH_3O$ | Cl | | |
| F | $CH_3O$ | $CH_3O$ | Br | | |
| F | CN | $CH_3O$ | $CH_3$ | | |
| Cl | F | $CH_3O$ | $CH_3O$ | | |
| Cl | Cl | $CH_3O$ | CN | | |
| Cl | Br | CN | F | | |
| Cl | $CH_3$ | CN | Cl | | |
| Cl | $CH_3O$ | CN | Br | | |
| Cl | CN | CN | $CH_3$ | | |
| Br | F | CN | $CH_3O$ | | |
| Br | Cl | CN | CN | | |
| Br | Br | H | F | | |

W = W-10

| $R^1$ | $R^2$ | $R^3$ |
|---|---|---|
| H | H | H |
| F | H | H |
| Cl | H | H |
| Br | H | H |
| $CH_3$ | H | H |
| $CH_3O$ | H | H |
| CN | H | H |
| H | F | H |
| H | Cl | H |
| H | Br | H |
| H | $CH_3$ | H |
| H | $CH_3O$ | H |
| H | CN | H |
| H | H | F |
| H | H | Cl |
| H | H | Br |
| H | H | $CH_3$ |
| H | H | $CH_3O$ |
| H | H | CN |

W = W-11

| $R^1$ | $R^2$ | $R^3$ |
|---|---|---|
| H | H | H |
| F | H | H |
| Cl | H | H |
| Br | H | H |
| $CH_3$ | H | H |
| $CH_3O$ | H | H |
| CN | H | H |
| H | F | H |
| H | Cl | H |
| H | Br | H |
| H | $CH_3$ | H |
| H | $CH_3O$ | H |
| H | CN | H |
| H | H | F |
| H | H | Cl |
| H | H | Br |
| H | H | $CH_3$ |
| H | H | $CH_3O$ |
| H | H | CN |

| W = W-14 | | W = W-28 | | W = W-27 | | W = W-26 | |
|---|---|---|---|---|---|---|---|
| $R^1$ | $R^2$ | $R^1$ | $R^2$ | $R^1$ | $R^2$ | $R^1$ | $R^2$ |
| H | H | H | H | H | H | H | H |
| F | H | H | F | H | F | H | F |
| Cl | H | H | Cl | H | Cl | H | Cl |
| Br | H | H | Br | H | Br | H | Br |
| $CH_3$ | H | H | $CH_3$ | H | $CH_3$ | H | $CH_3$ |
| $CH_3O$ | H | H | $CH_3O$ | H | $CH_3O$ | H | $CH_3O$ |
| CN | H | H | $CH_3O$ | H | $CH_3O$ | H | $CH_3O$ |
| H | F | H | CN | H | CN | H | CN |
| H | Cl | F | H | F | H | F | H |
| H | Br | Cl | H | Cl | H | Cl | H |
| H | $CH_3$ | Br | H | Br | H | Br | H |
| H | $CH_3O$ | $CH_3$ | H | $CH_3$ | H | $CH_3$ | H |
| H | CN | $CH_3O$ | H | $CH_3O$ | H | $CH_3O$ | H |
| F | F | CN | H | CN | H | CN | H |
| F | $CH_3$ | | | | | | |
| $CH_3$ | F | | | | | | |

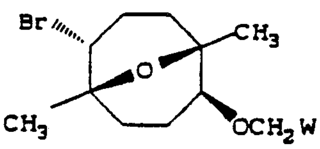 where W = 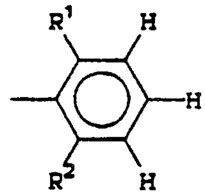

| $R^1$ | $R^2$ | $R^1$ | $R^2$ | $R^1$ | $R^2$ |
|---|---|---|---|---|---|
| H | H | Br | $CH_3O$ | H | $CH_3$ |
| F | H | Br | CN | H | $CH_3O$ |
| Cl | H | $CH_3$ | F | H | CN |
| Br | H | $CH_3$ | Cl | | |
| $CH_3$ | H | $CH_3$ | Br | | |
| $CH_3O$ | H | $CH_3$ | $CH_3$ | | |
| CN | H | $CH_3$ | $CH_3O$ | | |
| F | F | $CH_3$ | CN | | |
| F | Cl | $CH_3O$ | F | | |
| F | Br | $CH_3O$ | Cl | | |
| F | $CH_3$ | $CH_3O$ | Br | | |
| F | $CH_3O$ | $CH_3O$ | $CH_3$ | | |
| F | CN | $CH_3O$ | $CH_3O$ | | |
| Cl | F | $CH_3O$ | CN | | |
| Cl | Cl | CN | F | | |
| Cl | Br | CN | Cl | | |
| Cl | $CH_3$ | CN | Br | | |
| Cl | $CH_3O$ | CN | $CH_3$ | | |
| Cl | CN | CN | $CH_3O$ | | |
| Br | F | CN | CN | | |
| Br | Cl | H | F | | |
| Br | Br | H | Cl | | |
| Br | $CH_3$ | H | Br | | |

| W = W-10 | | | W = W-12 | | |
|---|---|---|---|---|---|
| $\underline{R}^1$ | $\underline{R}^2$ | $\underline{R}^3$ | $\underline{R}^1$ | $\underline{R}^2$ | $\underline{R}^3$ |
| H | H | H | H | H | H |
| F | H | H | F | H | H |
| Cl | H | H | Cl | H | H |
| Br | H | H | Br | H | H |
| $CH_3$ | H | H | $CH_3$ | H | H |
| $CH_3O$ | H | H | $CH_3O$ | H | H |
| CN | H | H | CN | H | H |
| H | F | H | H | F | H |
| H | Cl | H | H | Cl | H |
| H | Br | H | H | Br | H |
| H | $CH_3$ | H | H | $CH_3$ | H |
| H | $CH_3O$ | H | H | $CH_3O$ | H |
| H | CN | H | H | CN | H |
| H | H | F | H | H | F |
| H | H | Cl | H | H | Cl |
| H | H | Br | H | H | Br |
| H | H | $CH_3$ | H | H | $CH_3$ |
| H | H | $CH_3O$ | H | H | $CH_3O$ |
| H | H | CN | H | H | CN |

| W = W-14 | | W = W-28 | | W = W-27 | | W = W-26 | |
|---|---|---|---|---|---|---|---|
| $R^1$ | $R^2$ | $R^1$ | $R^2$ | $R^1$ | $R^2$ | $R^1$ | $R^2$ |
| H | H | H | H | H | H | H | H |
| F | H | H | F | H | F | H | F |
| Cl | H | H | Cl | H | Cl | H | Cl |
| Br | H | H | Br | H | Br | H | Br |
| $CH_3$ | H | H | $CH_3$ | H | $CH_3$ | H | $CH_3$ |
| $CH_3O$ | H | H | $CH_3O$ | H | $CH_3O$ | H | $CH_3O$ |
| CN | H | H | $CH_3O$ | H | $CH_3O$ | H | $CH_3O$ |
| H | F | H | CN | H | CN | H | CN |
| H | Cl | F | H | F | H | F | H |
| H | Br | Cl | H | Cl | H | Cl | H |
| H | $CH_3$ | Br | H | Br | H | Br | H |
| H | $CH_3O$ | $CH_3$ | H | $CH_3$ | H | $CH_3$ | H |
| H | CN | $CH_3O$ | H | $CH_3O$ | H | $CH_3O$ | H |
| F | F | CN | H | CN | H | CN | H |
| F | $CH_3$ | | | | | | |
| $CH_3$ | F | | | | | | |

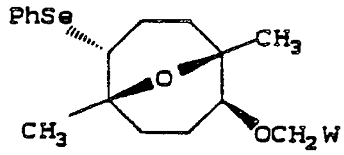 where W = 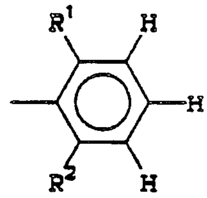

| $R^1$ | $R^2$ | $R^1$ | $R^2$ | $R^1$ | $R^2$ |
|---|---|---|---|---|---|
| H | H | Br | $CH_3O$ | H | $CH_3$ |
| F | H | Br | CN | H | $CH_3O$ |
| Cl | H | $CH_3$ | F | H | CN |
| Br | H | $CH_3$ | Cl | | |
| $CH_3$ | H | $CH_3$ | Br | | |
| $CH_3O$ | H | $CH_3$ | $CH_3$ | | |
| CN | H | $CH_3$ | $CH_3O$ | | |
| F | F | $CH_3$ | CN | | |
| F | Cl | $CH_3O$ | F | | |
| F | Br | $CH_3O$ | Cl | | |
| F | $CH_3$ | $CH_3O$ | Br | | |
| F | $CH_3O$ | $CH_3O$ | $CH_3$ | | |
| F | CN | $CH_3O$ | $CH_3O$ | | |
| Cl | F | $CH_3O$ | CN | | |
| Cl | Cl | CN | F | | |
| Cl | Br | CN | Cl | | |
| Cl | $CH_3$ | CN | Br | | |
| Cl | $CH_3O$ | CN | $CH_3$ | | |
| Cl | CN | CN | $CH_3O$ | | |
| Br | F | CN | CN | | |
| Br | Cl | H | F | | |
| Br | Br | H | Cl | | |
| Br | $CH_3$ | H | Br | | |

TABLE 6

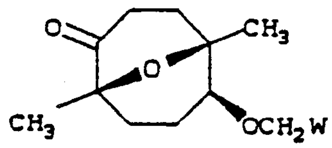

where W =

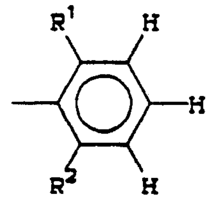

| $R^1$ | $R^2$ | $R^1$ | $R^2$ | $R^1$ | $R^2$ |
|---|---|---|---|---|---|
| H | H | Cl | $CH_3O$ | $CH_3O$ | $CH_3$ |
| F | H | Cl | CN | $CH_3O$ | $CH_3O$ |
| Cl | H | Br | F | $CH_3O$ | CN |
| Br | H | Br | Cl | CN | F |
| $CH_3$ | H | Br | Br | CN | Cl |
| $CH_3O$ | H | Br | $CH_3$ | CN | Br |
| CN | H | Br | $CH_3O$ | CN | $CH_3$ |
| F | F | Br | CN | CN | $CH_3O$ |
| F | Cl | $CH_3$ | F | CN | CN |
| F | Br | $CH_3$ | Cl | H | F |
| F | $CH_3$ | $CH_3$ | Br | H | Cl |
| F | $CH_3O$ | $CH_3$ | $CH_3$ | H | Br |
| F | CN | $CH_3$ | $CH_3O$ | H | $CH_3$ |
| Cl | F | $CH_3$ | CN | H | $CH_3O$ |
| Cl | Cl | $CH_3O$ | F | H | CN |
| Cl | Br | $CH_3O$ | Cl | | |
| Cl | $CH_3$ | $CH_3O$ | Br | | |

| W = W-10 | | | W = W-12 | | |
|---|---|---|---|---|---|
| $R^1$ | $R^2$ | $R^3$ | $R^1$ | $R^2$ | $R^3$ |
| H | H | H | H | H | H |
| F | H | H | F | H | H |
| Cl | H | H | Cl | H | H |
| Br | H | H | Br | H | H |
| $CH_3$ | H | H | $CH_3$ | H | H |
| $CH_3O$ | H | H | $CH_3O$ | H | H |
| CN | H | H | CN | H | H |
| H | F | H | H | F | H |
| H | Cl | H | H | Cl | H |
| H | Br | H | H | Br | H |
| H | $CH_3$ | H | H | $CH_3$ | H |
| H | $CH_3O$ | H | H | $CH_3O$ | H |
| H | CN | H | H | CN | H |
| H | H | F | H | H | F |
| H | H | Cl | H | H | Cl |
| H | H | Br | H | H | Br |
| H | H | $CH_3$ | H | H | $CH_3$ |
| H | H | $CH_3O$ | H | H | $CH_3O$ |
| H | H | CN | H | H | CN |

| W = W-14 | | W = W-28 | | W = W-27 | | W = W-26 | |
|---|---|---|---|---|---|---|---|
| $R^1$ | $R^2$ | $R^1$ | $R^2$ | $R^1$ | $R^2$ | $R^2$ | $R^1$ |
| H | H | H | H | H | H | H | H |
| F | H | H | F | H | F | H | F |
| Cl | H | H | Cl | H | Cl | H | Cl |
| Br | H | H | Br | H | Br | H | Br |
| $CH_3$ | H | H | $CH_3$ | H | $CH_3$ | H | $CH_3$ |
| $CH_3O$ | H | H | $CH_3O$ | H | $CH_3O$ | H | $CH_3O$ |
| CN | H | H | CN | H | CN | H | CN |
| H | F | F | H | F | H | F | H |
| H | Cl | Cl | H | Cl | H | Cl | H |
| H | Br | Br | H | Br | H | Br | H |
| H | $CH_3$ | $CH_3$ | H | $CH_3$ | H | $CH_3$ | H |
| H | $CH_3O$ | $CH_3O$ | H | $CH_3O$ | H | $CH_3O$ | H |
| H | CN | CN | H | CN | H | CN | H |
| F | F | | | | | | |
| F | $CH_3$ | | | | | | |
| $CH_3$ | F | | | | | | |

## TABLE 7

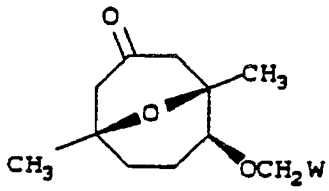

where W =

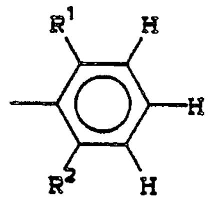

| $R^1$ | $R^2$ | $R^1$ | $R^2$ | $R^1$ | $R^2$ |
|---|---|---|---|---|---|
| H | H | Br | $CH_3$ | H | Cl |
| F | H | Br | $CH_3O$ | H | Br |
| Cl | H | Br | CN | H | $CH_3$ |
| Br | H | $CH_3$ | F | H | $CH_3O$ |
| $CH_3$ | H | $CH_3$ | Cl | H | CN |
| $CH_3O$ | H | $CH_3$ | Br | | |
| CN | H | $CH_3$ | $CH_3$ | | |
| F | F | $CH_3$ | $CH_3O$ | | |
| F | Cl | $CH_3$ | CN | | |
| F | Br | $CH_3O$ | F | | |
| F | $CH_3$ | $CH_3O$ | Cl | | |
| F | $CH_3O$ | $CH_3O$ | Br | | |
| F | CN | $CH_3O$ | $CH_3$ | | |
| Cl | F | $CH_3O$ | $CH_3O$ | | |
| Cl | Cl | $CH_3O$ | CN | | |
| Cl | Br | CN | F | | |
| Cl | $CH_3$ | CN | Cl | | |
| Cl | $CH_3O$ | CN | Br | | |
| Cl | CN | CN | $CH_3$ | | |
| Br | F | CN | $CH_3O$ | | |
| Br | Cl | CN | CN | | |
| Br | Br | H | F | | |

| W = W-10 | | | W = W-12 | | |
|---|---|---|---|---|---|
| $R^1$ | $R^2$ | $R^3$ | $R^1$ | $R^2$ | $R^3$ |
| H | H | H | H | H | H |
| F | H | H | F | H | H |
| Cl | H | H | Cl | H | H |
| Br | H | H | Br | H | H |
| $CH_3$ | H | H | $CH_3$ | H | H |
| $CH_3O$ | H | H | $CH_3O$ | H | H |
| CN | H | H | CN | H | H |
| H | F | H | H | F | H |
| H | Cl | H | H | Cl | H |
| H | Br | H | H | Br | H |
| H | $CH_3$ | H | H | $CH_3$ | H |
| H | $CH_3O$ | H | H | $CH_3O$ | H |
| H | CN | H | H | CN | H |
| H | H | F | H | H | F |
| H | H | Cl | H | H | Cl |
| H | H | Br | H | H | Br |
| H | H | $CH_3$ | H | H | $CH_3$ |
| H | H | $CH_3O$ | H | H | $CH_3O$ |
| H | H | CN | H | H | CN |

| W = W-28 | | W = W-27 | | W = W-26 | |
|---|---|---|---|---|---|
| $R^1$ | $R^2$ | $R^1$ | $R^2$ | $R^1$ | $R^2$ |
| H | H | H | H | H | H |
| H | F | H | F | H | F |
| H | Cl | H | Cl | H | Cl |
| H | Br | H | Br | H | Br |
| H | $CH_3$ | H | $CH_3$ | H | $CH_3$ |
| H | $CH_3O$ | H | $CH_3O$ | H | $CH_3O$ |
| H | CN | H | CN | H | CN |
| F | H | F | H | F | H |
| Cl | H | Cl | H | Cl | H |
| Br | H | Br | H | Br | H |
| $CH_3$ | H | $CH_3$ | H | $CH_3$ | H |
| $CH_3O$ | H | $CH_3O$ | H | $CH_3O$ | H |
| CN | H | CN | H | CN | H |

TABLE 8

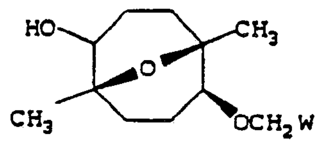

where W =

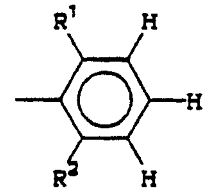

| $R^1$ | $R^2$ | $R^1$ | $R^2$ | $R^1$ | $R^2$ |
|---|---|---|---|---|---|
| H | H | Br | $CH_3O$ | H | $CH_3$ |
| F | H | Br | CN | H | $CH_3O$ |
| Cl | H | $CH_3$ | F | H | CN |
| Br | H | $CH_3$ | Cl | | |
| $CH_3$ | H | $CH_3$ | Br | | |
| $CH_3O$ | H | $CH_3$ | $CH_3$ | | |
| CN | H | $CH_3$ | $CH_3O$ | | |
| F | F | $CH_3$ | CN | | |
| F | Cl | $CH_3O$ | F | | |
| F | Br | $CH_3O$ | Cl | | |
| F | $CH_3$ | $CH_3O$ | Br | | |
| F | $CH_3O$ | $CH_3O$ | $CH_3$ | | |
| F | CN | $CH_3O$ | $CH_3O$ | | |
| Cl | F | $CH_3O$ | CN | | |
| Cl | Cl | CN | F | | |
| Cl | Br | CN | Cl | | |
| Cl | $CH_3$ | CN | Br | | |
| Cl | $CH_3O$ | CN | $CH_3$ | | |
| Cl | CN | CN | $CH_3O$ | | |
| Br | F | CN | CN | | |
| Br | Cl | H | F | | |
| Br | Br | H | Cl | | |
| Br | $CH_3$ | H | Br | | |

## TABLE 9

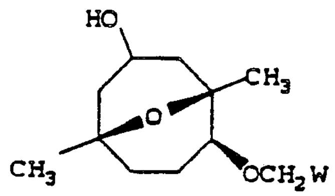

where W =

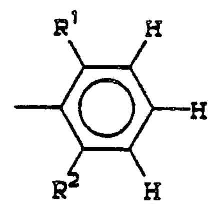

| $R^1$ | $R^2$ | $R^1$ | $R^2$ | $R^1$ | $R^2$ |
|---|---|---|---|---|---|
| H | H | Cl | $CH_3O$ | $CH_3O$ | $CH_3$ |
| F | H | Cl | CN | $CH_3O$ | $CH_3O$ |
| Cl | H | Br | F | $CH_3O$ | CN |
| Br | H | Br | Cl | CN | F |
| $CH_3$ | H | Br | Br | CN | Cl |
| $CH_3O$ | H | Br | $CH_3$ | CN | Br |
| CN | H | Br | $CH_3O$ | CN | $CH_3$ |
| F | F | Br | CN | CN | $CH_3O$ |
| F | Cl | $CH_3$ | F | CN | CN |
| F | Br | $CH_3$ | Cl | H | F |
| F | $CH_3$ | $CH_3$ | Br | H | Cl |
| F | $CH_3O$ | $CH_3$ | $CH_3$ | H | Br |
| F | CN | $CH_3$ | $CH_3O$ | H | $CH_3$ |
| Cl | F | $CH_3$ | CN | H | $CH_3O$ |
| Cl | Cl | $CH_3O$ | F | H | CN |
| Cl | Br | $CH_3O$ | Cl | | |
| Cl | $CH_3$ | $CH_3O$ | Br | | |

TABLE 10

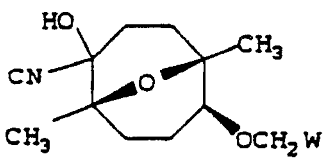

where W =

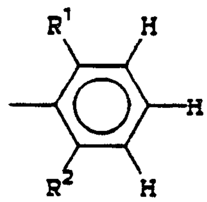

| $R^1$ | $R^2$ | $R^1$ | $R^2$ | $R^1$ | $R^2$ |
|---|---|---|---|---|---|
| H | H | Br | $CH_3$ | H | Cl |
| F | H | Br | $CH_3O$ | H | Br |
| Cl | H | Br | CN | H | $CH_3$ |
| Br | H | $CH_3$ | F | H | $CH_3O$ |
| $CH_3$ | H | $CH_3$ | Cl | H | CN |
| $CH_3O$ | H | $CH_3$ | Br | | |
| CN | H | $CH_3$ | $CH_3$ | | |
| F | F | $CH_3$ | $CH_3O$ | | |
| F | Cl | $CH_3$ | CN | | |
| F | Br | $CH_3O$ | F | | |
| F | $CH_3$ | $CH_3O$ | Cl | | |
| F | $CH_3O$ | $CH_3O$ | Br | | |
| F | CN | $CH_3O$ | $CH_3$ | | |
| Cl | F | $CH_3O$ | $CH_3O$ | | |
| Cl | Cl | $CH_3O$ | CN | | |
| Cl | Br | CN | F | | |
| Cl | $CH_3$ | CN | Cl | | |
| Cl | $CH_3O$ | CN | Br | | |
| Cl | CN | CN | $CH_3$ | | |
| Br | F | CN | $CH_3O$ | | |
| Br | Cl | CN | CN | | |
| Br | Br | H | F | | |

TABLE 11

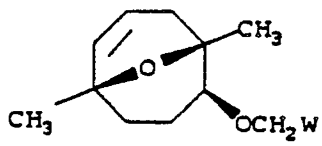

where W =

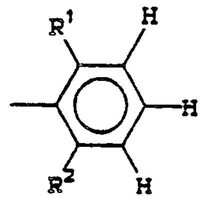

| $R^1$ | $R^2$ | $R^1$ | $R^2$ | $R^1$ | $R^2$ |
|---|---|---|---|---|---|
| H | H | Br | $CH_3O$ | H | $CH_3$ |
| F | H | Br | CN | H | $CH_3O$ |
| Cl | H | $CH_3$ | F | H | CN |
| Br | H | $CH_3$ | Cl | | |
| $CH_3$ | H | $CH_3$ | Br | | |
| $CH_3O$ | H | $CH_3$ | $CH_3$ | | |
| CN | H | $CH_3$ | $CH_3O$ | | |
| F | F | $CH_3$ | CN | | |
| F | Cl | $CH_3O$ | F | | |
| F | Br | $CH_3O$ | Cl | | |
| F | $CH_3$ | $CH_3O$ | Br | | |
| F | $CH_3O$ | $CH_3O$ | $CH_3$ | | |
| F | CN | $CH_3O$ | $CH_3O$ | | |
| Cl | F | $CH_3O$ | CN | | |
| Cl | Cl | CN | F | | |
| Cl | Br | CN | Cl | | |
| Cl | $CH_3$ | CN | Br | | |
| Cl | $CH_3O$ | CN | $CH_3$ | | |
| Cl | CN | CN | $CH_3O$ | | |
| Br | F | CN | CN | | |
| Br | Cl | H | F | | |
| Br | Br | H | Cl | | |
| Br | $CH_3$ | H | Br | | |

TABLE 12

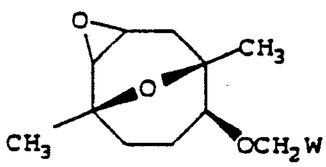

where W =

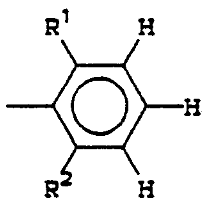

| $R^1$ | $R^2$ | $R^1$ | $R^2$ | $R^1$ | $R^2$ |
|---|---|---|---|---|---|
| H | H | Cl | CN | $CH_3O$ | CN |
| F | H | Br | F | CN | F |
| Cl | H | Br | Cl | CN | Cl |
| Br | H | Br | Br | CN | Br |
| $CH_3$ | H | Br | $CH_3$ | CN | $CH_3$ |
| $CH_3O$ | H | Br | $CH_3O$ | CN | $CH_3O$ |
| CN | H | Br | CN | CN | CN |
| F | F | $CH_3$ | F | H | F |
| F | Cl | $CH_3$ | Cl | H | Cl |
| F | Br | $CH_3$ | Br | H | Br |
| F | $CH_3$ | $CH_3$ | $CH_3$ | H | $CH_3$ |
| F | $CH_3O$ | $CH_3$ | $CH_3O$ | H | $CH_3O$ |
| F | CN | $CH_3$ | CN | H | CN |
| Cl | F | $CH_3O$ | F | | |
| Cl | Cl | $CH_3O$ | Cl | | |
| Cl | Br | $CH_3O$ | Br | | |
| Cl | $CH_3$ | $CH_3O$ | $CH_3$ | | |
| Cl | $CH_3O$ | $CH_3O$ | $CH_3O$ | | |

TABLE 13

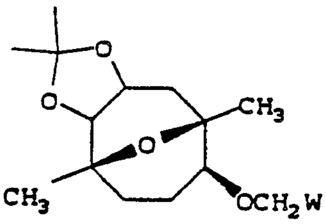

where W =

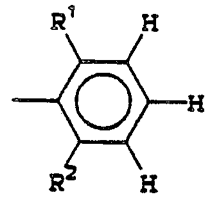

| $R^1$ | $R^2$ | $R^1$ | $R^2$ | $R^1$ | $R^2$ |
|---|---|---|---|---|---|
| H | H | Br | Br | CN | CN |
| F | H | Br | $CH_3$ | H | F |
| Cl | H | Br | $CH_3O$ | H | Cl |
| Br | H | Br | CN | H | Br |
| $CH_3$ | H | $CH_3$ | F | H | $CH_3$ |
| $CH_3O$ | H | $CH_3$ | Cl | H | $CH_3O$ |
| CN | H | $CH_3$ | Br | H | CN |
| F | F | $CH_3$ | $CH_3$ | | |
| F | Cl | $CH_3$ | $CH_3O$ | | |
| F | Br | $CH_3$ | CN | | |
| F | $CH_3$ | $CH_3O$ | F | | |
| F | $CH_3O$ | $CH_3O$ | Cl | | |
| F | CN | $CH_3O$ | Br | | |
| Cl | F | $CH_3O$ | $CH_3$ | | |
| Cl | Cl | $CH_3O$ | $CH_3O$ | | |
| Cl | Br | $CH_3O$ | CN | | |
| Cl | $CH_3$ | CN | F | | |
| Cl | $CH_3O$ | CN | Cl | | |
| Cl | CN | CN | Br | | |
| Br | F | CN | $CH_3$ | | |
| Br | Cl | CN | $CH_3O$ | | |

TABLE 14

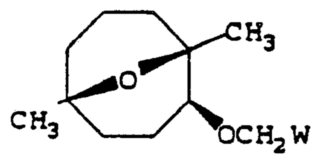

where W =

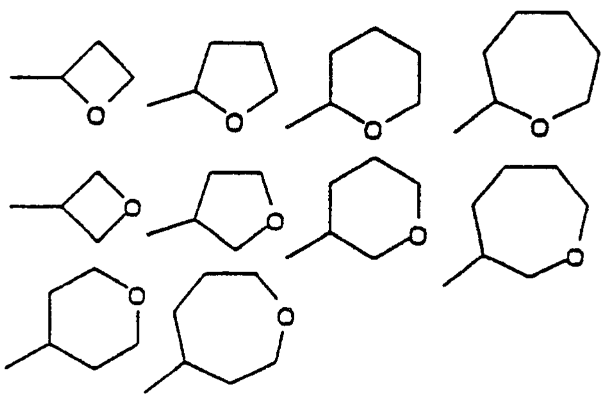

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

Table 15

| | Weight Percent* | | |
|---|---|---|---|
| | Active Ingredient | Diluent(s) | Surfactant(s) |
| Wettable Powders | 5-60 | 39-94 | 1-10 |
| Emulsifiable Concentrates | 3-80 | 20-95 | 0-20 |
| Dusts | 1-25 | 70-99 | 0-5 |
| Granules and Pellets | 0.1-50 | 50-99.9 | 0-15 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are some times desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

| Example 5 | |
|---|---|
| Wettable Powder | |
| 1,5-dimethyl-2-[(2-fluorophenyl)methoxy]-9-oxabicyclo[3.3.1]nonane | 60% |
| sodium alkylnaphthalenesulfonate | 2% |
| sodium ligninsulfonate | 2% |
| synthetic amorphous silica | 36% |

The active ingredient is first sprayed onto the amorphous silica, then the ingredients are blended, hammer-milled until all the solids are essentially under 50 microns, reblended, and packaged.

| Example 6 | |
|---|---|
| Wettable Powder | |
| 1,5-dimethyl-2-[(2,6-difluorophenyl)methoxy]-9-oxabicyclo[3.3.1]nonane | 50% |
| sodium alkylnaphthalenesulfonate | 2% |
| low viscosity methyl cellulose | 2% |
| diatomaceous earth | 46% |

The active ingredient is first sprayed onto the diatomaceous earth then the ingredients are blended, coarsely hammer-milled and then air-milled to produce particles essentially all below 10 microns in diameter. The product is reblended before packaging.

| Example 7 | |
|---|---|
| Granule | |
| Wettable Powder of Example 6 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

| Example 8 | |
|---|---|
| Emulsifiable Concentrate | |
| 1,5-dimethyl-2-[(2-fluorophenyl)methoxy]-9-oxabicyclo[3.3.1]nonane | 40% |
| Atlox 3403F | 3% |
| Atlox 3404F | 3% |
| xylene | 54% |

The active ingredient and Atlox emulsifiers are dissolved in the solvent, filtered and packaged. Atlox 3403F and 3404F are blends of anionic and ionic emulsifiers from ICI Americas, Inc.

| Example 9 | |
|---|---|
| Low Strength Granule | |
| 1,5-dimethyl-2-[(2,6-difluorophenyl)methoxy]-9-oxabicyclo[3.3.1]nonane | 5% |
| attapulgite granules (U.S.S. 20-40 mesh) | 95% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

| Example 10 | |
|---|---|
| Granule | |
| 1,5-dimethyl-2-[(2-fluorophenyl)methoxy]-9-oxabicyclo[3.3.1]nonane | 50% |
| wetting agent | 1% |
| crude ligninsulfonate salt (containing 5-20% of the natural sugars) | 10% |
| attapulgite clay | 39% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

| Example 11 | |
|---|---|
| Concentrated Emulsion | |
| 1,5-dimethyl-2-[(2-fluorophenyl)methoxy]-9-oxabicyclo[3.3.1]nonane | 25% |
| xylene | 25% |
| Atlox 3404F | 5% |
| G1284 | 5% |
| ethylene glycol | 8% |
| water | 32% |

The active ingredient, solvent and emulsifiers are blended together. This solution is added to a mixture of the ethylene glycol and water with stirring.

| Example 12 | |
|---|---|
| Solution | |
| 1,5-dimethyl-2-[(2-fluorophenyl)methoxy]-9-oxabicyclo[3.3.1]nonane | 5% |
| water | 95% |

The compound is added directly to the water with stirring to produce the solution, which may then be packaged for use.

| Example 13 | |
|---|---|
| Dust | |
| 1,5-dimethyl-2-[(2,6-difluorophenyl)methoxy]-9-oxabicyclo[3.3.1]nonane | 10% |
| attapulgite | 10% |
| Pyrophyllite | 80% |

The active ingredient is sprayed onto the attapulgite and then passed through a hammer-mill to produce particles substantially all below 200 microns. The ground concentrate is then blended with powdered pyrophyllite until homogeneous.

## Utility

Test results indicate that compounds of this invention are active postemergence and, in particular, preemergence herbicides. Many compounds in this invention are useful for the control of selected grass and broadleaf weeds with tolerance to important agronomic crops such as barley (Hordeum vulgare), corn (Zea mays), cotton (Gossypium hirsutum), rape (Brassica napus), rice (Oryza sativa), sorghum (Sorghum bicolor), soybean (Glycine max), sugar beet (Beta vulgaris), and wheat (Triticum aestivum). Grass weed species controlled include, but are not limited to, barnyardgrass (Echinochloa crus-galli), crabgrass (Digitaria spp.), and foxtail (Setaria spp.). Broadleaf weed species controlled include, but are not limited to, duck salad (Heteranthera limosa) and umbrella sedge (Cyperus difformis). Several compounds in this invention are particularly useful for the control of barnyardgrass, duck salad, and umbrella sedge in paddy rice and for the control of grass weeds such as barnyardgrass in upland rice. Utility in paddy rice includes both direct-seeded and transplanted paddy rice.

These compounds also have utility for weed control of selected vegetation in specified areas such as around storage tanks, parking lots, highways, and railways, and in fallow crop, citrus, and plantation crop areas. Alternatively, these compounds are useful to modify plant growth.

Rates of application for compounds of this invention are determined by a number of factors. These factors include: formulation selected, method of application, amount and type of vegetation present, growing

conditions, etc. In general terms, the subject compounds should be applied at rates from 0.008 to 20 kg/ha with a preferred rate range of 0.032 to 1 kg/ha. One skilled in the art can easily determine the application rate needed for the desired level of weed controls

Compounds of this invention may be used alone or in combination with other commercial herbicides, insecticides, or fungicides. The following list exemplifies some of the herbicides suitable for use in mixtures. A combination of a compound from this invention with one or more of the following herbicides may be particularly useful for weed control.

| Common Name | Chemical Name |
|---|---|
| acetochlor | 2-chloro-N-(ethoxymethyl)-N-(2-ethyl-6-methylphenyl)acetamide |
| acifluorfen | 5-[2-chloro-4-(trifluoromethyl)-phenoxy]-2-nitrobenzoic acid |
| acrolein | 2-propenal |
| alachlor | 2-chloro-N-(2,6-diethylphenyl)-N-(methoxymethyl)acetamide |
| anilofos | S-4-chloro-N-isopropylcarbaniloyl-methyl-O,O-dimethyl phosphorodi-thioate |
| ametryn | N-ethyl-N'-(1-methylethyl)-6-(methylthio)-1,3,5-triazine-2,4-diamine |
| amitrole | 1H-1,2,4-triazol-3-amine |
| AMS | ammonium sulfamate |
| asulam | methyl [(4-aminophenyl)sulfonyl]-carbamate |
| atrazine | 6-chloro-N-ethyl-N'-(1-methylethyl)-1,3,5-triazine-2,4-diamine |
| barban | 4-chloro-2-butynyl 3-chlorocarbamate |

| Common Name | Chemical Name |
|---|---|
| benefin | N-butyl-N-ethyl-2,6-dinitro-4-(trifluoromethyl)benzenamine |
| bensulfuron methyl | 2-[[[[[(4,6-dimethoxy-2-pyrimidinyl)amino]methylcarbonyl]-amino]sulfonyl]methyl]benzoic acid, methyl ester |
| bensulide | O,O-bis(1-methylethyl) S-[2-[(phenylsulfonyl)amino]-ethyl]phosphorodithioate |
| bentazon | 3-(1-methylethyl)-(1H)-2,1,3-benzothiadiazin-4(3H)-one, 2,2-dioxide |
| benzofluor | N-[4-(ethylthio)-2-(trifluoromethyl)phenyl]methanesulfonamide |
| benzoylprop | N-benzoyl-N-(3,4-dichlorophenyl)-DL-alanine |
| bifenox | methyl 5-(2,4-dichlorophenoxy)-2-nitrobenzoate |
| bromacil | 5-bromo-6-methyl-3-(1-methylpropyl)-2,4(1H,3H)pyrimidinedione |
| bromoxynil | 3,5-dibromo-4-hydroxybenzonitrile |
| butachlor | N-(butoxymethyl)-2-chloro-N-(2,6-diethylphenyl)acetamide |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)-carbamothioate |
| cacodylic acid | dimethyl arsinic oxide |
| CDAA | 2-chloro-N,N-di-2-propenylacetamide |
| CDEC | 2-chloroallyl diethyldithiocarbamate |

| Common Name | Chemical Name |
|---|---|
| CGA 142,464 | 3-(4,6-dimethoxy-1,3,5-triazin-2-yl)-1-[2-(2-methoxyethoxy)-pehnyl-sulfonyl]-urea |
| chloramben | 3-amino-2,5-dichlorobenzoic acid |
| chlorbromuron | 3-(4-bromo-3-chlorophenyl)-1-methoxy-1-methylurea |
| chlorimuron ethyl | 2-[[[[(4-chloro-6-methoxy-2-pyrimidinyl)ethylamino]carbonyl]-amino]sulfonyl]benzoic acid, ethyl ester |
| chlormethoxy-nil | 2,4-dichlorophenyl 4-nitro-3-methoxyphenyl ether |
| chlornitrofen | 2,4,6-trichlorophenyl-4-nitro-phenyl ether |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzene-sulfonamide |
| chlortoluron | N'-(3-chloro-4-methylphenyl)-N,N-dimethylurea |
| cinmethylin | exo-1-methyl-4-(1-methylethyl)-2-[(2-methylphenyl)methoxy]-7-oxabicyclo-[2.2.1]heptane |
| clethodim | (E,E)-(±)-2-[1-[[(3-chloro-2-propenyl)-oxy]imino]propyl]-5-[2-(ethylthio)-propyl]-3-hydroxy-2-cyclohexen-1-one |
| clomazone | 2-[(2-chlorophenyl)methyl]-4,4-dimethyl-3-isoxazolidinone |
| cloproxydim | (E,E)-2-[1-[[(3-chloro-2-propenyl)oxy)-imino]butyl]-5-[2-(ethylthio)propyl]-3-hydroxy-2-cyclohexen-1-one |

| Common Name | Chemical Name |
|---|---|
| clopyralid | 3,6-dichloro-2-pyridinecarboxylic acid |
| CMA | calcium salt of MAA |
| cyanazine | 2-[[4-chloro-6-(ethylamino)-1,3,5-triazin-2-yl]amino]-2-methylpropanenitrile |
| cycloate | S-ethyl cyclohexylethylcarbamothioate |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-s-triazine |
| cyprazole | N-[5-(2-chloro-1,1-dimethylethyl)-1,3,4-thiadiazol-2-yl]cyclopropanecarboxamide |
| cypromid | 3',4'-dichlorocyclopropanecarboxanilide |
| dalapon | 2,2-dichloropropanoic acid |
| dazomet | tetrahydro-3,5-dimethyl-2H-1,3,5-thiadiazine-2-thione |
| DCPA | dimethyl 2,3,5,6-tetrachloro-1,4-benzenedicarboxylate |
| desmediphan | ethyl [3-[[(phenylamino)carbonyl]oxy]phenyl]carbamate |
| desmetryn | 2-(isopropylamino)-4-(methylamino)-6-(methylthio)-s-triazine |
| diallate | S-(2,3-dichloro-2-propenyl)bis(1-methylethyl)carbamothioate |
| dicamba | 3,6-dichloro-2-methoxybenzoic acid |
| dichlobenil | 2,6-dichlorobenzonitrile |
| dichlorprop | (±)-2-(2,4-dichlorophenoxy)propanoic acid |
| dichlofop | (±)-2-[4-(2,4-dichlorophenoxy)phenoxy]-propanoic acid, methyl ester |

| Common Name | Chemical Name |
|---|---|
| diethatyl | N-(chloroacetyl)-N-(2,6-diethylphenyl)-glycine |
| difenzoquat | 1,2-dimethyl-3,5-diphenyl-1H-pyrazolium |
| dimepiperate | S-1-methyl-1-phenylethylpiperidine-1-carbothioate |
| dinitramine | $N^3$,$N^3$-diethyl-2,4-dinitro-6-(trifluoromethyl)-1,3-benzenediamine |
| dinoseb | 2-(1-methylpropyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-a:2',1'-c]-pyrazinedium ion |
| diuron | N'-(3,4-dichlorophenyl)-N,N-dimethylurea |
| DNOC | 2-methyl-4,6-dinitrophenol |
| DPX-M6316 | 3-[[[[(4-methoxy-6-methyl-1,3,5-triazin-2-yl)amino]carbonyl]amino]sulfonyl]-2-thiophenecarboxylic acid, methyl ester |
| DSMA | disodium salt of MAA |
| dymron | N-(4-methylphenyl)-N'-(1-methyl-1-phenylethyl)urea |
| endothall | 7-oxabicyclo[2.2.1]heptane-2,3-dicarboxylic acid |
| EPTC | S-ethyl dipropylcarbamothioate |
| esprocarb (SC2957) | S-benzyl-N-ethyl-N-(1,2-dimethyl)-propyl)thiolcarbamate |
| ethalfluralin | N-ethyl-N-(2-methyl-2-propenyl)-2,6-dinitro-4-(trifluoromethyl)-benzenamine |

| Common Name | Chemical Name |
|---|---|
| ethofumesate | (±)-2-ethoxy-2,3-dihydro-3,3-dimethyl-5-benzofuranyl methanesulfonate |
| Express® | 2-[[[[N-(4-methoxy-6-methyl-1,3,5-triazine-2-yl)-N-methylamino]-carbonyl]amino]sulfonyl]benzoic acid, methyl ester |
| fenac | 2,3,6-trichlorobenzeneacetic acid |
| fenoxaprop | (±)-2-[4-[(6-chloro-2-benzoxazolyl)oxy]-phenoxy]propanoic acid |
| fenuron | N,N-dimethyl-N'-phenylurea |
| fenuron TCA | Salt of fenuron and TCA |
| flamprop | N-benzoyl-N-(3-chloro-4-fluorophenyl)-DL-alanine |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)benzenamine |
| fluometuron | N,N-dimethyl-N'-[3-(trifluoromethyl)-phenyl]urea |
| fluorochloridone | 3-chloro-4-(chloromethyl)-1-[3-(trifluoromethyl)phenyl]-2-pyrrolidinone |
| fluorodifen | p-nitrophenyl α,α,α-trifluoro-2-nitro-p-tolyl ether |
| fluoroglycofen | carboxymethyl 5-[2-chloro-4-(trifluoromethyl)phenoxy]-2-nitrobenzoate |
| fluridone | 1-methyl-3-phenyl-5-[3-(trifluoromethyl)phenyl]-4(1H)-pyridinone |
| fomesafen | 5-[2-chloro-4-(trifluoromethyl)phenoxy]-N-(methylsulfonyl)-2-nitrobenzamide |
| fosamine | ethyl hydrogen (aminocarbonyl)-phosphate |

| Common Name | Chemical Name |
|---|---|
| glyphosate | N-(phosphonomethyl)glycine |
| haloxyfop | 2-[4-[[3-chloro-5-(trifluoromethyl)-2-pyridinyl]oxy]phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazamethabenz | 6-(4-isopropyl-4-methyl-5-oxo-2-imidazolin-2-yl)-*m*-toluic acid, methyl ester and 6-(4-isopropyl-4-methyl-5-oxo-2-imidazolin-2-yl)-*p*-toluic acid, methyl ester |
| imazapyr | (±)-2-[4,5-dihydro-4-methyl-4-(1-methylethyl)-5-oxo-1H-imidazol-2-yl]-3-pyridinecarboxylic acid |
| imazaquin | 2-[4,5-dihydro-4-methyl-4-(1-methylethyl)-5-oxo-1H-imidazol-2-yl]-3-quinolinecarboxylic acid |
| imazethapyr | (±)-2-[4,5-dihydro-4-methyl-4-(1-methylethyl)-5-oxo-1H-imidazol-2-yl]-5-ethyl-3-pyridinecarboxylic acid |
| ioxynil | 4-hydroxy-3,5-diiodobenzonitrile |
| isopropalin | 4-(1-methylethyl)-2,6-dinitro-N,N-dipropylbenzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]-phenyl-(1,1-dimethylethyl)carbamate |
| lactofen | (±)-2-ethoxy-1-methyl-2-oxoethyl 5-[2-chloro-4-(trifluoromethyl)phenoxy]-2-nitrobenzoate |

| Common Name | Chemical Name |
|---|---|
| lenacil | 3-cyclohexyl-6,7-dihydro-1H-cyclopenta-pyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methylphenoxy)acetic acid |
| MCPB | 4-(4-chloro-2-methylphenoxy)butanoic acid |
| MON 7200 | S,S-dimethyl-2-(difluoromethyl)-4-(2-methylpropyl)-6-(trifluoromethyl)-3,5-pyridinedicarbothionate |
| mecoprop | (±)-2-(4-chloro-2-methylphenoxy)-propanoic acid |
| mefenacet | 2-(2-benzothiazolyloxy-N-methyl-N-phenylacetamide |
| mefluidide | N-[2,4-dimethyl-5-[[(trifluoromethyl)-sulfonyl]amino]phenyl]acetamide |
| methal-propalin | N-(2-methyl-2-propenyl)-2,6-dinitro-N-propyl-4-(trifluoromethyl)benzenamide |
| methabenz-thiazuron | 1,3-dimethyl-3-(2-benzothiazolyl)urea |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)-N,N-dimethylurea |
| metolachlor | 2-chloro-N-(2-ethyl-6-methylphenyl)-N-(2-methoxy-1-methylethyl)acetamide |
| metribuzin | 4-amino-6-(1,1-dimethylethyl)-3-(methylthio)-1,2,4-triazin-5(4H)-one |

| Common Name | Chemical Name |
|---|---|
| metsulfuron methyl | 2-[[[[(4-methoxy-6-methyl-1,3,5-triazin-2-yl)amino]carbonyl]amino]sulfonyl]benzoic acid, methyl ester |
| MH | 1,2-dihydro-3,6-pyridazinedione |
| molinate | S-ethyl hexahydro-1H-azepine-1-carbothioate |
| monolinuron | 3-(p-chlorophenyl)-1-methoxy-1-methylurea |
| monuron | N'-(4-chlorophenyl)-N,N-dimethylurea |
| monuron TCA | Salt of monuron and TCA |
| MSMA | monosodium salt of MAA |
| napropamide | N,N-diethyl-2-(1-naphthalenyloxy)-propanamide |
| naptalam | 2-[(1-naphthalenylamino)carbonyl]-benzoic acid |
| neburon | 1-butyl-3-(3,4-dichlorophenyl)-1-methylurea |
| nitralin | 4-(methylsulfonyl)-2,6-dinitro-N,N-dipropylaniline |
| nitrofen | 2,4-dichloro-1-(4-nitrophenoxy)benzene |
| nitrofluorfen | 2-chloro-1-(4-nitrophenoxy)-4-(trifluoromethyl)benzene |
| norea | N,N-dimethyl-N'-(octahydro-4,7-methano-1H-inden-5-yl)urea 3aα,-4α,5α,7α,7aα-isomer |
| norflurazon | 4-chloro-5-(methylamino)-2-[3-(trifluoromethyl)phenyl]-3(2H)-pyridazinone |
| oryzalin | 4-(dipropylamino)-3,5-dinitrobenzenesulfonamide |

| Common Name | Chemical Name |
|---|---|
| oxadiazon | 3-[2,4-dichloro-5-(1-methylethoxy)-phenyl]-5-(1,1-dimethylethyl)-1,3,4-oxadiazol-2(3H)-one |
| oxyfluorfen | 2-chloro-1-(3-ethoxy-4-nitrophenoxy)-4-(trifluoromethyl)benzene |
| paraquat | 1,1'-dimethyl-4,4'-dipyridinium ion |
| pebulate | S-propyl butylethylcarbamothioate |
| pendimethalin | N-(1-ethylpropyl)-3,4-dimethyl-2,6-dinitrobenzenamine |
| perfluidone | 1,1,1-trifluoro-N-[2-methyl-4-(phenylsulfonyl)phenyl]methanesulfonamide |
| phenmedipham | 3-[(methoxycarbonyl)amino]phenyl (3-methylphenyl)carbamate |
| picloram | 4-amino-3,5,6-trichloro-2-pyridinecarboxylic acid |
| PPG-1013 | 5-[2-chloro-4-(trifluoromethyl)-phenoxy]-2-nitroacetophenone oxime-O-acetic acid, methyl ester |
| pretilachlor | α-chloro-2,6-diethyl-N-(2-propoxyethyl)acetanilide |
| procyazine | 2-[[4-chloro-6-(cyclopropylamino)-1,3,5-triazine-2-yl]amino]-2-methylpropanenitrile |
| profluralin | N-(cyclopropylmethyl)-2,6-dinitro-N-propyl-4-(trifluoromethyl)benzenamine |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propynyl)benzamide |
| propachlor | 2-chloro-N-(1-methylethyl)-N-phenylacetamide |

| Common Name | Chemical Name |
|---|---|
| propanil | N-(3,4-dichlorophenyl)propanamide |
| propazine | 6-chloro-N,N'-bis(1-methylethyl)-1,3,5-triazine-2,4-diamine |
| propham | 1-methylethyl phenylcarbamate |
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| pyrazolate | 4-(2,4-dichlorobenzoyl)-1,3-dimethylpyrazol-5-yl-p-toluenesulphonate |
| pyrazon | 5-amino-4-chloro-2-phenyl-3(2H)-pyridazinone |
| pyrazosulfuron ethyl | ethyl S-[3-(4,6-dimethoxypyrimidin-2-yl)ureadosulfonyl]-1-methylpyrazole-4-carboxylate |
| quinclorac | 3,7-dichloro-8-quinoline carboxylic acid |
| quizalofop ethyl | (±)-2-[4-[(6-chloro-2-quinoxalinyl)-oxy]phenoxy]propanoic acid, ethyl ester |
| secbumeton | N-ethyl-6-methoxy-N'-(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexen-1-one |
| siduron | N-(2-methylcyclohexyl)-N'-phenylurea |
| simazine | 6-chloro-N,N'-diethyl-1,3,5-triazine-2,4-diamine |
| SK-233 | 1-(α,α-dimethylbenzyl)-3-(4-methylphenyl)urea |
| sulfometuron methyl | 2-[[[[(4,6-dimethyl-2-pyrimidinyl)-amino]carbonyl]amino]sulfonyl]-benzoic acid, methyl ester |

| Common Name | Chemical Name |
|---|---|
| TCA | trichloroacetic acid |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4(1H,3H)-pyrimidinedione |
| terbuchlor | N-(butoxymethyl)-2-chloro-N-[2-(1,1-dimethylethyl)-6-methylphenyl]-acetamide |
| terbuthylazine | 2-(*tert*-butylamino)-4-chloro-6-(ethylamino)-*s*-triazine |
| terbutol | 2,6-di-*tert*-butyl-*p*-tolyl methylcarbamate |
| terbutryn | N-(1,1-dimethylethyl)-N'-ethyl-6-(methylthio)-1,3,5-triazine-2,4-diamine |
| thiobencarb | S-[(4-chlorophenyl)methyl] diethylcarbamothioate |
| triallate | S-(2,3,3-trichloro-2-propenyl) bis(1-methylethyl)carbamothioate |
| triclopyr | [(3,5,6-trichloro-2-pyridinyl)-oxy]acetic acid |
| tridiphane | 2-(3,5-dichlorophenyl)-2-(2,2,2-trichloroethyl)oxirane |
| trifluralin | 2,6-dinitro-N,N-dipropyl-4-(trifluoromethyl)benzenamine |
| trimeturon | 1-(*p*-chlorophenyl)-2,3,3-trimethylpseudourea |
| 2,4-D | (2,4-dichlorophenoxy)acetic acid |
| 2,4-DB | 4-(2,4-dichlorophenoxy)butanoic acid |
| vernolate | S-propyl dipropylcarbamothioate |
| xylachlor | 2-chloro-N-(2,3-dimethylphenyl)-N-(1-methylethyl)acetamide |

Herbicidal properties of the subject compounds were discovered in a number of greenhouse tests. Test procedures and results follow.

COMPOUND TABLES

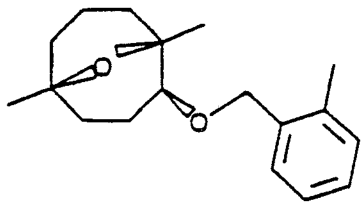

Compound 1
oil

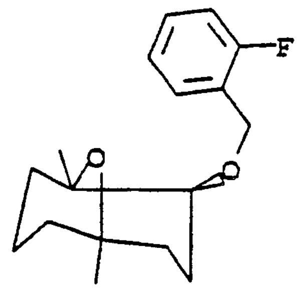

Compound 2
oil

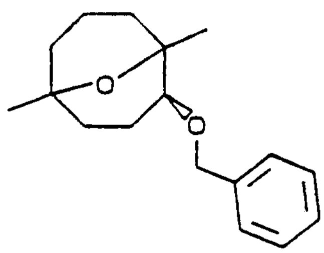

Compound 3
oil

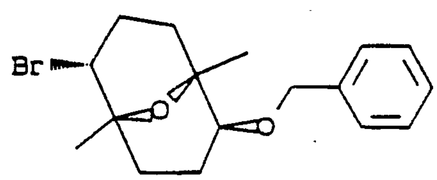

Compound 4
oil

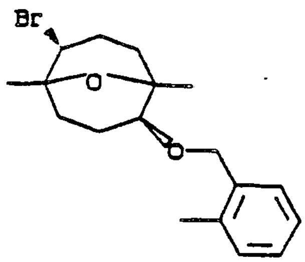

Compound 5
oil

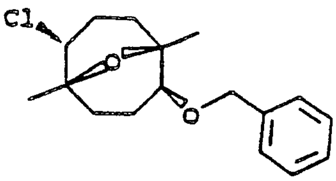

Compound 6
oil

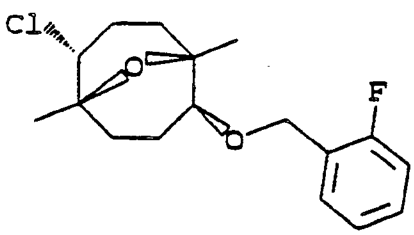

Compound 7
oil

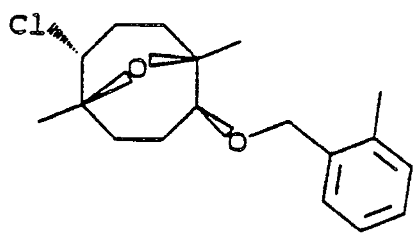

Compound 8
oil

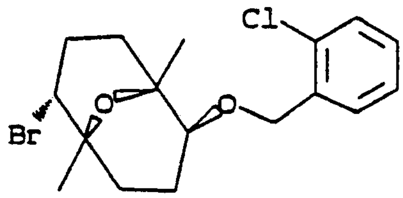

Compound 9
oil

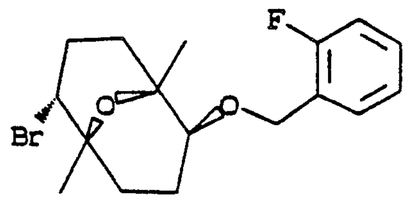

Compound 10
oil

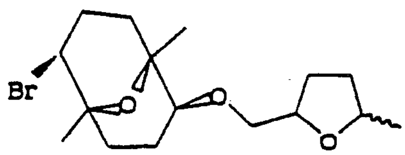

Compound 11
oil

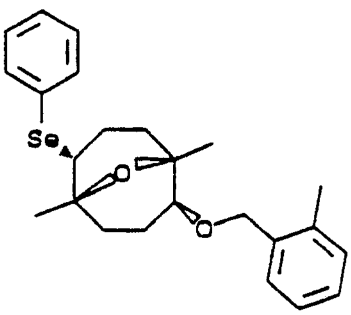

Compound 12
oil

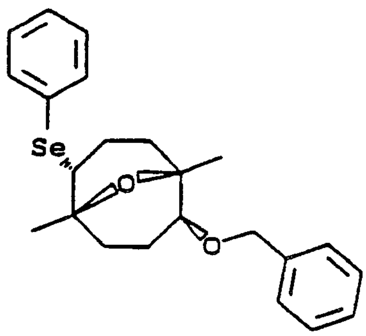

Compound 13
oil

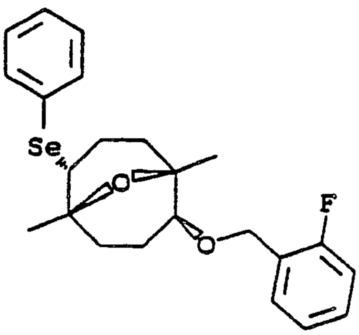

Compound 14
oil

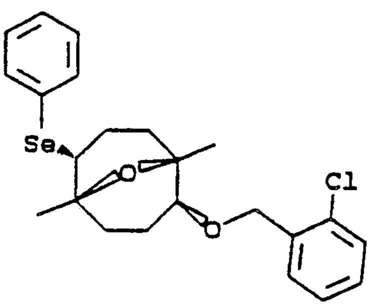

Compound 15
oil

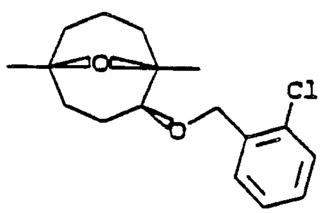

Compound 16
oil

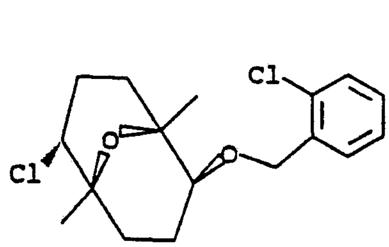

Compound 17
oil

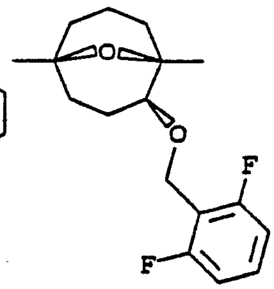

Compound 18
oil

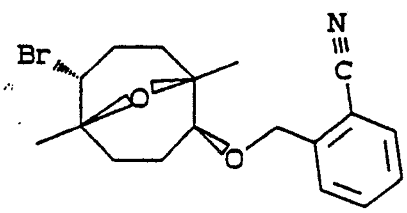

Compound 19
oil

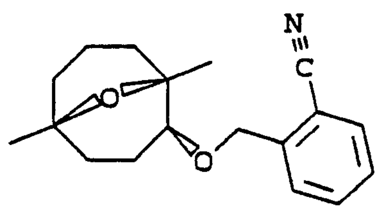

Compound 20
oil

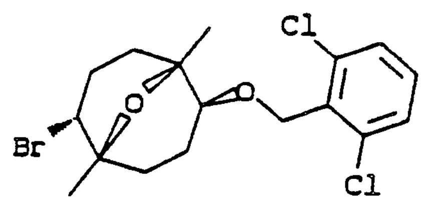

Compound 21
m p. 93-99°C

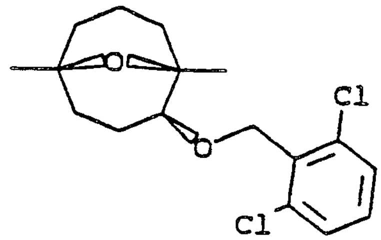

Compound 22
m p. 85-92°C

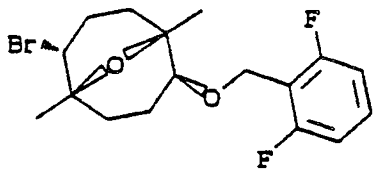

Compound 23
m p. 70-73°C

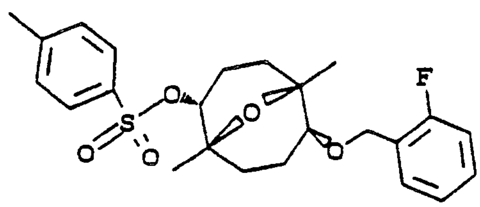

Compound 24
oil

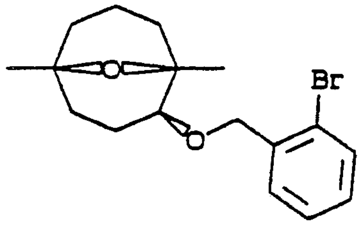

Compound 25
oil

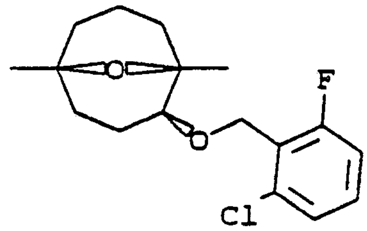

Compound 26
oil

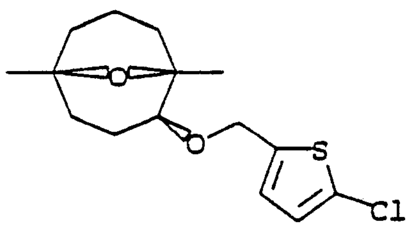

Compound 27
oil

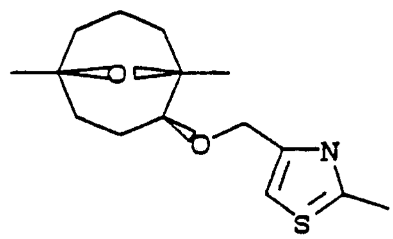

Compound 28
oil

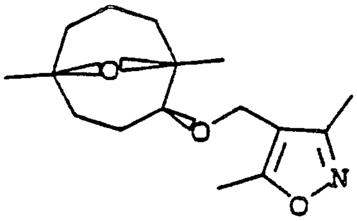

Compound 29
oil

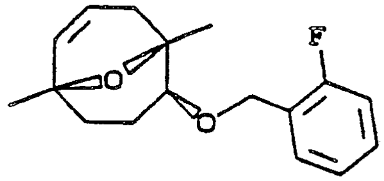

Compound 30
oil

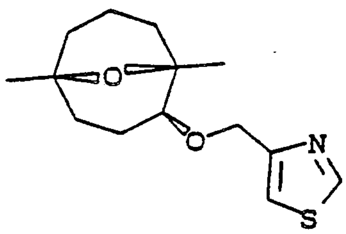

Compound 31
oil

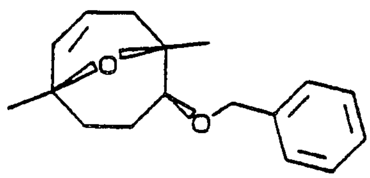

Compound 32
oil

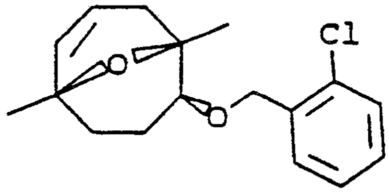

Compound 33
oil

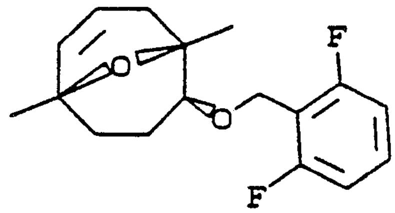

Compound 34
oil

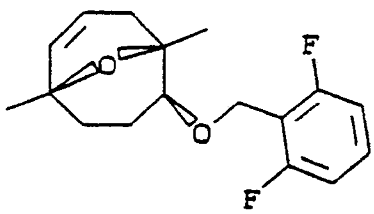

Compound 35
m p. 105-108°C

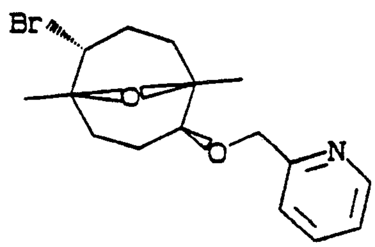

Compound 36
oil

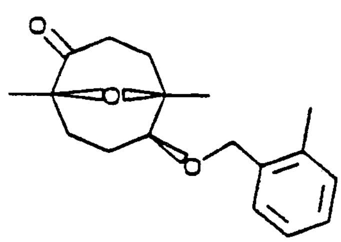

Compound 37
m p. 52-57°C

TEST A

Seeds of barley (Hordeum vulgare), barnyardgrass (Echinochloa crus-galli), cheatgrass (Bromus secalinus), cocklebur (Xanthium pensylvanicum), corn (Zea mays), cotton (Gossypium hirsutum), crabgrass (Digitaria spp.), giant foxtail (Setaria faberi), morningglory (Ipomoea spp.), rice (Oryza sativa), sorghum (Sorghum bicolor), soybean (Glycine max), sugar beet (Beta vulgaris), velvetleaf (Abutilon theophrasti),

wheat (Triticum aestivum), and wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treated plants and controls were maintained in a greenhouse for approximately sixteen days, after which all species were compared to controls and visually evaluated. Plant response ratings, summarized in Table A, are based on a scale of 0 to 10 where 0 is no effect and 10 is complete control. A dash (-) response means no test result. The accompanying descriptive symbols have the following meanings:

B = contact burn;
C = chlorosis/necrosis;
G = growth retardation;
H = formative effect; and
S = albinism.

Table A

| | Cmpd 1 | | Cmpd 2 | |
|---|---|---|---|---|
| RATE (g/ha) | 200 | 50 | 200 | 50 |
| POSTEMERGENCE | | | | |
| Barley | 0 | 0 | 0 | 0 |
| Barnyardgrass | 3C,8H | 0 | 9H | 5H |
| Cheatgrass | 0 | 0 | 0 | 0 |
| Cocklebur | 0 | 0 | 1C | 0 |
| Corn | 0 | 0 | 0 | 0 |
| Cotton | 0 | 0 | 0 | 0 |
| Crabgrass | 3C,8G | - | 9H | 2C,7G |
| Giant foxtail | 8G | 0 | 9G | 3C,7H |
| Morningglory | 0 | 0 | 0 | 0 |
| Nutsedge | 0 | 0 | 0 | 0 |
| Rice | 0 | 0 | 0 | 0 |
| Sorghum | 0 | 0 | 0 | 0 |
| Soybean | 0 | 0 | 0 | 0 |
| Sugar beet | 0 | 0 | 0 | 0 |
| Velvetleaf | 2G | 0 | 3G | 0 |
| Wheat | 0 | 0 | 0 | 0 |
| Wild oat | 0 | 0 | 0 | 0 |
| PREEMERGENCE | | | | |
| Barley | 0 | 0 | 5G | 0 |
| Barnyardgrass | 9H | 3C,8H | 10H | 3C,9H |
| Cheatgrass | 0 | 0 | 5G | 0 |
| Cocklebur | 5G | 0 | - | 0 |
| Corn | 0 | 0 | 2C,5G | 0 |
| Cotton | 0 | 0 | 1H | 0 |
| Crabgrass | 2C,9H | 2C,7G | 3C,9H | 2C,8G |
| Giant foxtail | 3C,8H | 2C,5G | 3C,9H | 3C,7G |
| Morningglory | 3H | 0 | 0 | 0 |
| Nutsedge | 0 | 0 | 0 | 0 |
| Rice | 0 | 0 | 0 | 0 |
| Sorghum | 0 | 0 | 0 | 0 |
| Soybean | 0 | 0 | 0 | 0 |
| Sugar beet | 0 | 0 | 2H | 0 |
| Velvetleaf | 2C,5H | 0 | 3H | 0 |
| Wheat | 0 | 0 | 2C,5G | 0 |
| Wild oat | 0 | 0 | 2C,8G | 0 |

| | Cmpd 3 | | | Cmpd 4 | |
|---|---|---|---|---|---|
| RATE (g/ha) | 200 | 100 | 50 | 200 | 50 |
| POSTEMERGENCE | | | | | |
| Barley | 0 | 0 | 0 | 0 | 0 |
| Barnyardgrass | 3C,8H | 2C,5H | 0 | 2G | 0 |
| Cheatgrass | 0 | 0 | 0 | 0 | 0 |
| Cocklebur | 1B | 1B | 0 | 1B | 1B |
| Corn | 0 | 0 | 0 | 0 | 0 |
| Cotton | 0 | 0 | 0 | 0 | 0 |
| Crabgrass | 3C,8H | 3C,5G | 0 | - | 0 |
| Giant foxtail | 3C,9H | 2C,5H | 0 | 4G | 0 |
| Morningglory | 0 | 0 | 0 | 0 | 0 |
| Nutsedge | 0 | 0 | 0 | 0 | 0 |
| Rice | 0 | 0 | 0 | 0 | 0 |
| Sorghum | 0 | 0 | 0 | 0 | 0 |
| Soybean | 0 | 0 | 0 | 0 | 0 |
| Sugar beet | 0 | 0 | 0 | 0 | 0 |
| Velvetleaf | 0 | 0 | 0 | 0 | 0 |
| Wheat | 0 | 0 | 0 | 0 | 0 |
| Wild oat | 0 | 0 | 0 | 0 | 0 |
| PREEMERGENCE | | | | | |
| Barley | 0 | 0 | 0 | 0 | 0 |
| Barnyardgrass | 5C,9H | 3C,9H | 3C,7H | 3C,9H | 3C,6G |
| Cheatgrass | 0 | 0 | 0 | 0 | 0 |
| Cocklebur | 0 | 0 | 0 | 0 | 0 |
| Corn | 1H | 0 | 0 | 0 | 0 |
| Cotton | 0 | 0 | 0 | 0 | 0 |
| Crabgrass | 5C,8G | 3C,6G | 1C,5G | 2C,8G | 0 |
| Giant foxtail | 4C,8H | 5G | 4G | 3C,9H | 2C,5G |
| Morningglory | 0 | 0 | 0 | 0 | 0 |
| Nutsedge | 5G | 0 | 0 | 0 | 0 |
| Rice | 0 | 0 | 0 | 0 | 0 |
| Sorghum | 0 | 0 | 0 | 0 | 0 |
| Soybean | 0 | 0 | 0 | 0 | 0 |
| Sugar beet | 0 | 0 | 0 | 0 | 0 |
| Velvetleaf | 2H | 1H | 0 | 2H | 0 |
| Wheat | 0 | 0 | 0 | 0 | 0 |
| Wild oat | 0 | 0 | 0 | 0 | 0 |

| | Cmpd 5 | | | Cmpd 6 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 2G | 0 | 0 |
| Cheatgrass | 0 | 0 | 0 | 0 | 0 | 0 |
| Cocklebur | 1B | 1B | 0 | 1B | 1B | 0 |
| Corn | 0 | 0 | 0 | 0 | 0 | 0 |
| Cotton | 0 | 0 | 0 | 0 | 0 | 0 |
| Crabgrass | 0 | 0 | 0 | 2C,7G | 0 | 0 |
| Giant foxtail | 2G | 0 | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 2G | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 2C,6G | 2G | 0 | 3C,9H | 3C,9 | 3C,8H |
| Cheatgrass | 3G | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 2C,5G | 0 | 0 | 4C,8G | 2C,7 | 3G |
| Giant foxtail | 7G | 2G | 0 | 4C,8H | 3C,7 | 2C,5G |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 5G | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 2H | 0 | 0 |
| Wheat | 0 | 0 | 0 | 0 | 0 | 0 |
| Wild oat | 0 | 0 | 0 | 0 | 0 | 0 |

| | Cmpd 7 | | | Cmpd 8 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 2H | 0 | 0 | 1C | 0 | 0 |
| Cheatgrass | 0 | 0 | 0 | 0 | 0 | 0 |
| Cocklebur | 1B | 1B | 1B | 1B | 1B | 1B |
| Corn | 0 | 0 | 0 | 0 | 0 | 0 |
| Cotton | 0 | 0 | 0 | 0 | 0 | 0 |
| Crabgrass | 3C,7G | 2C,5G | 0 | 2C,8G | 0 | 0 |
| Giant foxtail | 0 | 0 | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 2G | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3C,9H | 0 | 3C,5H | 3C,9H | 3C,8H | 0 |
| Cheatgrass | 0 | 0 | 0 | 0 | 0 | 0 |
| Cocklebur | 0 | 0 | 0 | 0 | 0 | 0 |
| Corn | 3H | 0 | 0 | 0 | 0 | 0 |
| Cotton | 0 | 0 | 0 | 0 | 0 | 0 |
| Crabgrass | 4C,9G | 3C,8H | 2C,6G | 4C,8G | 2G | 0 |
| Giant foxtail | 3C,8H | 3C,9H | 2C,8G | 3C,8G | 2C,5G | 0 |
| Morningglory | 0 | 0 | 0 | 0 | 0 | 0 |
| Nutsedge | 9G | 3C,8H | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 2H | 7G | 0 | 2H | 0 | 0 |
| Wheat | 0 | 0 | 0 | 0 | 0 | 0 |
| Wild oat | 0 | 0 | 0 | 0 | 0 | 0 |

| | Cmpd 9 | | | Cmpd 10 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 2H | 0 | 0 |
| Cheatgrass | 0 | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 0 | 0 | 0 | 0 | 0 | 0 |
| Giant foxtail | 0 | 0 | 0 | 0 | 0 | 0 |
| Morningglory | 0 | 0 | 0 | 0 | 0 | 0 |
| Nutsedge | - | 0 | 0 | - | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 9H | 5H | 2H | 9H | 9H | 9H |
| Cheatgrass | 3G | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 9H | 8H | 7H | 3C,9G | 2C,9G | 9G |
| Giant foxtail | 8H | 6H | 3H | 3C,9H | 3C,8H | 3C,8H |
| Morningglory | 0 | 0 | 0 | 0 | 0 | 0 |
| Nutsedge | 4G | 0 | - | 0 | 0 | 0 |
| Rice | 0 | 0 | 0 | 0 | 0 | 0 |
| Sorghum | 2G | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 2H | 0 | 0 |
| Wheat | 0 | 0 | 0 | 0 | 0 | 0 |
| Wild oat | 0 | 0 | 0 | 0 | 0 | 0 |

| | Cmpd 11 | | | Cmpd 12 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 1H | 0 | 0 |
| Cheatgrass | 0 | 0 | 0 | 0 | 0 | 0 |
| Cocklebur | 0 | 0 | 0 | 1B | 1B | 0 |
| Corn | 0 | 0 | 0 | 0 | 0 | 0 |
| Cotton | 0 | 0 | 0 | 0 | 0 | 0 |
| Crabgrass | 0 | 0 | 0 | 2C,6G | 2G | 0 |
| Giant foxtail | 0 | 0 | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 2G | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 2C,4H | 0 | 0 | 3C,8H | 5G | 0 |
| Cheatgrass | 0 | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 2G | 0 | 0 | 0 | 0 | 0 |
| Crabgrass | 9H | 7G | 5G | 3C,8H | 6G | 4G |
| Giant foxtail | 2C,8H | 2H | 0 | 7G | 2G | 0 |
| Morningglory | 0 | 0 | 0 | 0 | 0 | 0 |
| Nutsedge | 4G | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 1C,2H | 0 | 0 | 0 | 0 | 0 |
| Velvetleaf | 6G | - | 0 | 0 | 0 | 0 |
| Wheat | 0 | 0 | 0 | 0 | 0 | 0 |
| Wild oat | 0 | 0 | 0 | 0 | 0 | 0 |

| | Cmpd 13 | | | Cmpd 14 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 1H | 0 | 0 | 1H | 0 | 0 |
| Cheatgrass | 0 | 0 | 0 | 0 | 0 | 0 |
| Cocklebur | 1B | 1B | 0 | 1B | 0 | 0 |
| Corn | 0 | 0 | 0 | 0 | 0 | 0 |
| Cotton | 5H | 0 | 0 | 4H | 0 | 0 |
| Crabgrass | 3G | 5G | 0 | 2G | 3G | 0 |
| Giant foxtail | 0 | 0 | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 2H | 1H | 0 | 1H | 0 | 0 |
| Sugar beet | 0 | 0 | 0 | 0 | 0 | 0 |
| Velvetleaf | 1H | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 9H | 2C,8H | 4G | 3C,9H | 3C,8H | 1H |
| Cheatgrass | 2G | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 4C,9H | 2C,8G | 2G | 4C,9G | 2C,7G | 2C,5G |
| Giant foxtail | 3C,8H | 2C,7G | 3G | 3C,7G | 2C,6H | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 1H | 0 | 0 | 0 | 0 | 0 |
| Wheat | 0 | 0 | 0 | 0 | 0 | 0 |
| Wild oat | 0 | 0 | 0 | 0 | 0 | 0 |

| | Cmpd 15 | | | Cmpd 16 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 1H | 0 | 0 |
| Cheatgrass | 0 | 0 | 0 | 0 | 0 | 0 |
| Cocklebur | 1B | 1B | 0 | 2G | 0 | 0 |
| Corn | 0 | 0 | 0 | 0 | 0 | 0 |
| Cotton | 0 | 0 | 0 | 0 | 0 | 0 |
| Crabgrass | 2C,8G | 3G | 0 | 0 | 0 | 0 |
| Giant foxtail | 0 | 0 | 0 | 0 | 0 | 0 |
| Morningglory | 0 | 0 | 0 | 0 | 0 | 0 |
| Nutsedge | 0 | 0 | 0 | 0 | 0 | - |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 1H | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3C,7H | 3C,5G | 2G | 9H | 3C,9H | 2C,7G |
| Cheatgrass | 0 | 0 | 0 | 2G | 0 | 0 |
| Cocklebur | 3G | 0 | 0 | - | 0 | - |
| Corn | 0 | 0 | 0 | 0 | 0 | 0 |
| Cotton | 0 | 0 | 0 | 0 | 0 | 0 |
| Crabgrass | 3C,9G | 2C,5G | 0 | 9H | 2C,8H | 7G |
| Giant foxtail | 3C,7H | 2C,5G | 0 | 9H | 2C,8G | 5G |
| Morningglory | 0 | 0 | 0 | 0 | 0 | 0 |
| Nutsedge | 0 | 0 | 0 | 2G | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 3H | 0 | 0 |
| Wheat | 0 | 0 | 0 | 0 | 0 | 0 |
| Wild oat | 0 | 0 | 0 | 1H | 0 | 0 |

| | Cmpd 17 | | | Cmpd 18 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 7G | 0 | 0 |
| Barnyardgrass | 4H | 0 | 0 | 8H | 5H | 0 |
| Cheatgrass | 0 | 0 | 0 | 0 | 0 | 0 |
| Cocklebur | 1B | 1B | 0 | 2B | 1B | 0 |
| Corn | 0 | 0 | 0 | 0 | 0 | 0 |
| Cotton | 0 | 0 | 0 | 0 | 0 | 0 |
| Crabgrass | 5G | 0 | 0 | - | 0 | 0 |
| Giant foxtail | 0 | 0 | 0 | 1H | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 1H | 0 | 0 | 5G | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3C,9H | 7H | 7H | 9H | 9H | 8H |
| Cheatgrass | 0 | 0 | 0 | 5G | 0 | 0 |
| Cocklebur | 0 | 0 | 0 | 0 | 0 | 0 |
| Corn | 0 | 0 | 0 | 3H | 0 | 0 |
| Cotton | 0 | 0 | 0 | 0 | 0 | 0 |
| Crabgrass | 9H | 3C,9H | 6G | 3C,8H | 6G | 4G |
| Giant foxtail | 3C,9H | 6H | 3G | 9H | 9H | 7H |
| Morningglory | 0 | 0 | 0 | 0 | 0 | 0 |
| Nutsedge | 0 | 0 | 0 | 0 | 0 | 0 |
| Rice | 2G | 0 | 0 | 0 | 0 | 0 |
| Sorghum | 4G | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 1H | 0 | 0 | 4H | 1H | 0 |
| Wheat | 0 | 0 | 0 | 0 | 0 | 0 |
| Wild oat | 0 | 0 | 0 | 5G | 0 | 0 |

| | Cmpd 19 | | | Cmpd 20 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 1H | 0 | 0 |
| Cheatgrass | 0 | 0 | 0 | 0 | 0 | 0 |
| Cocklebur | 0 | 0 | 0 | 1B | 1B | 1B |
| Corn | 0 | 0 | 0 | 0 | 0 | 0 |
| Cotton | 0 | 0 | 0 | 0 | 0 | 0 |
| Crabgrass | 0 | 0 | 0 | 1H | 0 | 0 |
| Giant foxtail | 4G | 2G | 0 | 0 | 0 | 0 |
| Morningglory | 0 | 0 | 0 | 0 | 0 | 0 |
| Nutsedge | 2G | 0 | 0 | - | 0 | 0 |
| Rice | 2G,3C | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 1C,2G | 1C | 0 | 0 | 0 | 0 |
| Velvetleaf | 0 | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3H | 3H | 0 | 4C,9H | 3C,7H | 1C,5G |
| Cheatgrass | 0 | 0 | 0 | 0 | 0 | 0 |
| Cocklebur | 6G | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 2C,4G | 4G | - | 5S,9G | 3S,6G | 1C,3G |
| Giant foxtail | 5G | 5G | 4G | 9H | 9H | 7G |
| Morningglory | 0 | 0 | 0 | 0 | 0 | 0 |
| Nutsedge | 4G | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 2C,2H | 0 | 0 | 0 | 0 | 0 |
| Velvetleaf | 0 | 0 | 0 | 4H | 0 | 0 |
| Wheat | 0 | 0 | 0 | 0 | 0 | 0 |
| Wild oat | 0 | 0 | 0 | 0 | 0 | 0 |

| | Cmpd 21 | | | Cmpd 22 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 2H | 0 | 0 | 0 | 0 | 0 |
| Cheatgrass | 0 | 0 | 0 | 0 | 0 | 0 |
| Cocklebur | 0 | 0 | 0 | 1B | 1B | 0 |
| Corn | 0 | 0 | 0 | 0 | 0 | 0 |
| Cotton | 0 | 0 | 0 | 3H | 0 | 0 |
| Crabgrass | 4G | 3G | 0 | 7G | 2C,4G | 0 |
| Giant foxtail | 4G | 3G | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 1C,2G | 0 | 0 | 0 | 0 | 0 |
| Sugar beet | 2G | 0 | 0 | 0 | 0 | 0 |
| Velvetleaf | 0 | 0 | 0 | 1H | 2H | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 4H | 0 | 0 | 9H | 3C,8H | 7H |
| Cheatgrass | 0 | 0 | 0 | 0 | 0 | 0 |
| Cocklebur | 0 | 0 | 0 | - | 0 | 0 |
| Corn | 0 | 0 | 0 | 0 | 0 | 0 |
| Cotton | 0 | 0 | 0 | 0 | 0 | 0 |
| Crabgrass | 9G | 5G | 4G | 3C,7H | 3C,5G | 1C |
| Giant foxtail | 3C,9G | 3C,7G | 3G | 8H | 7G | 7G |
| Morningglory | 0 | 0 | 0 | 0 | 0 | 0 |
| Nutsedge | 3G | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 3H | 0 | 0 |
| Wheat | 0 | 0 | 0 | 0 | 0 | 0 |
| Wild oat | 0 | 0 | 0 | 0 | 0 | 0 |

| | Cmpd 23 | | | Cmpd 24 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 2C,6H | 2C,4G | 0 | 0 | 0 | 0 |
| Cheatgrass | 0 | 0 | 0 | 0 | 0 | 0 |
| Cocklebur | 1B | 0 | 0 | 1C | 0 | 0 |
| Corn | 0 | 0 | 0 | 0 | 0 | 0 |
| Cotton | 0 | 0 | 0 | 0 | 0 | 0 |
| Crabgrass | 0 | 0 | 0 | 0 | 0 | 0 |
| Giant foxtail | 0 | 0 | 0 | 0 | 0 | 0 |
| Morningglory | 0 | 0 | 0 | 0 | 0 | 0 |
| Nutsedge | - | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 1H | 0 | 0 | 0 | 0 | 0 |
| Sugar beet | 0 | 0 | 0 | 0 | 0 | 0 |
| Velvetleaf | 2G | 0 | 0 | 1H | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3C,9H | 9H | 5G | 2C,7H | 0 | 0 |
| Cheatgrass | 3G | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 9H | 9H | 2C,7H | 2G | 3G | 0 |
| Giant foxtail | 9H | 8H | 7H | 2H | 0 | 0 |
| Morningglory | 0 | 0 | 0 | 0 | 0 | 0 |
| Nutsedge | 0 | 0 | 0 | 0 | - | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 3H | 0 | 0 | 0 | 0 | 0 |
| Wheat | 0 | 0 | 0 | 0 | 0 | 0 |
| Wild oat | 0 | 0 | 0 | 0 | 0 | 0 |

| | Cmpd 25 | | | Cmpd 26 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 5H | 2H | 0 | 2H | 2H | 0 |
| Cheatgrass | 0 | 0 | 0 | 0 | 0 | 0 |
| Cocklebur | 1C | 0 | 0 | 2C | 1C | 2C |
| Corn | 0 | 0 | 0 | 0 | 0 | 0 |
| Cotton | 6H | 0 | 0 | 0 | 0 | 0 |
| Crabgrass | 2G | 0 | 0 | 8H | - | 0 |
| Giant foxtail | 0 | 0 | 0 | 0 | 0 | 0 |
| Morningglory | 0 | 0 | 0 | 0 | 0 | 0 |
| Nutsedge | - | 0 | - | 0 | 0 | 0 |
| Rice | 0 | 0 | 0 | 0 | 0 | 0 |
| Sorghum | 3G | 0 | 0 | 0 | 0 | 0 |
| Soybean | 0 | 0 | 0 | 0 | 0 | 0 |
| Sugar beet | 2C,3H | 2H | 0 | 0 | 0 | 0 |
| Velvetleaf | 2C,6G | 4G | 0 | 2C,7G | 3G | 2G |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 10H | 9H | 0 | 9H | 9H | 9H |
| Cheatgrass | 3G | 0 | 0 | 2G | 0 | 0 |
| Cocklebur | 2H | 0 | - | - | - | - |
| Corn | 0 | 0 | 0 | 0 | 0 | 0 |
| Cotton | 0 | 0 | 0 | 0 | 0 | 0 |
| Crabgrass | 4C,8H | 3C,6H | 6H | 10H | 4C,9H | 4C,8H |
| Giant foxtail | 9H | 8H | 6H | 9H | 9H | 8H |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 3H | 0 | 0 | 2C,6H | 2C,4H | 0 |
| Wheat | 0 | 0 | 0 | 0 | 0 | 0 |
| Wild oat | 0 | 0 | 0 | 0 | 0 | 0 |

| | Cmpd 27 | | | Cmpd 28 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 0 | 0 | 0 |
| Cheatgrass | 0 | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 0 | 0 | 0 | 0 | 0 | 0 |
| Giant foxtail | 0 | 0 | 0 | 0 | 0 | 0 |
| Morningglory | 0 | 0 | 0 | 0 | 0 | 0 |
| Nutsedge | 0 | - | 0 | 0 | - | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 2C,7H | 3G | 0 | 1H | 0 | 0 |
| Cheatgrass | 0 | 0 | 0 | 0 | 0 | 0 |
| Cocklebur | 0 | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 2C,6G | 2G | - | 0 | 0 | 0 |
| Giant foxtail | 2C,7G | 2G | 0 | 2G | 0 | 0 |
| Morningglory | 0 | 0 | 0 | 0 | 0 | 0 |
| Nutsedge | - | 0 | 0 | 0 | 0 | 0 |
| Rice | 2G | 0 | 0 | 0 | 0 | 0 |
| Sorghum | 2G | 0 | 0 | 0 | 0 | 0 |
| Soybean | 0 | 0 | 0 | 0 | 0 | 0 |
| Sugar beet | 1H | 0 | 0 | 0 | 0 | 0 |
| Velvetleaf | 1H | 0 | 0 | 0 | 0 | 0 |
| Wheat | 0 | 0 | 0 | 0 | 0 | 0 |
| Wild oat | 0 | 0 | 0 | 0 | 0 | 0 |

| | Cmpd 29 | | | Cmpd 30 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 0 | 0 | 0 |
| Cheatgrass | 0 | 0 | 0 | 0 | 0 | 0 |
| Cocklebur | 0 | 0 | 0 | 0 | - | 0 |
| Corn | 0 | 0 | 0 | 0 | 0 | 0 |
| Cotton | 0 | 0 | 0 | 0 | 0 | 0 |
| Crabgrass | 0 | 0 | 0 | 0 | 0 | 0 |
| Giant foxtail | 0 | 0 | 0 | 0 | 0 | 0 |
| Morningglory | 0 | 0 | 0 | 0 | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 2G | 0 | 0 | 0 | 0 | 0 |
| Cheatgrass | 0 | 0 | 0 | 0 | 0 | 0 |
| Cocklebur | - | 0 | 0 | 0 | 0 | 0 |
| Corn | 0 | 0 | 0 | 0 | 0 | 0 |
| Cotton | 0 | 0 | 0 | 0 | 0 | 0 |
| Crabgrass | 3C,9G | 1C,5G | - | 0 | 0 | 0 |
| Giant foxtail | 8H | 3G | 0 | 3G | 0 | 0 |
| Morningglory | 0 | 0 | 0 | 0 | 0 | 0 |
| Nutsedge | 0 | 0 | - | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | 0 | 0 | 0 | 0 | 0 |
| Wheat | 0 | 0 | 0 | 0 | 0 | 0 |
| Wild oat | 0 | 0 | 0 | 0 | 0 | 0 |

| | Cmpd 31 | | | Cmpd 32 | | |
|---|---|---|---|---|---|---|
| RATE (g/ha) | 200 | 100 | 50 | 200 | 100 | 50 |
| POSTEMERGENCE | | | | | | |
| Barley | 2G | 0 | 0 | 0 | 0 | 0 |
| Barnyardgrass | 3C,7H | 3C,4H | 0 | 2H | 0 | 0 |
| Cheatgrass | 0 | 0 | 0 | 0 | 0 | 0 |
| Cocklebur | 0 | 0 | 0 | 1B | 0 | 0 |
| Corn | 2C,7H | 3G | 0 | 0 | 0 | 0 |
| Cotton | 0 | 0 | 0 | - | 0 | 0 |
| Crabgrass | 2C,3G | 0 | 0 | 2C,7H | 0 | 0 |
| Giant foxtail | 5G | 0 | 0 | 2C,8G | 0 | 0 |
| Morningglory | 0 | 0 | 0 | 0 | 0 | 0 |
| Nutsedge | 0 | 0 | 0 | 0 | 0 | 0 |
| Rice | 1S,3G | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | - | 0 | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 8G | 5G | 0 | 0 | 0 | 0 |
| Barnyardgrass | 10H | 9H | 3C,8H | 9H | 2C,8H | 8H |
| Cheatgrass | 8G | 5G | 2G | 0 | 0 | 0 |
| Cocklebur | - | 0 | 0 | - | 0 | 0 |
| Corn | 3C,5G | 2G | 0 | 0 | 0 | 0 |
| Cotton | 2G | 0 | 0 | 0 | 0 | 0 |
| Crabgrass | 10H | 9H | 4S,9G | 5S,9H | 1S,5H | 4G |
| Giant foxtail | 3C,9H | 3C,8H | 3C,7G | 9H | 8H | 7H |
| Morningglory | 0 | 0 | 0 | 0 | 0 | 0 |
| Nutsedge | 0 | 0 | 0 | 0 | 0 | 0 |
| Rice | 3G | 0 | 0 | 0 | 0 | 0 |
| Sorghum | 3C,3G | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 2C,3H | 0 | 0 | 2H | 0 | 0 |
| Wheat | 7G | 3G | 0 | 0 | 0 | 0 |
| Wild oat | 7G | 3G | 0 | 0 | 0 | 0 |

| | Cmpd 33 | | | Cmpd 34 | | |
|---|---|---|---|---|---|---|
| RATE (g/ha) | 200 | 100 | 50 | 200 | 100 | 50 |
| POSTEMERGENCE | | | | | | |
| Barley | 0 | 0 | 0 | 0 | 0 | 0 |
| Barnyardgrass | 0 | 0 | 0 | 2C,8H | 1H | 0 |
| Cheatgrass | 0 | 0 | 0 | 0 | 0 | 0 |
| Cocklebur | 1B | 1B | 0 | 1B | - | 0 |
| Corn | 0 | 0 | 0 | 0 | 0 | 0 |
| Cotton | 0 | 0 | 0 | - | 0 | 0 |
| Crabgrass | 0 | 0 | 0 | 2S,6H | 1H | 0 |
| Giant foxtail | 0 | 0 | 0 | 6H | 1H | 0 |
| Morningglory | 0 | - | 0 | 1S | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 0 | - | 0 | 0 | 0 | 0 |
| Wheat | 0 | 0 | 0 | 0 | 0 | 0 |
| Wild oat | 0 | 0 | 0 | 0 | 0 | 0 |
| PREEMERGENCE | | | | | | |
| Barley | 0 | 0 | 0 | 0 | 0 | 0 |
| Barnyardgrass | 3C,9H | 3C,9H | 6G | 9H | 9H | 3C,8H |
| Cheatgrass | 0 | 0 | 0 | 5G | 3G | 0 |
| Cocklebur | 0 | - | 0 | 3H | 0 | 0 |
| Corn | 0 | 0 | 0 | 1H | 0 | 0 |
| Cotton | 0 | 0 | 0 | 0 | 0 | 0 |
| Crabgrass | 5S,9G | 1S,7G | 1S,6G | 5S,9G | 5S,9G | 3S,8G |
| Giant foxtail | 9H | 8H | 7G | 9H | 9H | 2C,8H |
| Morningglory | 0 | 0 | 0 | 1H | 0 | 0 |
| Nutsedge | 0 | 0 | 0 | 0 | 0 | 0 |
| Rice | 0 | 0 | 0 | 0 | 0 | 0 |
| Sorghum | 0 | 0 | 0 | 0 | 0 | 0 |
| Soybean | 0 | 0 | 0 | 0 | 0 | 0 |
| Sugar beet | 0 | 0 | 0 | 0 | 0 | 0 |
| Velvetleaf | 2H | 1H | 0 | 4H | 2H | 0 |
| Wheat | 0 | 0 | 0 | 0 | 0 | 0 |
| Wild oat | 0 | 0 | 0 | 4H | 0 | 0 |

| | Cmpd 35 | | |
|---|---|---|---|
| RATE (g/ha) | 200 | 100 | 50 |
| POSTEMERGENCE | | | |
| Barley | 0 | 0 | 0 |
| Barnyardgrass | 1H | 0 | 0 |
| Cheatgrass | 0 | 0 | 0 |
| Cocklebur | 1B | 1B | 0 |
| Corn | 0 | 0 | 0 |
| Cotton | 0 | 0 | 0 |
| Crabgrass | 1H | 0 | 0 |
| Giant foxtail | 0 | 0 | 0 |
| Morningglory | 0 | 0 | 0 |
| Nutsedge | 0 | - | 0 |
| Rice | 0 | 0 | 0 |
| Sorghum | 0 | 0 | 0 |
| Soybean | 0 | 0 | 0 |
| Sugar beet | 0 | 0 | 0 |
| Velvetleaf | - | 0 | 0 |
| Wheat | 0 | 0 | 0 |
| Wild oat | 0 | 0 | 0 |
| PREEMERGENCE | | | |
| Barley | 0 | 0 | 0 |
| Barnyardgrass | 4C,8H | 3C,7H | 0 |
| Cheatgrass | 0 | 0 | 0 |
| Cocklebur | 0 | 0 | 0 |
| Corn | 0 | 0 | 0 |
| Cotton | 0 | 0 | 0 |
| Crabgrass | 4S,8H | 3S,7H | 1C |
| Giant foxtail | 9H | 8H | 7G |
| Morningglory | 0 | 0 | 0 |
| Nutsedge | 0 | 0 | - |
| Rice | 0 | 0 | 0 |
| Sorghum | 0 | 0 | 0 |
| Soybean | 0 | 0 | 0 |
| Sugar beet | 0 | 0 | 0 |
| Velvetleaf | 3H | 1H | 0 |
| Wheat | 0 | 0 | 0 |
| Wild oat | 0 | 0 | 0 |

TEST B

Seeds of barley (Hordeum vulgare), barnyardgrass (Echinochloa crus-galli), blackgrass (Alopecurus myosuroides), chickweed (Stellaria media), cocklebur (Xanthium pensylvanicum), corn (Zea mays), cotton (Gossypium hirsutum), crabgrass (Digitaria spp.), downy brome (Bromus tectorum), giant foxtail (Setaria faberi), green foxtail (Setaria viridis), jimsonweed (Datura stramonium), johnsongrass (Sorghum halepense), lambsquarters (Chenopodium album), morningglory (Ipomoea spp.), rape (Brassica napus), rice (Oryza sativa), sicklepod (Cassia obtusifolia), soybean (Glycine max), sugar beet (Beta vulgaris), teaweed (Sida spinosa), velvetleaf (Abutilon theophrasti), wheat (Triticum aestivum), wild buckwheat (Polygonum convolvulus), and wild oat (Avena fatua) and purple nutsedge (Cyperus rotundus) tubers were planted and treated preemergence with the test chemical dissolved in a non-phytotoxic solvent. At the same time, these crop and weed species were also treated with postemergence applications of the test chemical. Plants ranged in height from two to eighteen cm (two to three leaf stage) for postemergence treatments. Treated plants and

controls were maintained in a greenhouse for approximately 24 days, after which all species were compared to controls and visually evaluated. The ratings, summarized in Table B, are based on a scale of 0 to 100 where 0 is no effect and 100 is complete control. A dash (-) response means no test result.

Table B

Cmpd 2

| RATE (g/ha) | 250 | 125 | 62 | 31 |
|---|---|---|---|---|
| POSTEMERGENCE | | | | |
| Barley | 0 | 0 | 0 | 0 |
| Barnyardgrass | 90 | 60 | 40 | 40 |
| Blackgrass | 70 | 70 | 50 | 40 |
| Chickweed | 0 | 0 | 0 | 0 |
| Cocklebur | 0 | 0 | 0 | 0 |
| Corn | 0 | 0 | 0 | 0 |
| Cotton | 0 | 0 | 0 | 0 |
| Crabgrass | 0 | 0 | 0 | 0 |
| Downy brome | 0 | 0 | 0 | 0 |
| Giant foxtail | 20 | 0 | 0 | 0 |
| Green foxtail | 0 | 0 | 0 | 0 |
| Jimsonweed | 0 | 0 | 0 | 0 |
| Johnsongrass | 0 | 0 | 0 | 0 |
| Lambsquarters | - | 0 | 0 | 0 |
| Morningglory | - | - | - | - |
| Nutsedge | 0 | 0 | 0 | 0 |
| Rape | 0 | 0 | 0 | 0 |
| Rice | 0 | 0 | 0 | 0 |
| Sicklepod | 0 | 0 | 0 | 0 |
| Soybean | 0 | 0 | 0 | 0 |
| Sugar beet | 0 | 0 | 0 | 0 |
| Teaweed | 0 | 0 | 0 | 0 |
| Velvetleaf | 30 | 20 | 0 | 0 |
| Wheat | 0 | 0 | 0 | 0 |
| Wild buckwheat | 0 | 0 | 0 | 0 |
| Wild oat | 0 | 0 | 0 | 0 |

Cmpd 2

| RATE (g/ha) | 250 | 125 | 62 | 31 |
|---|---|---|---|---|
| PREEMERGENCE | | | | |
| Barley | 20 | 0 | 0 | 0 |
| Barnyardgrass | 100 | 100 | 100 | 70 |
| Blackgrass | 80 | 60 | 50 | 20 |
| Chickweed | 70 | 50 | 20 | 0 |
| Cocklebur | 0 | 0 | 0 | 0 |
| Corn | 50 | 30 | 0 | 0 |
| Cotton | 0 | 0 | 0 | 0 |
| Crabgrass | 100 | 100 | 90 | 80 |
| Downy brome | 30 | 0 | 0 | 0 |
| Giant foxtail | 100 | 100 | 90 | 80 |
| Green foxtail | 100 | 100 | 50 | 30 |
| Jimsonweed | 0 | 0 | 0 | 0 |
| Johnsongrass | 70 | 50 | 0 | 0 |
| Lambsquarters | 80 | 80 | 80 | 70 |
| Morningglory | 20 | 20 | 0 | 0 |
| Nutsedge | 90 | 30 | 0 | 0 |
| Rape | 20 | 0 | 0 | 0 |
| Rice | 0 | 0 | 0 | 0 |
| Sicklepod | 30 | 0 | 0 | 0 |
| Soybean | 20 | 0 | 0 | 0 |
| Sugar beet | 80 | 20 | 0 | 0 |
| Teaweed | 30 | 0 | 0 | 0 |
| Velvetleaf | 80 | 30 | 0 | 0 |
| Wheat | 20 | 0 | 0 | 0 |
| Wild buckwheat | 30 | 0 | 0 | 0 |
| Wild oat | 70 | 50 | 20 | 0 |

TEST C

Plastic pots were partially filled with silt loam soil. The soil was then saturated with water. Indica and Japonica rice (Oryza sativa) seedlings at the 2.0 to 2.5 leaf stage, seeds of barnyardgrass (Echinochloa crus-galli), bulrush (Scirpus mucronatus), duck salad (Heteranthera limosa), and umbrella sedge (Cyperus difformis), and sprouted tubers of arrowhead (Sagittaria spp.) and waterchestnut (Eleocharis spp.) were planted into this soil. Several days after planting, water levels were raised to 3 cm above the soil surface and maintained at this level throughout the test. Chemical treatments were formulated in a non-phytotoxic solvent and applied directly to the paddy water. Treated plants and controls were maintained in a greenhouse for approximately 21 days, after which all species were compared to controls and visually evaluated. Plant response ratings, summarized in Table C, are based on a scale of 0 to 100 where 0 is no effect and 100 is complete control. A dash response (-) means no test result.

Table C

| | Cmpd 1 | | | | |
|---|---|---|---|---|---|
| RATE (g/ha) | 500 | 250 | 125 | 64 | 32 |
| Arrowhead | 98 | 98 | 40 | 0 | - |
| Barnyardgrass | 100 | 100 | 100 | 100 | 95 |
| Bulrush | 95 | 90 | 60 | 50 | 30 |
| Duck salad | 100 | 98 | 98 | 98 | 95 |
| Indica rice | 70 | 30 | 0 | 0 | 0 |
| Japonica rice | 40 | 20 | 20 | 20 | 0 |
| Umbrella sedge | 100 | 100 | 100 | 98 | 95 |
| Waterchestnut | 0 | 0 | 0 | 0 | 0 |

| | Cmpd 2 | | | | |
|---|---|---|---|---|---|
| RATE (g/ha) | 500 | 250 | 125 | 64 | 32 |
| Arrowhead | 98 | 95 | 0 | 0 | - |
| Barnyardgrass | 100 | 100 | 100 | 100 | 100 |
| Bulrush | 70 | 50 | 50 | 30 | 40 |
| Duck salad | 100 | 98 | 95 | 90 | 90 |
| Indica rice | 80 | 80 | 30 | 0 | 0 |
| Japonica rice | 50 | 40 | 30 | 0 | 0 |
| Umbrella sedge | 100 | 100 | 100 | 100 | 95 |
| Waterchestnut | 50 | 40 | 30 | - | 0 |

| | Cmpd 3 | | | | |
|---|---|---|---|---|---|
| RATE (g/ha) | 500 | 250 | 125 | 64 | 32 |
| Arrowhead | 40 | 0 | 0 | 0 | 0 |
| Barnyardgrass | 100 | 100 | 95 | 90 | 70 |
| Bulrush | 90 | 90 | 60 | 0 | 0 |
| Duck salad | 100 | 100 | 95 | 95 | 90 |
| Indica rice | 80 | 30 | 0 | 0 | 0 |
| Japonica rice | 0 | 0 | 0 | 0 | 0 |
| Umbrella sedge | 100 | 100 | 100 | 90 | 80 |
| Waterchestnut | 80 | 0 | 0 | 0 | 0 |

| | Cmpd 4 | | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 95 | 100 | 95 | 95 | 70 |
| Bulrush | 80 | 0 | 0 | 0 | 0 |
| Duck salad | 80 | 90 | 80 | 0 | 0 |
| Indica rice | 20 | 0 | 0 | 0 | 0 |
| Japonica rice | 30 | 0 | 0 | 0 | 0 |
| Umbrella sedge | 95 | 95 | 95 | 60 | 0 |
| Waterchestnut | 0 | 0 | 0 | 0 | 0 |

| | Cmpd 5 | | | |
|---|---|---|---|---|
| RATE (g/ha) | 250 | 125 | 64 | 32 |
| Arrowhead | 0 | 0 | 0 | 0 |
| Barnyardgrass | 95 | 70 | 40 | 30 |
| Bulrush | 0 | 0 | 0 | 0 |
| Duck salad | 80 | 70 | 0 | 0 |
| Indica rice | 0 | 0 | 0 | 0 |
| Japonica rice | 20 | 0 | 0 | 0 |
| Umbrella sedge | 90 | 95 | 80 | 50 |
| Waterchestnut | 0 | 0 | 0 | 0 |

| | Cmpd 6 | | | | |
|---|---|---|---|---|---|
| RATE (g/ha) | 500 | 250 | 125 | 64 | 32 |
| Arrowhead | - | - | - | - | - |
| Barnyardgrass | 90 | 100 | 100 | 90 | 60 |
| Bulrush | 80 | 60 | 0 | 0 | 0 |
| Duck salad | 100 | 80 | 0 | 0 | 0 |
| Indica rice | 20 | 20 | 0 | 0 | 0 |
| Japonica rice | 30 | 20 | 0 | 0 | 0 |
| Umbrella sedge | 100 | 95 | 90 | 70 | 60 |
| Waterchestnut | 0 | 0 | 0 | 0 | 0 |

| | Cmpd 7 | | | | |
|---|---|---|---|---|---|
| RATE (g/ha) | 500 | 250 | 125 | 64 | 32 |
| Arrowhead | 20 | 20 | 0 | 0 | 0 |
| Barnyardgrass | 90 | 80 | 80 | 80 | 60 |
| Bulrush | 0 | 0 | 0 | 0 | 0 |
| Duck salad | 100 | 80 | 30 | 20 | 0 |
| Indica rice | 20 | 20 | 10 | 10 | 0 |
| Japonica rice | 40 | 30 | 20 | 20 | 0 |
| Umbrella sedge | 100 | 95 | 90 | 60 | 0 |
| Waterchestnut | 30 | 30 | 0 | 0 | 0 |

| | Cmpd 10 | | | | |
|---|---|---|---|---|---|
| RATE (g/ha) | 500 | 250 | 125 | 64 | 32 |
| Arrowhead | - | - | - | - | - |
| Barnyardgrass | 80 | 100 | 100 | 60 | 50 |
| Bulrush | 0 | 0 | 0 | 0 | 0 |
| Duck salad | 100 | 90 | 90 | 80 | 0 |
| Indica rice | 0 | 0 | 0 | 0 | 0 |
| Japonica rice | 0 | 0 | 0 | 0 | 0 |
| Umbrella sedge | 100 | 100 | 95 | 95 | 60 |
| Waterchestnut | 0 | 0 | 0 | 0 | 0 |

| | Cmpd 13 | | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 100 | 80 | 80 | 70 | 20 |
| Bulrush | 0 | 0 | 0 | 0 | 0 |
| Duck salad | 0 | 100 | 100 | 80 | 30 |
| Indica rice | 0 | 0 | 0 | 0 | 0 |
| Japonica rice | 0 | 0 | 0 | 0 | 0 |
| Umbrella sedge | 100 | 95 | 50 | 30 | 0 |
| Waterchestnut | 30 | 0 | 0 | 0 | 0 |

| | Cmpd 14 | | | | |
|---|---|---|---|---|---|
| RATE (g/ha) | 500 | 250 | 125 | 64 | 32 |
| Arrowhead | 70 | 0 | 0 | 0 | 0 |
| Barnyardgrass | 100 | 100 | 100 | 80 | 40 |
| Bulrush | 0 | 0 | 0 | 0 | 0 |
| Duck salad | 100 | 100 | 70 | 80 | 30 |
| Indica rice | 30 | 0 | 0 | 0 | 0 |
| Japonica rice | 0 | 0 | 0 | 0 | 0 |
| Umbrella sedge | 100 | 100 | 80 | 60 | 0 |
| Waterchestnut | 0 | 0 | 0 | 0 | 0 |

| | Cmpd 15 | | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 100 | 70 | 70 | 30 | 0 |
| Bulrush | 0 | 0 | 0 | 0 | 0 |
| Duck salad | 0 | 90 | 50 | 30 | 0 |
| Indica rice | 0 | 0 | 0 | 0 | 0 |
| Japonica rice | 0 | 0 | 0 | 0 | 0 |
| Umbrella sedge | 95 | 90 | 70 | 40 | 0 |
| Waterchestnut | 30 | 0 | 0 | 0 | 0 |

| | Cmpd 16 | | | | |
|---|---|---|---|---|---|
| RATE (g/ha) | 500 | 250 | 125 | 64 | 32 |
| Arrowhead | - | - | - | - | - |
| Barnyardgrass | 100 | 90 | 100 | 80 | 70 |
| Bulrush | 70 | 70 | 0 | 0 | 0 |
| Duck salad | 100 | 90 | 80 | 70 | 0 |
| Indica rice | 20 | 0 | 0 | 0 | 0 |
| Japonica rice | 40 | 0 | 0 | 0 | 0 |
| Umbrella sedge | 100 | 100 | 100 | 100 | 95 |
| Waterchestnut | 0 | 0 | 0 | 0 | 0 |

| | | | Cmpd 17 | | | |
|---|---|---|---|---|---|
| RATE (g/ha) | 500 | 250 | 125 | 64 | 32 |
| Arrowhead | - | - | - | - | - |
| Barnyardgrass | 100 | 90 | 70 | 40 | 30 |
| Bulrush | 0 | 0 | 0 | 0 | 0 |
| Duck salad | 0 | 0 | 0 | 0 | 0 |
| Indica rice | 0 | 0 | 0 | 0 | 0 |
| Japonica rice | 0 | 0 | 0 | 0 | 0 |
| Umbrella sedge | 100 | 100 | 95 | 0 | 0 |
| Waterchestnut | 0 | 0 | 0 | 0 | 0 |

| | | Cmpd 18 | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 100 | 100 | 100 | 100 | 100 |
| Bulrush | 90 | 80 | 70 | 0 | 0 |
| Duck salad | 100 | 100 | 80 | 70 | 60 |
| Indica rice | 80 | 60 | 0 | 0 | 0 |
| Japonica rice | 70 | 30 | 0 | 0 | 0 |
| Umbrella sedge | 100 | 100 | 100 | 100 | 100 |
| Waterchestnut | 0 | 0 | 0 | 0 | 0 |

| | | Cmpd 19 | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 90 | 50 | 0 | 0 | 0 |
| Bulrush | 0 | 0 | 0 | 0 | 0 |
| Duck salad | 0 | 0 | 0 | 0 | 0 |
| Indica rice | 0 | 0 | 0 | 0 | 0 |
| Japonica rice | 0 | 0 | 0 | 0 | 0 |
| Umbrella sedge | 80 | 60 | 0 | 0 | 0 |
| Waterchestnut | 0 | 0 | 0 | 0 | 0 |

| | | Cmpd 21 | | | |
|---|---|---|---|---|---|
| RATE (g/ha) | 500 | 250 | 125 | 64 | 32 |
| Arrowhead | 20 | 20 | 0 | 0 | 0 |
| Barnyardgrass | 80 | 80 | 70 | 60 | 50 |
| Bulrush | 20 | 0 | 0 | 0 | 0 |
| Duck salad | 80 | 70 | 20 | 0 | 0 |
| Indica rice | 20 | 0 | 0 | 0 | 0 |
| Japonica rice | 40 | 0 | 0 | 0 | 0 |
| Umbrella sedge | 100 | 90 | 0 | 0 | 0 |
| Waterchestnut | 0 | 0 | 0 | 0 | 0 |

| Cmpd 22 | | | | | |
|---|---|---|---|---|---|
| RATE (g/ha) | 500 | 250 | 125 | 64 | 32 |
| Arrowhead | - | - | - | - | - |
| Barnyardgrass | 100 | 100 | 70 | 100 | 80 |
| Bulrush | 70 | 60 | 0 | 0 | 0 |
| Duck salad | 100 | 90 | 80 | 80 | 0 |
| Indica rice | 20 | 0 | 0 | 0 | 0 |
| Japonica rice | 30 | 20 | 0 | 0 | 0 |
| Umbrella sedge | 100 | 100 | 100 | 90 | 70 |
| Waterchestnut | 0 | 0 | 0 | 0 | 0 |

| Cmpd 23 | | | | | |
|---|---|---|---|---|---|
| RATE (g/ha) | 500 | 250 | 125 | 64 | 32 |
| Arrowhead | 0 | 0 | 0 | 0 | 0 |
| Barnyardgrass | 100 | 100 | 100 | 100 | 70 |
| Bulrush | 50 | 40 | 0 | 0 | 0 |
| Duck salad | 80 | 80 | 0 | 0 | 0 |
| Indica rice | 0 | 0 | 0 | 0 | 0 |
| Japonica rice | 30 | 0 | 0 | 0 | 0 |
| Umbrella sedge | 100 | 100 | 100 | 100 | 80 |
| Waterchestnut | 0 | 0 | 0 | 0 | 0 |

| Cmpd 25 | | | | | |
|---|---|---|---|---|---|
| RATE (g/ha) | 500 | 250 | 125 | 64 | 32 |
| Arrowhead | - | - | - | - | - |
| Barnyardgrass | 100 | 95 | 80 | 95 | 70 |
| Bulrush | 80 | 70 | 0 | 0 | 0 |
| Duck salad | 100 | 80 | 0 | 0 | 0 |
| Indica rice | 30 | 30 | 30 | 0 | 0 |
| Japonica rice | 30 | 30 | 30 | 0 | 0 |
| Umbrella sedge | 100 | 100 | 100 | 100 | 70 |
| Waterchestnut | 0 | 0 | 0 | 0 | 0 |

| Cmpd 26 | | | | | |
|---|---|---|---|---|---|
| RATE (g/ha) | 500 | 250 | 125 | 64 | 32 |
| Arrowhead | - | - | - | - | - |
| Barnyardgrass | 100 | 100 | 95 | 95 | 100 |
| Bulrush | 95 | 70 | 60 | 40 | 0 |
| Duck salad | 100 | 95 | 90 | 80 | 80 |
| Indica rice | 60 | 30 | 20 | 0 | 0 |
| Japonica rice | 50 | 30 | 20 | 0 | 0 |
| Umbrella sedge | 100 | 100 | 100 | 100 | 100 |
| Waterchestnut | 0 | 0 | 0 | 0 | 0 |

## Claims

1. The compounds of Formula I:

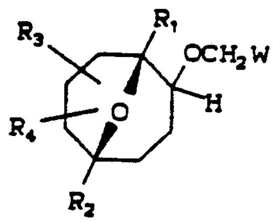

I

wherein:

$R_1$ is H or a $C_1$-$C_4$ straight chain alkyl group;

$R_2$ is H, $C_1$-$C_6$ alkyl, CN, $C_2$-$C_4$ alkenyl, $C_2$-$C_4$ alkynyl, phenyl, $CH_2Ph$ or $C_1$-$C_3$ alkyl substituted with OH, CN, $C_1$-$C_3$ alkoxy, OPh, $C_1$-$C_3$ alkyl- sulfonyl, $SO_2Ph$, $SO_2CH_2Ph$, $N_3$, $C_1$-$C_6$ alkoxy- carbonyl or $CO_2H$.

$R_3$ is H, F, Cl, Br, I, OH, PhSe, $C_1$-$C_3$ alkoxy, $OSO_2Ph$ optionally substituted with 1-3 $CH_3$, $PhSO_n$ optionally substituted with 1-3 $CH_3$ (n = 0, 1 or 2), $CH_3Se$, phenyl, $C_2$-$C_4$ alkenyl or $C_1$-$C_4$ alkyl optionally substituted with 1-3 halogens, or 1-3 $C_1$-$C_3$ alkoxy, or amino substituted with 0-2 alkyl groups of 1-3 carbons provided that when $R_3$ is $C_1$-$C_4$ alkyl substituted by $C_1$-$C_3$ alkoxy, $R_3$ is not on the carbon adjacent to the carbon bearing $R_1$;

$R_4$ is H, OH, $C_1$-$C_4$ alkyl, phenyl or CN;

$R_3$ and $R_4$ may be taken together to form a 5 membered ring acetonide optionally substituted with 1-2 $CH_3$ or $R_3$ and $R_4$ when on the same carbon may be taken together to form a ketone or $R_3$ may be taken together with an adjacent carbon to form a double bond with the proviso said double bond is not at a bridgehead carbon or the $OCH_2W$ carbon. $R_3$ and $R_4$ on adjacent carbons may be taken together to form an epoxide. If $R_4$ = OH and $R_3$ and $R_4$ are on the same carbon, then $R_3$ cannot be OH, F, Cl, Br, I, PhSe, $CH_3Se$, alkoxy or amino.

W is phenyl optionally substituted with 1-3 substituents selected from F, Cl, Br, $C_1$-$C_3$ alkyl, $C_1$-$C_3$ alkoxy, OH, CN, $C_1$-$C_3$ haloalkyl, $C_1$-$C_3$ haloalkoxy, $C_1$-$C_3$ alkylthio, $C_2$-$C_4$ alkenyl or $C_2$-$C_4$ alkynyl, or W is a 4, 5, 6 or 7-membered heterocyclic ring containing at least one from among 0-3 nitrogen, 0-2 oxygen or 0-2 sulfur, each ring optionally substituted with 1-2 substituents selected from F, Br, CN, Cl, $CH_3$ or $OCH_3$.

2. The compounds according to Claim 1 wherein the compounds are:

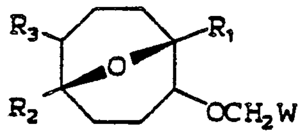 ,

Ia

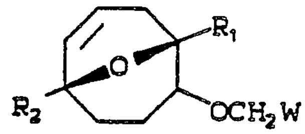 ,

Ib

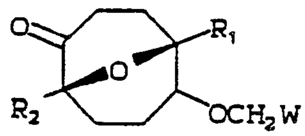 ,

Ic

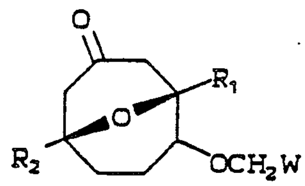 ,

Id

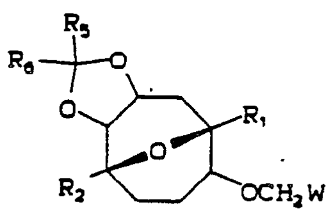 ,

Ie

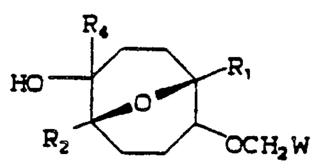 ,

If

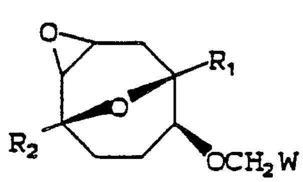

Ig

or

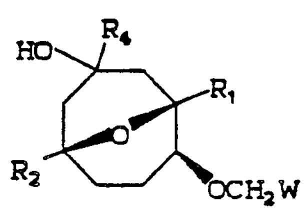

Ih

wherein:
$R_5$ is H or $CH_3$; and;
$R_6$ is H, or $CH_3$.

3. The compounds of claim 2 wherein:
$R_1$ is $C_1$-$C_3$ alkyl;
$R_2$ is $C_1$-$C_3$ alkyl, $C_2$-$C_3$ alkenyl, $C_2$-$C_3$ alkynyl, phenyl, $CH_2Ph$ or $C_1$-$C_2$ alkyl substituted by $OCH_3$, CN, $SO_2CH_3$ or $C_1$-$C_3$ alkoxycarbonyl.

4. The compounds of Claim 3 wherein:
W is phenyl optionally substituted by 1-2 substituents selected from F, Cl, Br, $CH_3$ or $OCH_3$ or W is a 5 or 6-membered heterocyclic ring containing 0-2 nitrogen, 0-2 oxygen or 0-2 sulfur;
$R_3$ is H, F, Br or Cl; and;
$R_4$ is H.

5. The compounds of Claim 4 wherein:
W is phenyl optionally substituted by 1-2 substituents selected from F, Cl, Br, $CH_3$ or $OCH_3$ or W is tetrahydropyran, tetrahydrofuran, thiophene, thiazole, isoxazole, pyridine or pyrazine, each ring optionally substituted with 1-2 substituents selected from F, Cl, Br, $CH_3$ or $OCH_3$.

6. The compounds of Claim 5 wherein:
Formula I is Ia.

7. The compound of Claim 6 which is:
2-[(2-fluorophenyl)methoxy]-1,5-dimethyl-9-oxabicyclo[3.3.1]nonane.

8. The compound of Claim 6 which is:
2-[(2,6-difluorophenyl)methoxy]-1,5-dimethyl-9-oxabicyclo[3.3 1]nonane.

9. The compounds of Claim 1 wherein:
W is selected from the class consisting of oxetane, furan, dioxane, oxepane, dioxolane, oxadiazole, thiadiazole, triazole, thiophene, tetrahydropyran, tetrahydrofuran, isoxazole, oxazole, pyrazole, imidazole, thiazole, pyridine and pyrazines; provided that the total number of heteroatoms is 3 or less.

10. A method of controlling the undesired vegetation in rice which comprises applying to a locus an effective amount of a compound of Claim 1.

11. The compound of Claims 1-9 wherein at least one of the following, surfactant, solid diluent, or liquid diluent, is present in addition to the compound.

European Patent Office

# EUROPEAN SEARCH REPORT

Application Number

EP 90 30 1210

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | DE-A-2 937 645 (BAYER)<br>* Claims 1,4; page 10, lines 34-36 *<br>--- | 1,10 | C 07 D 493/08<br>C 07 D 493/18<br>A 01 N 43/90 //<br>(C 07 D 493/08<br>C 07 D 311:00<br>C 07 D 311:00 )<br>(C 07 D 493/18<br>C 07 D 317:00<br>C 07 D 311:00<br>C 07 D 311:00 )<br>(C 07 D 493/18<br>C 07 D 311:00<br>C 07 D 311:00<br>C 07 D 303:00 ) |
| D,A | EP-A-0 081 893 (SHELL)<br>* Claims 1,24 * & US-A-4 567 283<br>----- | 1,10 | |
| | | | **TECHNICAL FIELDS SEARCHED (Int. Cl.5)** |
| | | | C 07 D 493/00<br>A 01 N |

The present search report has been drawn up for all claims

| Place of search | Date of completion of the search | Examiner |
|---|---|---|
| THE HAGUE | 03-05-1990 | VOYIAZOGLOU D. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)